(19) Europäisches Patentamt
European Patent Office
Office européen des brevets

(11) EP 4 212 534 A1

(12) EUROPEAN PATENT APPLICATION
published in accordance with Art. 153(4) EPC

(43) Date of publication:
**19.07.2023 Bulletin 2023/29**

(21) Application number: **21866003.3**

(22) Date of filing: **08.09.2021**

(51) International Patent Classification (IPC):
***C07D 487/04** (2006.01)* ***A61K 31/519** (2006.01)*
***A61P 35/00** (2006.01)*

(52) Cooperative Patent Classification (CPC):
**A61K 31/519; A61P 35/00; C07D 487/04**

(86) International application number:
**PCT/CN2021/117174**

(87) International publication number:
**WO 2022/052950 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.09.2020 CN 202010933538**
**03.08.2021 CN 202110869600**

(71) Applicant: **Haisco Pharmaceuticals Pte. Ltd.**
**Singapore 079903 (SG)**

(72) Inventors:
- **ZHANG, Chen**
  **Tibet 856000 (CN)**
- **LIAO, Yuting**
  **Tibet 856000 (CN)**
- **WANG, Jianmin**
  **Tibet 856000 (CN)**
- **HUANG, Longbin**
  **Tibet 856000 (CN)**
- **ZHU, Guozhi**
  **Tibet 856000 (CN)**
- **LI, Yao**
  **Tibet 856000 (CN)**
- **YAN, Pangke**
  **Tibet 856000 (CN)**

(74) Representative: **Sagittarius IP**
**Marlow International**
**Parkway**
**Marlow, SL7 1YL (GB)**

# (54) SALT OF COMPOUND FOR DEGRADING BTK, CRYSTAL FORM THEREOF, AND USE THEREOF IN MEDICINE

(57) Provided are a salt of a compound for degrading BTK, and/or a crystal form, preparation therefor, and an application thereof. The pharmaceutical salt of the compound as shown in formula (I) and the crystal form, wherein the pharmaceutical salt is selected from maleate, fumarate, halogen acid salt (preferably hydrobromide and hydrochloride), sulfate, phosphate, L-tartrate, citrate, L-malate, hippurate, D-glucuronate, glycollate, mucate, succinate, lactate, orotate, pamoate, glycinate, alanine salt, arginine salt, cinnamate, benzoate, benzenesulfonate, p-toluenesulfonate, acetate, propionate, valerianate, triphenyl acetate, L-proline salt, ferulate, 2-hydroxyethanesulfonate, mandelate, nitrate, mesylate, malonate, gentisate, salicylate, oxalate, or glutarate:

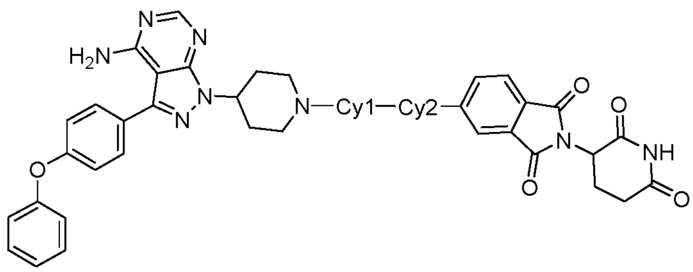

(I).

## Description

### Technical Field

The present invention relates to the field of medicine, and specifically relates to a crystal form of a salt of a compound for degrading BTK, preparation therefor, and an application thereof.

### Background Art

Bruton's tyrosine kinase (BTK), a member of the Tec family of non-receptor protein tyrosine kinases, is a key regulator in the B cell antigen receptor (BCR) signalling pathway, and is distributed in the lymphatic system, hematopoietic system and blood system. BTK mutations may activate downstream signalling pathways in tumour cell proliferation, differentiation, angiogenesis, etc., which may lead to X-linked agammaglobulinemia, non-Hodgkin's lymphoma (NHL) and many B-cell malignancies, including chronic lymphocytic leukaemia (CLL), mantle cell lymphoma, and diffuse large B-cell lymphoma. As mainly expressed in B cells and myeloid cells, BTK is a target with relatively high targeting ability and safety.

PROTAC (proteolysis targeting chimera) molecules are a class of dual function compounds which are capable of binding to both targeted proteins and E3 ubiquitin ligases. This class of compounds can induce recognition of targeted proteins by proteasomes in a cell to cause the degradation of the targeted protein, which can effectively reduce the contents of the targeted proteins in the cell. By introducing a ligand capable of binding to various targeted proteins into the PROTAC molecules, it is possible to apply the PROTAC technology to the treatment of various diseases, and this technology has attracted extensive attention in recent years.

### Summary of the Invention

An object of the present invention is to provide a compound with a novel structure and a good pharmaceutical effect for degrading BTK, a pharmaceutical composition thereof and the use thereof in the anti-tumour field. The compound for degrading BTK of the present invention has good stability (including chemical stability and crystal form stability), is convenient for oral administration, and has relatively good solubility and bioavailability.

An object of the present invention is to provide a pharmaceutical salt of a compound with a novel structure and a good pharmaceutical effect for degrading BTK or crystals of the compound for degrading BTK and the pharmaceutical salt thereof, a pharmaceutical composition thereof and the use thereof in the anti-tumour field.

The crystals of the present invention are easy to be processed, crystallized and treated, has good stability, is convenient for oral administration, and has relatively good solubility and bioavailability.

Another object of the present invention is to provide a method for preparing the compound for degrading BTK and/or the crystal.

Another object of the present invention is to provide a pharmaceutical composition containing the compound for degrading BTK and/or the crystal.

Yet another object of the present invention is to provide an application of the compound for degrading BTK and/or the crystal.

The present invention provides a pharmaceutical salt of a compound as shown in formula (I),

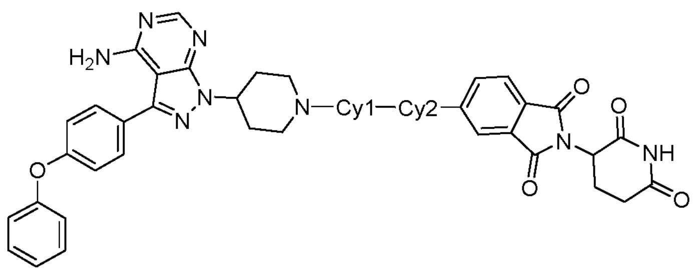

(I).

In some embodiments, Cy1 or Cy2 is each independently selected from piperidyl or azacyclobutyl.

In some embodiments, Cy1 or Cy2 is each independently selected from

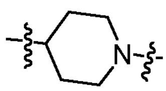

or

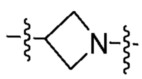

.

In some embodiments, the pharmaceutical salt of the compound as shown in formula (I) is selected from maleate, fumarate, halogen acid salt (preferably hydrobromide and hydrochloride), sulfate, phosphate, L-tartrate, citrate, L-malate, hippurate, D-glucuronate, glycollate, mucate, succinate, lactate, orotate, pamoate, glycinate, alanine salt, arginine salt, cinnamate, benzoate, benzenesulfonate, p-toluenesulfonate, acetate, propionate, valerianate, triphenyl acetate, L-proline salt, ferulate, 2-hydroxyethanesulfonate, mandelate, nitrate, mesylate, malonate, gentisate, salicylate, oxalate or glutarate.

In some embodiments, the halogen acid salt is hydrobromide or hydrochloride.

In some embodiments, the molar ratios of the compound (free base) as shown in formula (I) to different acids are about 1 : 1, 1 : 1.5, 1 : 2, 1 : 2.5 or 1 : 3.

The present invention further provides a pharmaceutical salt of the compound as shown in formula (Ia) or (Ib) below,

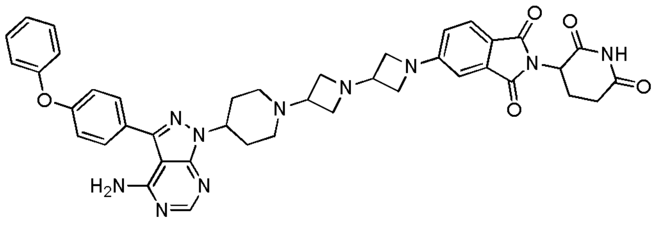

(Ia)

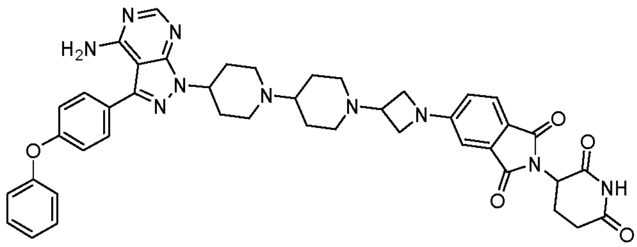

(Ib).

In some embodiments, the pharmaceutical salt of the compound as shown in formula (Ia) or (Ib) is selected from maleate, fumarate, halogen acid salt (preferably hydrobromide and hydrochloride), sulfate, phosphate, L-tartrate, citrate, L-malate, hippurate, D-glucuronate, glycollate, mucate, succinate, lactate, orotate, pamoate, glycinate, alanine salt, arginine salt, cinnamate, benzoate, benzenesulfonate, p-toluenesulfonate, acetate, propionate, valerianate, triphenyl acetate, L-proline salt, ferulate, 2-hydroxyethanesulfonate, mandelate, nitrate, mesylate, malonate, gentisate, salicylate, oxalate or glutarate, preferably maleate, fumarate, L-tartrate, citrate, L-malate, salicylate or oxalate.

In some embodiments, the pharmaceutical salt of the compound as shown in formula (Ia) is selected from maleate, and the molar ratio of the compound as shown in formula (Ia) to the maleate is about 1 : 1, 1 : 1.5, 1 : 2, 1 : 2.5 or 1 : 3.

In some embodiments, the pharmaceutical salt of the compound as shown in formula (I) has a structure as shown in formula (II).

The present invention further provides a compound as shown in formula (II) below,

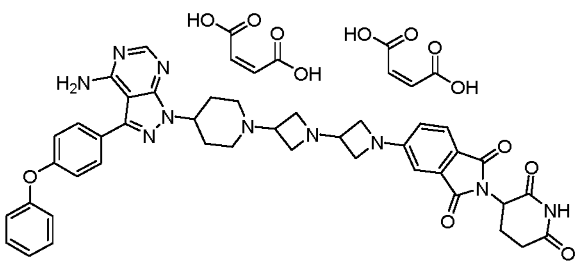

(II).

The present invention further provides a crystal form I of the compound as shown in formula (II), wherein the crystal form I has an X-ray powder diffraction pattern comprising characteristic diffraction peaks at 5.96° $\pm$ 0.2°, 9.30° $\pm$ 0.2°, 11.86° $\pm$ 0.2°, 15.80° $\pm$ 0.2°, 21.75° $\pm$ 0.2° and 23.93° $\pm$ 0.2° 2$\theta$, as determined by using Cu-K$\alpha$ radiation.

Preferably, the crystal form I of the compound as shown in formula (II) of the present invention has an X-ray

powder diffraction pattern further comprising characteristic diffraction peaks at 3.98° ± 0.2°, 7.65° ± 0.2°, 10.87° ± 0.2°, 16.88° ± 0.2°, 17.89° ± 0.2° and 26.21° ± 0.2° 2θ, as determined by using Cu-Kα radiation.

More preferably, the crystal form I of the compound as shown in formula (II) of the present invention has an X-ray powder diffraction pattern further comprising characteristic diffraction peaks at 15.29° ± 0.2°, 17.33° ± 0.2°, 18.55° ± 0.2°, 19.21° ± 0.2°, 19.91° ± 0.2° and 22.41° ± 0.2° 2θ, as determined by using Cu-Kα radiation.

More preferably, the crystal form I of the compound as shown in formula (II) of the present invention has an X-ray powder diffraction pattern further comprising characteristic diffraction peaks at 4.72° ± 0.2°, 9.58° ± 0.2°, 9.92° ± 0.2°, 12.85° ± 0.2°, 13.37° ± 0.2°, 13.75° ± 0.2°, 14.45° ± 0.2°, 27.37° ± 0.2°, 28.43° ± 0.2°, 30.27° ± 0.2°, 31.51° ± 0.2° and 34.21° ± 0.2° 2θ, as determined by using Cu-Kα radiation.

In some embodiments, the crystal form I of the compound as shown in formula (II) of the present invention has an X-ray powder diffraction pattern substantially as shown in Figure 28.

In some embodiments, the crystal form I of the compound as shown in formula (II) of the present invention has a differential scanning calorimetry (DSC) curve as shown in Figure 29 or a thermogravimetric analysis curve as shown in Figure 30.

The present invention further provides an amorphous form of the compound as shown in formula (II), which, as determined by using Cu-Kα radiation, has an X-ray powder diffraction pattern substantially as shown in Figure 31.

In some embodiments, the amorphous form of the compound as shown in formula (II) of the present invention has a differential scanning calorimetry (DSC) curve as shown in Figure 32 or a thermogravimetric analysis curve as shown in Figure 33.

The present invention further provides a crystal form II of the compound as shown in formula (II), wherein the crystal form II has an X-ray powder diffraction pattern comprising characteristic diffraction peaks at 3.98° ± 0.2°, 6.35° ± 0.2°, 8.10° ± 0.2°, 9.66° ± 0.2°, 12.21° ± 0.2°, 15.79° ± 0.2°, 16.75° ± 0.2° and 19.39° ± 0.2° 2θ, as determined by using Cu-Kα radiation.

Preferably, the crystal form II of the compound as shown in formula (II) of the present invention has an X-ray powder diffraction pattern further comprising characteristic diffraction peaks at 12.78° ± 0.2°, 16.33° ± 0.2°, 17.13° ± 0.2°, 17.41° ± 0.2°, 20.45° ± 0.2°, 21.43° ± 0.2°, 23.23° ± 0.2°, 24.65° ± 0.2° and 25.75° ± 0.2° 2θ, as determined by using Cu-Kα radiation.

In some embodiments, the crystal form II of the compound as shown in formula (II) of the present invention has an X-ray powder diffraction pattern substantially as shown in Figure 34.

In some embodiments, the crystal form II of the compound as shown in formula (II) of the present invention has a differential scanning calorimetry (DSC) curve as shown in Figure 35 or a thermogravimetric analysis curve as shown in Figure 36.

The present invention further provides an amorphous form of the compound as shown in formula (Ia), which, as determined by using Cu-Kα radiation, has an X-ray powder diffraction pattern substantially as shown in Figure 1.

In some embodiments, the amorphous form of the compound as shown in formula (Ia) of the present invention has a differential scanning calorimetry (DSC) curve as shown in Figure 2 or a thermogravimetric analysis curve as shown in Figure 3.

The present invention further provides a crystal form I of the compound as shown in formula (Ia), wherein the crystal form I has an X-ray powder diffraction pattern comprising characteristic diffraction peaks at 8.32° ± 0.2°, 15.69° ± 0.2°, 16.41° ± 0.2°, 17.57° ± 0.2°, 18.89° ± 0.2° and 19.75° ± 0.2° 2θ, as determined by using Cu-Kα radiation.

Preferably, the crystal form I of the compound as shown in formula (Ia) of the present invention has an X-ray powder diffraction pattern further comprising characteristic diffraction peaks at 10.94° ± 0.2°, 11.99° ± 0.2°, 13.30° ± 0.2°, 14.39° ± 0.2°, 16.67° ± 0.2°, 17.24° ± 0.2°, 18.00° ± 0.2°, 21.25° ± 0.2°, 22.27° ± 0.2°, 23.85° ± 0.2° and 26.45° ± 0.2° 2θ, as determined by using Cu-Kα radiation.

In some embodiments, the crystal form I of the compound as shown in formula (Ia) of the present invention has an X-ray powder diffraction pattern substantially as shown in Figure 4.

In some embodiments, the crystal form I of the compound as shown in formula (Ia) of the present invention has a differential scanning calorimetry (DSC) curve as shown in Figure 5 or a thermogravimetric analysis curve as shown in Figure 6.

The present invention further provides a crystal form II of the compound as shown in formula (Ia), wherein the crystal form II has an X-ray powder diffraction pattern comprising characteristic diffraction peaks at 4.98° ± 0.2°, 7.86° ± 0.2°, 13.72° ± 0.2°, 17.65° ± 0.2° and 20.01° ± 0.2° 2θ, as determined by using Cu-Kα radiation.

Preferably, the crystal form II of the compound as shown in formula (Ia) of the present invention has an X-ray powder diffraction pattern further comprising characteristic diffraction peaks at 5.48° ± 0.2°, 13.43° ± 0.2°, 14.93° ± 0.2°, 15.90° ± 0.2°, 16.57° ± 0.2°, 16.95° ± 0.2°, 21.29° ± 0.2°, 22.05° ± 0.2°, 24.97° ± 0.2° and 25.77° ± 0.2° 2θ, as determined by using Cu-Kα radiation.

In some embodiments, the crystal form II of the compound as shown in formula (Ia) of the present invention has an X-ray powder diffraction pattern substantially as shown in Figure 7.

In some embodiments, the crystal form II of the compound as shown in formula (Ia) of the present invention has a differential scanning calorimetry (DSC) curve as shown in Figure 8 or a thermogravimetric analysis curve as shown in Figure 9.

The present invention further provides a crystal form III of the compound as shown in formula (Ia), wherein the crystal form III has an X-ray powder diffraction pattern comprising characteristic diffraction peaks at 5.02° ± 0.2°, 8.04° ± 0.2°, 16.91° ± 0.2°, 17.23° ± 0.2°, 18.19° ± 0.2°, 19.41° ± 0.2° and 20.03° ± 0.2° 2θ, as determined by using Cu-Kα radiation.

Preferably, the crystal form III of the compound as shown in formula (Ia) has an X-ray powder diffraction pattern comprising characteristic diffraction peaks at 12.36° ± 0.2°, 14.60° ± 0.2°, 15.03° ± 0.2°, 15.73° ± 0.2°, 20.57° ± 0.2°, 21.31° ± 0.2° and 25.45° ± 0.2° 2θ, as determined by using Cu-Kα radiation.

More preferably, the crystal form III of the compound as shown in formula (Ia) has an X-ray powder diffraction pattern comprising characteristic diffraction peaks at 5.19° ± 0.2°, 16.32° ± 0.2°, 18.75° ± 0.2°, 19.73° ± 0.2°, 21.91° ± 0.2°, 22.41° ± 0.2°, 23.48° ± 0.2°, 23.95° ± 0.2° and 26.33° ± 0.2° 2θ, as determined by using Cu-Kα radiation.

More preferably, the crystal form III of the compound as shown in formula (Ia) has an X-ray powder diffraction pattern comprising characteristic diffraction peaks at 10.34° ± 0.2°, 24.85° ± 0.2°, 26.93° ± 0.2°, 27.57° ± 0.2°, 28.41° ± 0.2°, 29.59° ± 0.2°, 30.19° ± 0.2°, 31.77° ± 0.2°, 33.13° ± 0.2° and 35.75° ± 0.2° 2θ, as determined by using Cu-Kα radiation.

In some embodiments, the crystal form III of the compound as shown in formula (Ia) of the present invention has an X-ray powder diffraction pattern substantially as shown in Figure 10.

In some embodiments, the crystal form III of the compound as shown in formula (Ia) of the present invention has a differential scanning calorimetry (DSC) curve as shown in Figure 11 or a thermogravimetric analysis curve as shown in Figure 12.

The present invention further provides a crystal form I of the compound as shown in formula (Ib), wherein the crystal form I has an X-ray powder diffraction pattern comprising characteristic diffraction peaks at 4.38° ± 0.2°, 8.66° ± 0.2°, 13.06° ± 0.2°, 14.34° ± 0.2°, 18.18° ± 0.2°, 20.28° ± 0.2° and 21.82° ± 0.2° 2θ, as determined by using Cu-Kα radiation.

Preferably, the crystal form I of the compound as shown in formula (Ib) has an X-ray powder diffraction pattern further comprising characteristic diffraction peaks at 11.92° ± 0.2°, 12.74° ± 0.2° and 17.44° ± 0.2° 2θ, as determined by using Cu-Kα radiation.

More preferably, the crystal form I of the compound as shown in formula (Ib) has an X-ray powder diffraction pattern comprising characteristic diffraction peaks at 9.76° ± 0.2°, 11.26° ± 0.2°, 14.14° ± 0.2°, 17.04° ± 0.2°, 23.23° ± 0.2°, 24.06° ± 0.2°, 25.26° ± 0.2° and 26.42 ± 0.2° 2θ, as determined by using Cu-Kα radiation. In some embodiments, the crystal form I of the compound as shown in formula (Ib) of the present invention has an X-ray powder diffraction pattern substantially as shown in Figure 13-1 and/or Figure 13-2.

In some embodiments, the crystal form I of the compound as shown in formula (Ib) of the present invention has a differential scanning calorimetry (DSC) curve as shown in Figure 14 or a thermogravimetric analysis curve as shown in Figure 15.

The present invention further provides a crystal form II of the compound as shown in formula (Ib), wherein the crystal form II has an X-ray powder diffraction pattern comprising characteristic diffraction peaks at 5.12° ± 0.2°, 6.68° ± 0.2°, 16.50° ± 0.2° and 20.18° ± 0.2° 2θ, as determined by using Cu-Kα radiation.

Preferably, the crystal form II of the compound as shown in formula (Ib) of the present invention has an X-ray powder diffraction pattern further comprising characteristic diffraction peaks at 9.98° ± 0.2°, 13.44° ± 0.2°, 13.86° ± 0.2°, 15.34° ± 0.2°, 22.40° ± 0.2° and 23.12° ± 0.2° 2θ, as determined by using Cu-Kα radiation.

More preferably, the crystal form II of the compound as shown in formula (Ib) of the present invention has an X-ray powder diffraction pattern further comprising characteristic diffraction peaks at 15.76° ± 0.2°, 20.99° ± 0.2°, 24.14° ± 0.2° and 26.28° ± 0.2° 2θ, as determined by using Cu-Kα radiation.

In some embodiments, the crystal form II of the compound as shown in formula (Ib) of the present invention has an X-ray powder diffraction pattern substantially as shown in Figure 16-1 and/or Figure 16-2.

In some embodiments, the crystal form II of the compound as shown in formula (Ib) of the present invention has a differential scanning calorimetry (DSC) curve as shown in Figure 17 or a thermogravimetric analysis curve as shown in Figure 18.

The present invention further provides a crystal form III of the compound as shown in formula (Ib), wherein the crystal form III has an X-ray powder diffraction pattern comprising characteristic diffraction peaks at 7.48° ± 0.2°, 12.24° ± 0.2°, 20.50° ± 0.2° and 25.77° ± 0.2° 2θ, as determined by using Cu-Kα radiation.

Preferably, the crystal form III of the compound as shown in formula (Ib) of the present invention has an X-ray powder diffraction pattern further comprising characteristic diffraction peaks at 15.59° ± 0.2°, 18.74° ± 0.2° and 23.85° ± 0.2° 2θ, as determined by using Cu-Kα radiation.

More preferably, the crystal form III of the compound as shown in formula (Ib) of the present invention has an

X-ray powder diffraction pattern further comprising characteristic diffraction peaks at 14.95° ± 0.2°, 16.18° ± 0.2°, 16.70° ± 0.2°, 19.00° ± 0.2° and 21.39° ± 0.2° 2θ, as determined by using Cu-Kα radiation.

In some embodiments, the crystal form III of the compound as shown in formula (Ib) of the present invention has an X-ray powder diffraction pattern substantially as shown in Figure 19-1 and/or Figure 19-2.

In some embodiments, the crystal form III of the compound as shown in formula (Ib) of the present invention has a differential scanning calorimetry (DSC) curve as shown in Figure 20 or a thermogravimetric analysis curve as shown in Figure 21.

The present invention further provides a crystal form IV of the compound as shown in formula (Ib), wherein the crystal form IV has an X-ray powder diffraction pattern comprising characteristic diffraction peaks at 3.92° ± 0.2°, 8.7° ± 0.2°, 15.54° ± 0.2° and 18.22° ± 0.2° 2θ, as determined by using Cu-Kα radiation.

Preferably, the crystal form IV of the compound as shown in formula (Ib) of the present invention has an X-ray powder diffraction pattern further comprising characteristic diffraction peaks at 7.76° ± 0.2°, 10.48° ± 0.2°, 12.46° ± 0.2°, 16.79° ± 0.2°, 18.94° ± 0.2° and 19.67° ± 0.2° 2θ, as determined by using Cu-Kα radiation.

In some embodiments, the crystal form IV of the compound as shown in formula (Ib) of the present invention has an X-ray powder diffraction pattern substantially as shown in Figure 22-1 and/or Figure 22-2.

In some embodiments, the crystal form IV of the compound as shown in formula (Ib) of the present invention has a differential scanning calorimetry (DSC) curve as shown in Figure 23 or a thermogravimetric analysis curve as shown in Figure 24.

The present invention further provides an amorphous form of the compound as shown in formula (Ib), which, as determined by using Cu-Kα radiation, has an X-ray powder diffraction pattern substantially as shown in Figure 25.

In some embodiments, the amorphous form of the compound as shown in formula (Ib) of the present invention has a differential scanning calorimetry (DSC) curve as shown in Figure 26 or a thermogravimetric analysis curve as shown in Figure 27.

The present invention further provides a method for preparing a pharmaceutical salt of a compound as shown in formula (I), wherein the method comprises: a step of allowing the compound as shown in formula (I) and an acid to form a salt.

In some embodiments of the method for preparing the compound as shown in formula (I) of the present invention, the solvent used therein is selected from one or more of a $C_{1-6}$ halogenated alkane solvent, a $C_{2-6}$ ester solvent, a $C_{2-6}$ ether solvent, a $C_{1-6}$ alcohol solvent or water, preferably one or more of dichloromethane, 1,2-dichloroethane, ethyl acetate, methanol, ethanol, isopropanol, diethyl ether, tetrahydrofuran and water, more preferably one or more of dichloromethane, methanol, ethanol and water.

In some embodiments of the method for preparing the compound as shown in formula (I) of the present invention, the method comprises: a step of allowing the compound as shown in formula (Ia) and an acid to form a salt, wherein the acid is selected from maleic acid, fumaric acid, halogen acid (preferably hydrobromic acid and hydrochloric acid), sulfuric acid, phosphoric acid, L-tartaric acid, citric acid, L-malic acid, hippuric acid, D-glucuronic acid, glycolic acid, mucic acid, succinic acid, lactic acid, orotic acid, pamoic acid, glycine, alanine, arginine, cinnamic acid, benzoic acid, benzenesulfonic acid, p-toluenesulfonic acid, acetic acid, propionic acid, valeric acid, triphenylacetic acid, L-proline, ferulic acid, 2-hydroxyethanesulfonic acid, mandelic acid, nitric acid, methanesulfonic acid, malonic acid, gentisic acid, salicylic acid, oxalic acid or glutaric acid.

In some embodiments of the method for preparing the maleate of the compound as shown in formula (I) of the present invention, the method comprises: allowing the compound as shown in formula (Ia) and maleic acid to form a salt, and preparing a compound as shown in formula (II).

The present invention further provides a method for preparing a crystal form of a compound as shown in formula (Ia), (Ib) or (II), wherein the method comprises a step of preparing the compound as shown in formula (II), (Ia) or (Ib) in any crystal form or the compound as shown in formula (II), (Ia) or (Ib) in an amorphous form by means of recrystallization or slurrying, wherein a solvent for the recrystallization or slurrying is selected from one of or a mixed solvent of two or more of a $C_{2-6}$ ester solvent, a $C_{2-6}$ ether solvent, a $C_{1-6}$ alcohol solvent, a $C_{1-6}$ nitrile solvent, an alkane solvent and water. The solvent for the recrystallization or slurrying is preferably one of or a mixed solvent of two or more of ethyl acetate, isopropyl acetate, n-heptane, acetonitrile, tetrahydrofuran, trifluoroethanol, methanol, ethanol and water.

In some embodiments of the method for preparing the crystal form of the compound as shown in formula (Ia), (Ib) or (II) of the present invention, the recrystallization or slurrying is performed at a temperature of 4°C to 100°C, preferably room temperature to 90°C, more preferably 40°C to 90°C.

In some embodiments of the method for preparing the crystal form I of the compound as shown in formula (II) of the present invention, the method comprises steps of mixing the compound as shown in formula (II) with a suitable solvent to form a suspension, heating, stirring and slurrying the mixture, leaving the resulting product to stand for crystallization, and performing filtering and separation, wherein the solvent is preferably ethanol, and the slurrying is performed at a temperature of preferably 90°C.

In some embodiments of the method for preparing the crystal form III of the compound as shown in formula

(Ia) of the present invention, the method comprises steps of mixing the compound as shown in formula (Ia) in an amorphous form with a suitable solvent, heating, stirring and slurrying the mixture, and performing filtering and separation, wherein the solvent is preferably an acetonitrile/water mixed solvent, and the slurrying is performed at a temperature of preferably 40°C.

In another aspect, the present invention further provides a pharmaceutical composition, wherein the pharmaceutical composition contains a therapeutically effective amount of the compound or the crystal according to any one of the present invention as described above, and a pharmaceutically acceptable excipient.

In yet another aspect, the present invention further provides use of the pharmaceutical salt of the compound as shown in formula (I) or the crystals of the compounds as shown in formula (Ia), (Ib) and (II) and the pharmaceutical composition in the preparation of a drug for treating and/or preventing tumour.

In yet another aspect, the present invention further provides a method for treating and/or preventing tumour. The method comprises administering a therapeutically effective amount of the pharmaceutical salt of the compound as shown in formula (I) or the crystals of the compounds as shown in formula (Ia), (Ib) and (II) and the pharmaceutical composition.

It can be understood that the expression "preferably, ...has an X-ray powder diffraction pattern further comprising characteristic diffraction peaks at ... 2θ" or "more preferably, ...has an X-ray powder diffraction pattern further comprising characteristic diffraction peaks at ... 2θ" and other similar expressions of the present invention means that in addition to comprising characteristic diffraction peaks at 2θ positions described above, the X-ray powder diffraction pattern further comprises characteristic diffraction peaks at "2θ positions described below".

Other patterns substantially the same as the X-ray powder diffraction pattern, DSC pattern or TGA pattern disclosed in the present invention also fall within the scope of the present invention.

Unless stated to the contrary, the terms used in the description and claims have the following meanings.

The "therapeutically effective amount" means an amount that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

The "$IC_{50}$" refers to the half maximal inhibitory concentration, i.e., a concentration where half of the maximum inhibitory effect is achieved.

The "ether solvent" of the present invention refers to a chain compound or a cyclic compound containing an ether bond -O- and having 1 to 10 carbon atoms, and the specific examples thereof include, but are not limited to: tetrahydrofuran, diethyl ether, propylene glycol methyl ether, methyl tert-butyl ether, isopropyl ether or 1,4-dioxane.

The "alcohol solvent" of the present invention refers to a group derived from "$C_{1-6}$ alkyl" on which one or more hydrogen atoms are substituted with one or more "hydroxyl groups", wherein the "hydroxyl group" and "$C_{1-6}$ alkyl" are as defined above, and the specific examples thereof include, but are not limited to: methanol, ethanol, isopropanol, n-propanol, isopentanol or trifluoroethanol.

The "ester solvent" of the present invention refers to a combination of a lower organic acid containing 1-4 carbon atoms and a lower alcohol containing 1-6 carbon atoms, and the specific examples thereof include, but are not limited to: ethyl acetate, isopropyl acetate or butyl acetate.

The "ketone solvent" of the present invention refers to a compound in which a carbonyl group (-C(O)-) is connected to two hydrocarbon groups. According to the difference of hydrocarbon groups in molecules, ketones can be divided into aliphatic ketone, alicyclic ketone, aromatic ketone, saturated ketone and unsaturated ketone, and the specific examples thereof include, but are not limited to: acetone, acetophenone and 4-methyl-2-pentanone.

The "nitrile solvent" of the present invention refers to a group derived from "$C_{1-6}$ alkyl" on which one or more hydrogen atoms are substituted with one or more "cyano groups", wherein the "cyano group" and "$C_{1-6}$ alkyl" are as defined above, and the specific examples thereof include, but are not limited to: acetonitrile or propionitrile.

The "halogenated hydrocarbon solvent" of the present invention refers to a group derived from "$C_{1-6}$ alkyl" on which one or more hydrogen atoms are substituted with one or more "halogen atoms", wherein the "halogen atom" and "$C_{1-6}$ alkyl" are as defined above, and the specific examples thereof include, but are not limited to: dichloromethane, 1,2-dichloroethane, chloroform or carbon tetrachloride.

As used in the present invention, "the crystal of the present invention", "the crystal form of the present invention", "the polymorph of the present invention" and the like can be used interchangeably.

The "room temperature" of the present invention generally refers to 4°C to 30°C, preferably 20°C $\pm$ 5°C.

The structure of the crystal form of the present invention can be analysed by using various analytical techniques known to those skilled in the art, including but not limited to X-ray powder diffraction (XRD), differential scanning calorimetry (DSC) and/or thermogravimetric analysis (TGA). Thermogravimetric analysis (TGA) is also called as thermogravimetry (TG).

The X-ray powder diffractometer (XRD) used in the present invention is Bruker D8 Advance diffractometer, using Ka radiation (40 Kv, 40 mA) with a copper target wavelength of 1.54 Å, a θ-2θ goniometer, a Mo monochromator,

and an Lynxeye detector, using $Al_2O_3$ as a calibration material, Diffrac Plus XRD Commander as an acquisition software, and MDI Jade 6 as an analysis software; the method parameters involve: a non-reflective sample plate at 24.6 mm diameter × 1.0 mm thickness, manufactured by MTI corporation; a variable-temperature hot stage, manufactured by Shanghai Weitu Instrument Technology Development Co., Ltd., using a copper plate as a sample plate; a detection angle of 3-40° 2θ/3-30° 2θ (hot-stage XRPD); and a step length of 0.02° 2θ.

The differential scanning calorimeter (DSC) used in the present invention is TA Instruments Q200 DSC or DSC 3, operated under nitrogen protection with a gas flow rate of 50 mL/min.

The thermogravimetric analyser (TGA) used in the present invention is TA Instruments Q500 TGA or TGA/DSC $3^+$, operated under nitrogen protection with a gas flow rate of 40 mL/min or 50 mL/min.

The "2θ or 2θ angle" of the present invention refers to a diffraction angle, wherein θ is the Bragg angle in the unit of ° or degree, and the error range of the 2θ can be ± 0.3, ± 0.2 or ± 0.1.

It can be understood that the numerical values described and claimed in the present invention are approximate values. Changes in values may be attributed to device calibration, device errors, crystal purity, crystal size, sample size and other factors.

It can be understood that the crystal forms of the present invention are not limited to the characteristic patterns such as XRD, DSC and TGA which are completely identical to those described in the drawings disclosed in the present invention, and any crystal form having a characteristic pattern which is essentially or substantially the same as those described in the drawings falls within the scope of the present invention.

It can be understood that, as is well known in the field of differential scanning calorimetry (DSC), a melting peak height of a DSC curve depends on many factors related to sample preparation and geometric shapes of instruments, and a peak position is relatively insensitive to experiment details. Therefore, in some embodiments, the crystallized compounds of the present invention are characterized in that DSC patterns comprising characteristic peak positions have substantially the same properties as the DSC patterns provided in the drawings of the present invention, with an error tolerance of ± 3°C.

The crystal forms disclosed in the present invention can be prepared by the following common methods for preparing crystal forms:

1. a volatilization experiment, in which a clear solution of a sample is exposed to an atmosphere at various temperatures until the solvent is volatilized and removed;
2. a crystal slurrying experiment, in which a supersaturated solution of a sample (containing an undissolved solid) is stirred in different solvent systems at a certain temperature;
3. an anti-solvent experiment, in which a sample is dissolved in a good solvent, an anti-solvent is added to precipitate a solid, followed by brief stirring and immediate filtration;
4. a cooling crystallization experiment, in which a certain amount of samples is dissolved in a corresponding solvent at a high temperature, and the mixture is directly stirred at room temperature or a low temperature for crystallization;
5. a polymer template experiment, in which various polymer materials are added to a clear solution of a sample, and the resulting solution is exposed to an atmosphere at room temperature until the solvent is volatilized and removed;
6. a thermal method experiment, in which a sample is treated according to a certain thermal method under crystallization conditions and cooled to room temperature; and
7. a water vapor diffusion experiment, in which a sample is left in a certain humidity environment at room temperature.

Brief Description of the Drawings

Figure 1 is an XRD pattern of an amorphous form of compound 1.
Figure 2 is a DSC pattern of an amorphous form of compound 1.
Figure 3 is a TGA pattern of an amorphous form of compound 1.
Figure 4 is an XRD pattern of a crystal form I of compound 1.
Figure 5 is a DSC pattern of a crystal form I of compound 1.
Figure 6 is a TGA pattern of a crystal form I of compound 1.
Figure 7 is an XRD pattern of a crystal form II of compound 1.
Figure 8 is a DSC pattern of a crystal form II of compound 1.
Figure 9 is a TGA pattern of a crystal form II of compound 1.
Figure 10 is an XRD pattern of a crystal form III of compound 1.
Figure 11 is a DSC pattern of a crystal form III of compound 1.
Figure 12 is a TGA pattern of a crystal form III of compound 1.

Figure 13-1 is an XRD pattern of a crystal form I of compound 2.
Figure 13-2 is an XRD pattern of a crystal form I of compound 2.
Figure 14 is a DSC pattern of a crystal form I of compound 2.
Figure 15 is a TGA pattern of a crystal form I of compound 2.
Figure 16-1 is an XRD pattern of a crystal form II of compound 2.
Figure 16-2 is an XRD pattern of a crystal form II of compound 2.
Figure 17 is a DSC pattern of a crystal form II of compound 2.
Figure 18 is a TGA pattern of a crystal form II of compound 2.
Figure 19-1 is an XRD pattern of a crystal form III of compound 2.
Figure 19-2 is an XRD pattern of a crystal form III of compound 2.
Figure 20 is a DSC pattern of a crystal form III of compound 2.
Figure 21 is a TGA pattern of a crystal form III of compound 2.
Figure 22-1 is an XRD pattern of a crystal form IV of compound 2.
Figure 22-2 is an XRD pattern of a crystal form IV of compound 2.
Figure 23 is a DSC pattern of a crystal form IV of compound 2.
Figure 24 is a TGA pattern of a crystal form IV of compound 2.
Figure 25 is an XRD pattern of an amorphous form of compound 2.
Figure 26 is a DSC pattern of an amorphous form of compound 2.
Figure 27 is a TGA pattern of an amorphous form of compound 2.
Figure 28 is an XRD pattern of a crystal form I of compound 3.
Figure 29 is a DSC pattern of a crystal form I of compound 3.
Figure 30 is a TGA pattern of a crystal form I of compound 3.
Figure 31 is an XRD pattern of an amorphous form of compound 3.
Figure 32 is a DSC pattern of an amorphous form of compound 3.
Figure 33 is a TGA pattern of an amorphous form of compound 3.
Figure 34 is an XRD pattern of a crystal form II of compound 3.
Figure 35 is a DSC pattern of a crystal form II of compound 3.
Figure 36 is a TGA pattern of a crystal form II of compound 3.

## Detailed Description of Embodiments

The implementation process and beneficial effects of the present invention are described in detail below through specific examples, which are intended to help readers better understand the essence and characteristics of the present invention, and are not intended to limit the scope of implementation of the present invention.

### Example 1: Preparation of compound 1

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (**compound 1, also known as a compound as shown in formula (Ia)**)

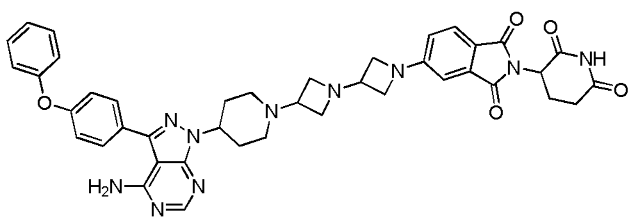

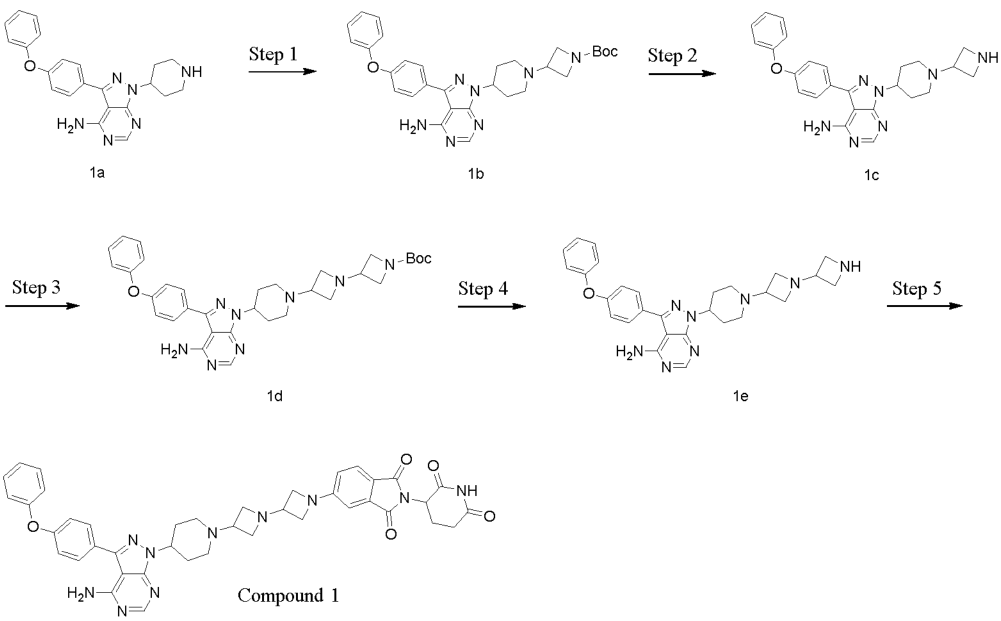


1a 1b 1c

1d 1e

Compound 1

Step 1:

tert-butyl 3-[4-[4-amino-3-(4-phenoxyphenyl)pyrazolo[3,4-d]pyrimidin-1-yl]-1-piperidyl]azetidine-1-carboxylate (1b)

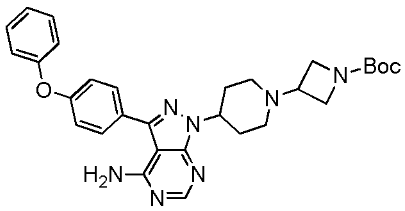

3-(4-phenoxyphenyl)-1-(piperidin-4-yl)-1H-pyrazolo[3,4-d]pyrimidin-4-amine (1a) (see J. Med. Chem. 2015, 58, 9625-9638 for a synthetic method) (11.0 g, 28.5 mmol) was dissolved in 100 mL of 1,2-dichloroethane. Tert-butyl 3-oxoazetidine-1-carboxylate (9.74 g, 56.9 mmol) and glacial acetic acid (3.42 g, 57.0 mmol) were sequentially added. Upon completion of the addition, the reaction was carried out at 65°C for 3 h. The reaction liquid was cooled to room temperature. Sodium triacetoxyborohydride (12.1 g, 57.1 mmol) was added. Upon completion of the addition, the reaction was carried out at room temperature overnight. The pH was adjusted to 9-10 by dropwise adding a saturated sodium bicarbonate solution to the reaction liquid. The resulting solution was concentrated under reduced pressure, and then the crude product was separated and purified by silica gel column chromatography (dichloromethane/methanol (v/v) = 100 : 0 to 19 : 1) to obtain tert-butyl 3-[4-[4-amino-3-(4-phenoxyphenyl)pyrazolo[3,4-d]pyrimidin-1-yl]-1-piperidyl]azetidine-1-carboxylate (1b) (7.20 g, yield: 47%).

LCMS m/z = 542.3 $[M+1]^+$

Step 2:

1-[1-(azetidin-3-yl)-4-piperidyl]-3-(4-phenoxyphenyl)pyrazolo[3,4-d]pyrimidin-4-amine (1c)

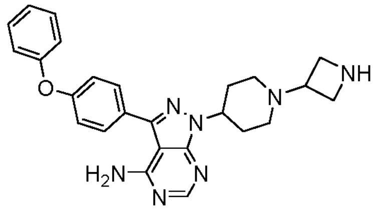

Tert-butyl 3-[4-[4-amino-3-(4-phenoxyphenyl)pyrazolo[3,4-d]pyrimidin-1-yl]-1-piperidyl]azetidine-1-carboxylate (1b) (7.20 g, 13.3 mmol) was dissolved in 15 mL of dichloromethane. 50 mL of 4 N ethyl acetate hydrochloride solution and 10 mL of anhydrous methanol were added. The resulting mixture was stirred at room temperature for 2 h. The reaction liquid was concentrated under reduced pressure, and then 20 mL of dichloromethane was added to the residue. The pH was adjusted to 9-10 by using a saturated sodium bicarbonate solution. Liquid separation was performed. The aqueous layer was extracted (100 mL × 3) with methanol/dichloromethane (v/v = 1 : 10), and the organic layers were combined, dried over anhydrous sodium sulfate and concentrated under reduced pressure to obtain 1-[1-(azetidin-3-yl)-4-piperidyl]-3-(4-phenoxyphenyl)pyrazolo[3,4-d]pyrimidin-4-amine (1c) (5.80 g, yield: 99%).
LCMS m/z = 442.2 $[M+1]^+$

Step 3:

tert-butyl 3-[3-[4-[4-amino-3-(4-phenoxyphenyl)pyrazolo[3,4-d]pyrimidin-1-yl]-1-piperidyl]azetidin-1-yl]azetidine-1-carboxylate (1d)

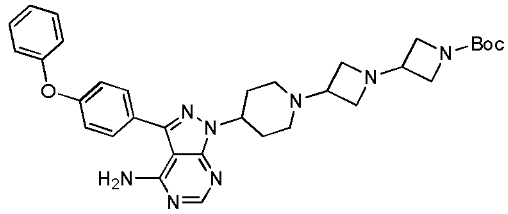

1-[1-(azetidin-3-yl)-4-piperidyl]-3-(4-phenoxyphenyl)pyrazolo[3,4-d]pyrimidin-4-amine (1c) (5.80 g, 13.1 mmol) was dissolved in 25 mL of 1,2-dichloroethane. Tert-butyl 3-oxoazetidine-1-carboxylate (4.50 g, 26.3 mmol) and glacial acetic acid (1.58 g, 26.3 mmol) were sequentially added. Upon completion of the addition, the reaction was carried out at 65°C for 3 h. The reaction liquid was cooled to room temperature. Sodium triacetoxyborohydride (5.57 g, 26.3 mmol) was added. Upon completion of the addition, the reaction was carried out at room temperature overnight. The pH was adjusted to 9-10 by dropwise adding a saturated sodium bicarbonate solution to the reaction liquid. The resulting solution was concentrated under reduced pressure, and then the crude product was separated and purified by silica gel column chromatography (dichloromethane/methanol (v/v) = 100 : 0 to 19 : 1) to obtain tert-butyl 3-[3-[4-[4-amino-3-(4-phenoxyphenyl)pyrazolo[3,4-d]pyrimidin-1-yl]-1-piperidyl]azetidin-1-yl]azetidine-1-carboxylate (1d) (3.60 g, yield: 46%).
LCMS m/z = 597.3 $[M+1]^+$

Step 4:

1-[1-[1-(azetidin-3-yl)azetidin-3-yl]-4-piperidyl]-3-(4-phenoxyphenyl)pyrazolo[3,4-d]pyrimidin-4-amine (1e)

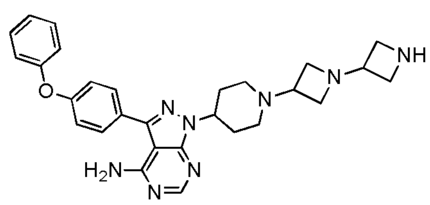

Tert-butyl 3-[3-[4-[4-amino-3-(4-phenoxyphenyl)pyrazolo[3,4-d]pyrimidin-1-yl]-1-piperidyl]azetidin-1-yl]azetidine-1-carboxylate (1d) (3.60 g, 6.03 mmol) was dissolved in 5 mL of dichloromethane. 5 mL of trifluoroacetic acid was added. The resulting mixture was stirred at room temperature for 2 h. The reaction liquid was concentrated under reduced pressure, and then 20 mL of dichloromethane was added to the residue. The pH was adjusted to 9-10 by using a saturated sodium bicarbonate solution. Liquid separation was performed. The aqueous layer was extracted with 100 mL of dichloromethane, and the organic layers were combined, dried over anhydrous sodium sulfate and concentrated under reduced pressure to obtain 1-[1-[1-(azetidin-3-yl)azetidin-3-yl]-4-piperidyl]-3-(4-phenoxyphenyl)pyrazolo[3,4-d]pyrimidin-4-amine (1e) as a crude product (3.0 g).
LCMS m/z = 497.3 $[M+1]^+$

Step 5:

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (compound 1)

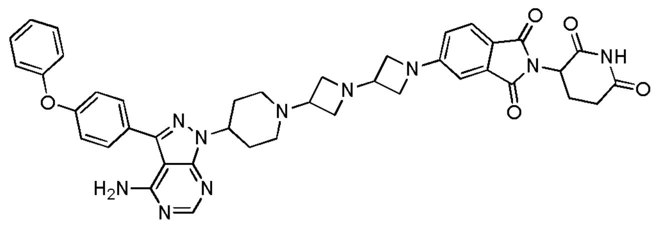

The above crude product of 1-[1-[1-(azetidin-3-yl)azetidin-3-yl]-4-piperidyl]-3-(4-phenoxyphenyl)pyrazolo[3,4-d]pyrimidin-4-amine (1e) (3.00 g) was dissolved in 15 mL of dimethylsulfoxide. 2-(2,6-dioxopiperidin-3-yl)-5-fluoroisoindoline-1,3-dione (see WO 2017197056 for a synthetic method) (2.00 g, 7.25 mmol) and diisopropylethylamine (3.90 g, 30.2 mmol) were sequentially added. Upon completion of the addition, the reaction was carried out at 90°C for 2 h. The reaction liquid was cooled to room temperature, and 10 mL of water was slowly added dropwise. Filtration was performed. The filter cake was dissolved in 50 mL of dichloromethane, and then washed with 15 mL of saturated sodium chloride solution. Liquid separation was performed. The organic layers were dried over anhydrous sodium sulfate and concentrated under reduced pressure, and then the crude product was separated and purified by silica gel column chromatography (dichloromethane/methanol (v/v) = 100 : 0 to 19 : 1), and the purified product solution obtained by column chromatography was directly concentrated under reduced pressure to obtain 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (compound 1) (2.90 g, the two-step yield calculated from compound 1d: 64%),
which is an amorphous form (yellow solid) of compound 1 as analysed by XRD, DSC and TGA. Reference was made to Figures 1, 2 and 3.

$^1$H NMR (400 MHz, $CDCl_3$) $\delta$ 9.99 (s, 1H), 8.39 (s, 1H), 7.72-7.57 (m, 3H), 7.45-7.32 (m, 2H), 7.21-7.11 (m, 3H), 7.10-7.04 (m, 2H), 6.78 (d, 1H), 6.52 (dd, 1H), 5.81 (brs, 2H), 4.92 (dd, 1H), 4.87-4.71 (m, 1H), 4.08-3.99 (m, 2H), 3.94-3.83 (m, 2H), 3.76-3.65 (m, 1H), 3.64-3.49 (m, 2H), 3.20-3.04 (m, 3H), 3.00-2.64 (m, 5H), 2.52-2.34 (m, 2H), 2.18-1.89 (m, 5H).
LCMS m/z = 377.3 $[M/2 + 1]^+$

**Example 2: Preparation of crystal form I of compound 1**

8 mL of ethyl acetate was added to the amorphous form (40 mg) of compound 1 prepared in example 1 to obtain a clear solution. The solution was exposed to an atmosphere at 40°C and volatilized to obtain a crystal form I (yellow solid) of compound 1. The crystal form I of compound 1 was characterized by XRD, DSC and TGA. Reference was made to Figures 4, 5 and 6.

**Example 3: Preparation of crystal form II of compound 1**

6 mL of ethanol was added to the amorphous form (200 mg) of compound 1 prepared in example 1. Crystal slurrying was performed at room temperature for 3 days. Centrifugation was performed, and then the solid was dried under vacuum overnight at room temperature to obtain a crystal form II (yellow solid) of compound 1. The crystal form

II of compound 1 was characterized by XRD, DSC and TGA. Reference was made to Figures 7, 8 and 9.

**Example 4: Preparation of crystal form III of compound 1**

6 mL of acetonitrile and 6 mL of water were added to the amorphous form (400 mg) of compound 1 prepared in example 1. The resulting solution was stirred at 40°C for 72 h, and suction filtration was performed. The filter cake was collected and dried under vacuum overnight at 40°C to obtain a crystal form III (yellow solid) of compound 1. The crystal form III of compound 1 was characterized by XRD, DSC and TGA. Reference was made to Figures 10, 11 and 12.

**Example 5: Preparation of compound 2**

5-(3-(4-(4-amino-3-(4-phenoxyphenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione **(compound 2, which also known as a compound as shown in formula (Ib))**

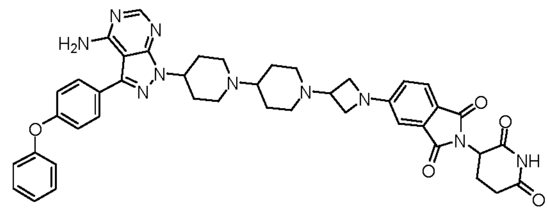

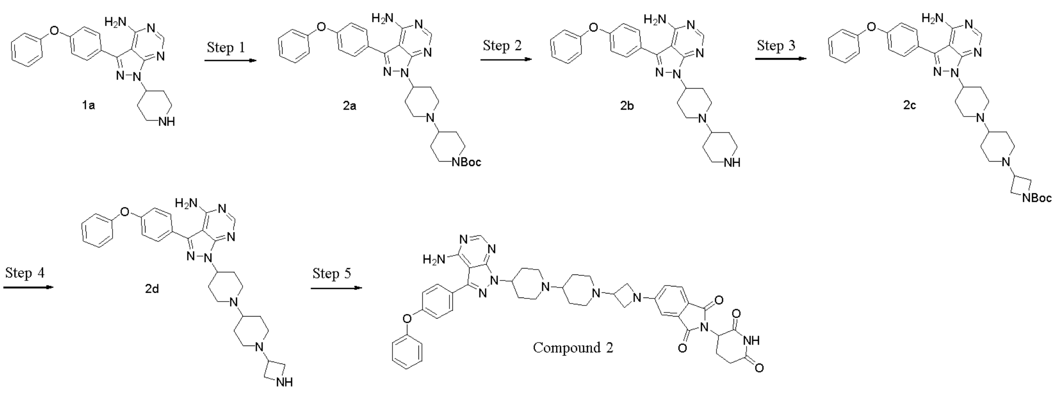


Step 1:

tert-butyl 4-[4-[4-amino-3-(4-phenoxyphenyl)pyrazolo[3,4-d]pyrimidin-1-yl]-1-piperidyl]piperidine-1-carboxylate (2a)

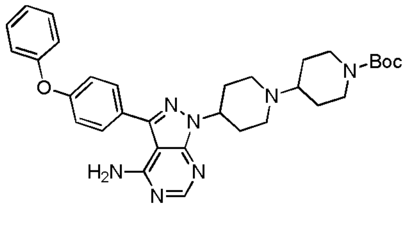

3-(4-phenoxyphenyl)-1-(piperidin-4-yl)-1H-pyrazolo[3,4-d]pyrimidin-4-amine (1a) (see J. Med. Chem. 2015, 58, 9625-9638 for a synthetic method) (10.42 g, 26.97 mmol) was dissolved in 200 mL of 1,2-dichloroethane. Tert-butyl 4-oxopiperidine-1-carboxylate (13.43 g, 67.42 mmol) and glacial acetic acid (4.2 g, 67.42 mmol) were sequentially added. The resulting solution was heated to 65°C, stirred for 2 h and cooled to room temperature, and sodium triacetoxyborohydride (34.29 g, 161.79 mmol) was added. The reaction was carried out under stirring at room temperature for 16 h. After TLC showed the reaction was completed, the reaction liquid was left to stand. 50 mL of 2 M aqueous solution of sodium hydroxide was added. The pH was adjusted to 8-9 by using a saturated sodium bicarbonate solution. The

resulting mixture was allowed to stand for layer separation. The aqueous phase was extracted with dichloromethane (200 mL × 3), and the organic phases were combined, washed once with a saturated brine (300 mL), dried over anhydrous sodium sulfate, filtered and concentrated under reduced pressure to obtain a crude product. The crude product was purified by column chromatography (200-300 mesh silica gel, dichloromethane/methanol (v/v) = 100/1 to 15/1) to obtain tert-butyl 4-[4-[4-amino-3-(4-phenoxyphenyl)pyrazolo[3,4-d]pyrimidin-1-yl]-1-piperidyl]piperidine-1-carboxylate (2a) (10.72 g, yield: 70%).

Step 2:

3-(4-phenoxyphenyl)-1-[1-(4-piperidyl)-4-piperidyl]pyrazolo[3,4-d]pyrimidin-4-amine (2b)

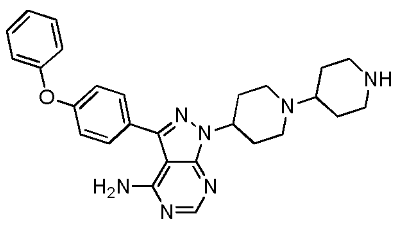

Tert-butyl 4-[4-[4-amino-3-(4-phenoxyphenyl)pyrazolo[3,4-d]pyrimidin-1-yl]-1-piperidyl]piperidine-1-carboxylate (2a) (45 g, 92.48 mmol) was added to a reaction flask. 410 mL of dichloromethane was added and dissolved under stirring, and then 80 mL of trifluoroacetic acid was added. The reaction was carried out under stirring at room temperature overnight. After the reaction was completed, the reaction liquid was concentrated under reduced pressure to obtain an oil. 500 mL of dichloromethane was added. The pH was adjusted to 10 by slowly adding a 2 mol/L sodium hydroxide solution dropwise under stirring. Liquid separation was performed. The aqueous phase was extracted with dichloromethane (400 mL × 3), and the organic phases were combined. The organic layer was washed with a 15% aqueous solution of sodium chloride (500 mL), dried over anhydrous sodium sulfate, filtered and concentrated under reduced pressure to obtain 3-(4-phenoxyphenyl)-1-[1-(4-piperidyl)-4-piperidyl]pyrazolo[3,4-d]pyrimidin-4-amine (2b) (29.3 g, yield: 82%).

Step 3:

tert-butyl 3-(4-(4-amino-3-(4-phenoxyphenyl)-1H-pyrazolo[3,4-d]pyrimidin-1-yl)-[1,4'-bipiperidin]-1'-yl)azetidine-1-carboxylate (2c)

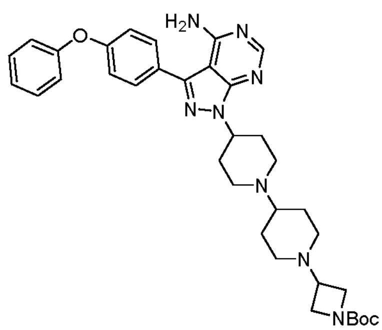

3-(4-phenoxyphenyl)-1-[1-(4-piperidyl)-4-piperidyl]pyrazolo[3,4-d]pyrimidin-4-amine (2b) (29.3 g, 0.076 mol) was added to 1,2-dichloroethane (0.5 L). 1-Boc-3-azetidinone (37.7 g, 0.189 mol), acetic acid (11.4 g, 0.189 mol) and anhydrous sodium sulfate (30 g) were sequentially added. Upon completion of the addition, sodium triacetoxyborohydride (96.3 g, 0.45 mol) was slowly added. The reaction was carried out under stirring at room temperature for 2 h. The reaction liquid was poured into a 2 L plastic beaker. Ice was added, and the pH was adjusted to 12-13 by using a 2M aqueous solution of sodium hydroxide. The resulting mixture was allowed to stand for layer separation. The aqueous phase was extracted with dichloromethane (400 mL × 3), and the organic phases were combined, washed with a saturated brine

(600 mL), dried over anhydrous sodium sulfate, filtered and concentrated under reduced pressure to obtain a crude product. The crude product was purified by column chromatography (200-300 mesh silica gel, dichloromethane/methanol (v/v) = 100/0 to 12/1) to obtain tert-butyl 3-(4-(4-amino-3-(4-phenoxyphenyl)-1H-pyrazolo[3,4-d]pyrimidin-1-yl)-[1,4'-bipiperidin]-1'-yl)azetidine-1-carboxylate (2c) (35 g, yield: 81%).

Step 4:

1-(1'-(azetidin-3-yl)-[1,4'-bipiperidin]-4-yl)-3-(4-phenoxyphenyl)-1H-pyrazolo[3,4-d]pyrimidin-4-amine (2d)

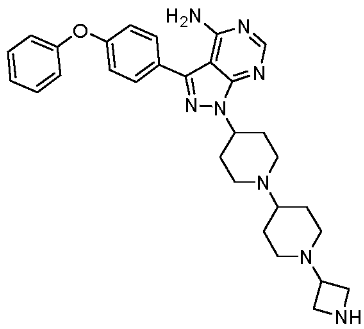

Tert-butyl 3-(4-(4-amino-3-(4-phenoxyphenyl)-1H-pyrazolo[3,4-d]pyrimidin-1-yl)-[1,4'-bipiperidin]-1'-yl)azetidine-1-carboxylate (2c) (25 g, 0.04 mmol) was added to a reaction flask. 125 mL of dichloromethane was added, and 50 mL of trifluoroacetic acid was slowly added dropwise. Upon completion of the addition, the reaction was carried out under stirring at room temperature for 2 h. After the reaction was completed, the reaction liquid was concentrated under reduced pressure to obtain an oil. 200 mL of methyl tert-butyl ether was added under stirring, with a white solid gradually precipitated. Stirring was performed for crystallization at room temperature for 1 h. The resulting product was filtered and concentrated under reduced pressure to obtain 1-(1'-(azetidin-3-yl)-[1,4'-bipiperidin]-4-yl)-3-(4-phenoxyphenyl)-1H-pyrazolo[3,4-d]pyrimidin-4-amine trifluoroacetate (2d) (50 g, yield: 99%).

Step 5:

5-(3-(4-(4-amino-3-(4-phenoxyphenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 2)

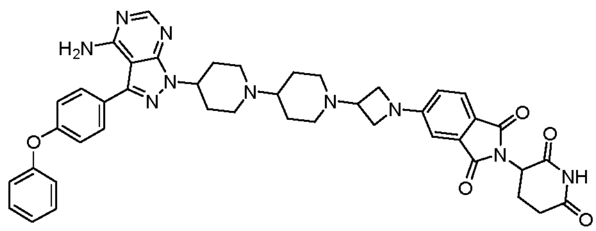

2-(2,6-dioxopiperidin-3-yl)-5-fluoroisoindoline-1,3-dione (see WO 2017197056 for a synthetic method) (10.3 g, 0.037 mmol), N,N'-diisopropylethylamine (40 g, 0.31 mmol) and dimethylsulfoxide (0.2 L) were sequentially added to 1-(1'-(azetidin-3-yl)-[1,4'-bipiperidin]-4-yl)-3-(4-phenoxyphenyl)-1H-pyrazolo[3,4-d]pyrimidin-4-amine trifluoroacetate (2d) (48 g, 0.031 mmol). The reaction was carried out under stirring at 120°C for 3 h. The reaction liquid was cooled to room temperature with ice water, and water (0.2 L) was added to the reaction liquid under stirring, with a large amount of solids precipitated. The resulting mixture was continuously stirred for 30 min, filtered and dried with suction. The filter cake was dissolved in 0.5 L of dichloromethane under stirring, washed with concentrated ammonia water ( 200 mL × 3), dried over anhydrous sodium sulfate and filtered. The filtrate was concentrated under reduced pressure, and the residue was separated and purified by silica gel column chromatography (dichloromethane/methanol (v/v) = 100/0 to 94/6) to collect the product. Ethyl acetate (0.28 L) was added to the above product obtained by column chromatography.

The resulting mixture was slurried under stirring for 20 h and filtered. The filter cake was dried under vacuum at 45°C for 92 h to obtain 5-(3-(4-(4-amino-3-(4-phenoxyphenyl)-1H-pyrazolo[3,4-d]pyrimidin-1-yl)-[1,4'-bipiperidin]-1'-yl)azetidin-1-yl)-2-(2,6-dioxopiperidin-3-yl)isoindoline-1,3-dione (compound 2) (17 g, yield: 72%), which is a crystal form I (yellow solid) of compound 2 as analysed by XRD, DSC and TGA. Reference was made to Figures 13-1, 13-2, 14 and 15.

$^1H$ NMR (400 MHz, $CDCl_3$) δ 10.22 (brs, 1H), 8.39 (s, 1H), 7.67-7.60 (m, 3H), 7.42-7.34 (m, 2H), 7.19-7.10 (m, 3H), 7.10-7.04 (m, 2H), 6.78 (d, 1H), 6.51 (dd, 1H), 5.89 (brs, 2H), 4.96-4.88 (m, 1H), 4.83-4.70 (m, 1H), 4.14-4.04 (m, 2H), 3.92-3.84 (m, 2H), 3.39-3.30 (m, 1H), 3.18-3.04 (m, 2H), 3.00-2.91 (m, 2H), 2.90-2.65 (m, 3H), 2.56-2.32 (m, 5H), 2.16-2.01 (m, 3H), 2.01-1.84 (m, 4H), 1.73-1.59 (m, 2H).

LC-MS m/z = 781.4 $[M+1]^+$.

**Example 6: Preparation of crystal form II of compound 2**

2.8 mL of tetrahydrofuran and 1.4 mL of water were added to the crystal form I (210 mg) of compound 2. The resulting mixture was heated and stirred at 60°C to obtain a clear solution. The solution was stirred at 4°C overnight, with a solid precipitated. Suction filtration was performed under reduced pressure. The resulting product was dried under vacuum at room temperature for about 3 h to obtain a crystal form II (yellow solid) of compound 2. The crystal form II of compound 2 was characterized by XRD, DSC and TGA. Reference was made to Figures 16-1, 16-2, 17 and 18.

**Example 7: Preparation of crystal form III of compound 2**

14 mL of water and 1.4 mL of tetrahydrofuran were added to the crystal form I (210 mg) of compound 2. Crystal slurrying was performed at 4°C for 3 days. The resulting product was filtered with suction to dryness under reduced pressure to obtain a crystal form III (yellow solid) of compound 2. The crystal form III of compound 2 was characterized by XRD, DSC and TGA. Reference was made to Figures 19-1, 19-2, 20 and 21.

**Example 8: Preparation of crystal form IV of compound 2**

1 mL of isopropyl acetate and 1 mL of n-heptane were added to the crystal form I (30 mg) of compound 2. Crystal slurrying was performed at room temperature for 3 days. Centrifugation was performed, and then the sample was dried under vacuum at room temperature for about 5 h to obtain a crystal form IV (yellow solid) of compound 2. The crystal form IV of compound 2 was characterized by XRD, DSC and TGA. Reference was made to Figures 22-1, 22-2, 23 and 24.

**Example 9: Preparation of amorphous form of compound 2**

5 mL of dichloromethane was added to the crystal form I (400 mg) of compound 2. The resulting mixture was heated to obtain a clear solution. Filtration was performed. The filtrate was concentrated to dryness under reduced pressure at 40°C to obtain an amorphous form (yellow solid) of compound 2. The amorphous form of compound 2 was characterized by XRD, DSC and TGA. Reference was made to Figures 25, 26 and 27.

**Example 10: Preparation of compound 3**

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione dimaleate (**compound 3, also known as a compound as shown in formula (II)**)

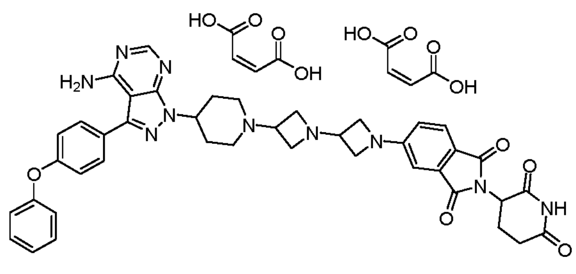

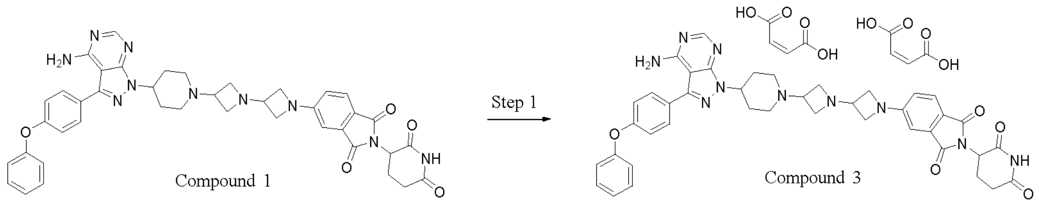


Dichloromethane (10 mL) was added to 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (compound 1) (1.0 g, 1.33 mmol). The mixture was stirred at room temperature until a clear solution was obtained. A solution of maleic acid (0.309 g, 2.66 mmol) in methanol (1 mL) was added dropwise, during which a solid was gradually precipitated. The resulting mixture was continuously stirred at room temperature for 3 h, and then suction filtration was performed under reduced pressure. The filter cake was washed with 10 mL of dichloromethane, collected and then concentrated under reduced pressure at 40°C to remove the residual solvent, so as to obtain 0.96 g of a crude product. 20 mL of ethanol was added to the above crude product. The resulting mixture was heated at 90°C and slurried under stirring for 0.5 h. The suspension was cooled to room temperature for crystallization for 2 h. Suction filtration was performed under reduced pressure. The filter cake was washed with 10 mL of ethanol, collected and then concentrated under reduced pressure at 40°C to remove the residual solvent, so as to obtain 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione dimaleate (compound 3) (0.62 g, yield: 47%),
which is a crystal form I (yellow solid) of compound 3 as analysed by XRD, DSC and TGA. Reference was made to Figures 28, 29 and 30.

$^1H$ NMR (400 MHz, DMSO-$d_6$) δ 11.06 (s, 1H), 8.27 (s, 1H), 7.72-7.63 (m, 3H), 7.48-7.41 (m, 2H), 7.24-7.09 (m, 5H), 6.86 (d, 1H), 6.72 (dd, 1H), 6.15 (s, 4H), 5.06 (dd, 1H), 5.01-4.86 (m, 1H), 4.24-4.12 (m, 2H), 4.11-3.93 (m, 3H), 3.92-3.79 (m, 2H), 3.77-3.59 (m, 3H), 3.37-3.22 (m, 2H), 2.98-2.68 (m, 3H), 2.65-2.51 (m, 2H), 2.46-2.31 (m, 2H), 2.18-2.06 (m, 2H), 2.06-1.96 (m, 1H).

**Example 11: Preparation of amorphous form of compound 3**

50 mL of trifluoroethanol and 50 mL of dichloromethane were sequentially added to compound 3 (300 mg) (crystal form I) to obtain a clear solution. The solution was concentrated to dryness under reduced pressure at 40°C to obtain an amorphous form (yellow solid) of compound 3,
which is an amorphous form of compound 3 as analysed by XRD, DSC and TGA. Reference was made to Figures 31, 32 and 33.

**Example 12: Preparation of crystal form II of compound 3**

6.0 mL of methanol and 4.0 mL of water were added to compound 3 (150 mg) (crystal form I). The resulting mixture was placed in a water bath at 70°C to obtain a clear solution. The solution was stirred at 4°C overnight, with a solid precipitated. Suction filtration was performed under reduced pressure. The resulting product was dried under vacuum at room temperature overnight to obtain a crystal form II (yellow solid) of compound 3,
which is a crystal form II of compound 3 as analysed by XRD, DSC and TGA. Reference was made to Figures 34, 35 and 36.

**Example 13:**

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione L-malate (**compound 4**)

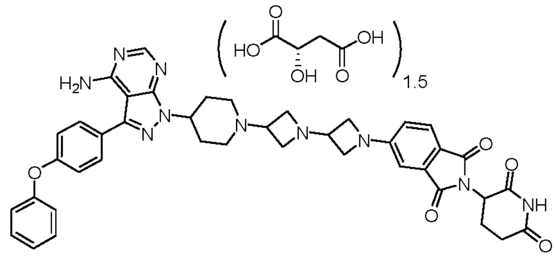

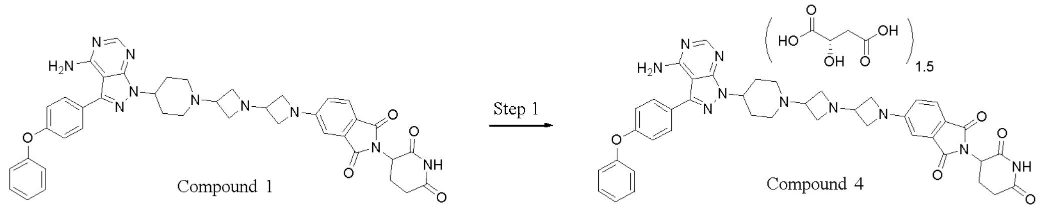


Dichloromethane (8 mL) was added to 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (compound 1) (0.40 g, 0.531 mmol). The mixture was stirred at room temperature until a clear solution was obtained. A solution of L-malic acid (0.28 g, 2.09 mmol) in methanol (0.5 mL) was added dropwise, during which a viscous solid was gradually precipitated. The resulting mixture was continuously stirred at room temperature for 2 h, and then concentrated under reduced pressure at 40°C. Ethanol (10 mL) was added to the residue. The resulting mixture was heated to 90°C, stirred for 1 h, then cooled to room temperature and stirred for 2 h. Suction filtration was performed. The filter cake was dried under vacuum at 50°C for 18 h to obtain 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione L-malate (compound 4) (yellow solid) (0.41 g, yield: 81%).

$^1$H NMR (400 MHz, DMSO-$d_6$) δ 11.05 (s, 1H), 8.24 (s, 1H), 7.70-7.61 (m, 3H), 7.48-7.40 (m, 2H), 7.23-7.08 (m, 5H), 6.80 (d, 1H), 6.67 (dd, 1H), 5.05 (dd, 1H), 4.77-4.64 (m, 1H), 4.21 (dd, 1.5H), 4.11-4.02 (m, 2H), 3.88-3.80 (m, 2H), 3.76-3.66 (m, 1H), 3.55-3.46 (m, 2H), 3.16-3.04 (m, 3H), 3.00-2.80 (m, 3H), 2.65-2.52 (m, 3H), 2.49-2.38 (m, 2H), 2.31-2.07 (m, 4H), 2.06-1.89 (m, 3H).

**Example 14:**

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione citrate (**compound 5**)

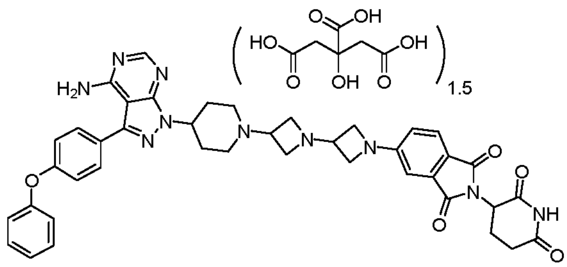

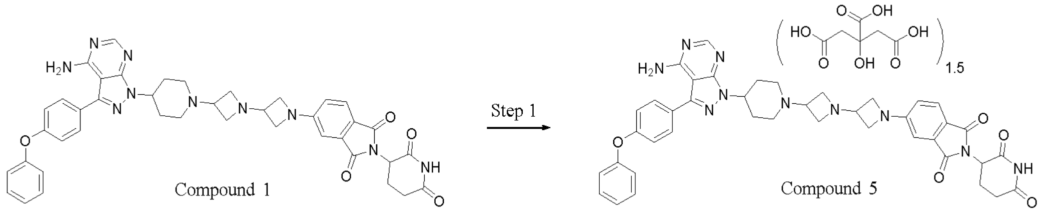


Dichloromethane (8 mL) was added to 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (compound 1) (0.40 g, 0.531 mmol). The mixture was stirred at room temperature until a clear solution was obtained. A solution of citric acid monohydrate

(0.45 g, 2.14 mmol) in methanol (0.7 mL) was added dropwise, during which a viscous solid was gradually precipitated. The resulting mixture was continuously stirred at room temperature for 2 h, and then concentrated under reduced pressure at 40°C. Ethanol (10 mL) was added to the residue. The resulting mixture was heated to 90°C, stirred for 1 h, then cooled to room temperature and stirred for 2 h. Suction filtration was performed. The filter cake was dried under vacuum at 50°C for 18 h to obtain 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione citrate (compound 5) (yellow solid) (0.48 g, yield: 87%).

$^1$H NMR (400 MHz, DMSO-$d_6$) δ 11.06 (s, 1H), 8.25 (s, 1H), 7.70-7.63 (m, 3H), 7.48-7.40 (m, 2H), 7.23-7.09 (m, 5H), 6.82 (d, 1H), 6.68 (dd, 1H), 5.06 (dd, 1H), 4.83-4.71 (m, 1H), 4.15-4.04 (m, 2H), 3.91-3.74 (m, 3H), 3.64-3.53 (m, 2H), 3.33-3.20 (m, 3H), 3.09-2.96 (m, 2H), 2.94-2.82 (m, 1H), 2.79-2.53 (m, 8H), 2.40-2.20 (m, 4H), 2.07-1.91 (m, 3H).

**Example 15:**

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione fumarate (**compound 6**)

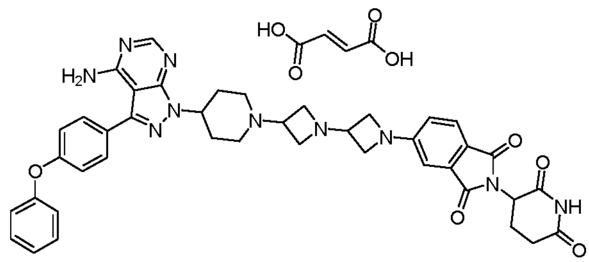

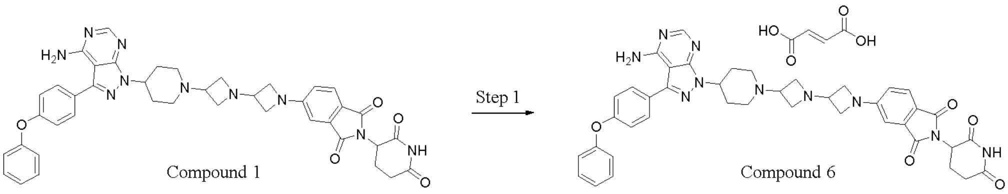


5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (compound 1) (0.500 g, 0.664 mmol) was dissolved in dichloromethane (5 mL). Anhydrous methanol (1.25 mL) and fumaric acid (0.616 g, 5.31 mmol) were sequentially added. The resulting mixture was stirred at room temperature for 7 h and then filtered. The filter cake was collected, and anhydrous ethanol (15 mL) was added to the filter cake. The resulting product was heated to 80°C, stirred for 2 h, cooled to room temperature and filtered. The filter cake was dried under vacuum at 50°C for 16 h to obtain 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione fumarate (compound 6) (yellow solid) (0.400 g, yield: 69%).

$^1$H NMR (400 MHz, DMSO-$d_6$) δ 11.05 (s, 1H), 8.23 (s, 1H), 7.70-7.60 (m, 3H), 7.47-7.40 (m, 2H), 7.23-7.09 (m, 5H), 6.79 (d, 1H), 6.66 (dd, 1H), 6.62 (s, 2H), 5.05 (dd, 1H), 4.74-4.62 (m, 1H), 4.09-4.01 (m, 2H), 3.86-3.78 (m, 2H), 3.71-3.62 (m, 1H), 3.50-3.40 (m, 2H), 3.08-2.97 (m, 3H), 2.94-2.82 (m, 3H), 2.64-2.46 (m, 2H), 2.29-2.14 (m, 2H), 2.12-1.86 (m, 5H).

**Example 16:**

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione L-tartrate (**compound 7**)

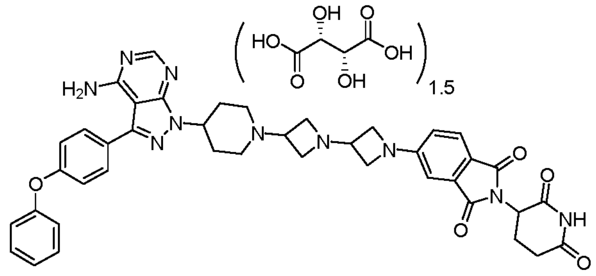

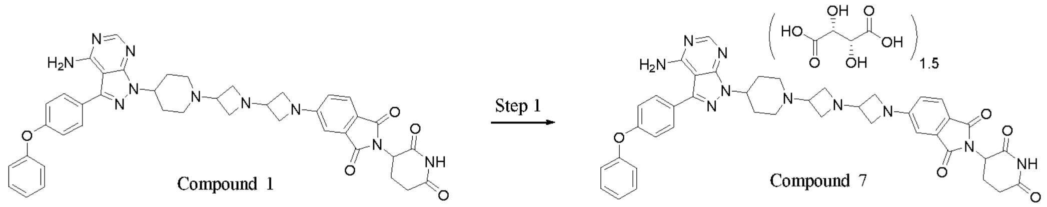


5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (compound 1) (0.500 g, 0.664 mmol) was dissolved in dichloromethane (5 mL). Anhydrous methanol (0.75 ml) and L-tartaric acid (0.399 g, 2.66 mmol) were sequentially added. The resulting mixture was stirred at room temperature for 4 h and then filtered. The filter cake was collected, and anhydrous ethanol (15 mL) was added to the filter cake. The resulting product was heated to 80°C, stirred for 2 h, cooled to room temperature and filtered. The filter cake was dried under vacuum at 50°C for 16 h to obtain 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione L-tartrate (compound 7) (yellow solid) (0.510 g, yield: 79%).

$^1H$ NMR (400 MHz, DMSO-$d_6$) $\delta$ 11.06 (s, 1H), 8.24 (s, 1H), 7.70-7.61 (m, 3H), 7.48-7.40 (m, 2H), 7.23-7.09 (m, 5H), 6.80 (d, 1H), 6.67 (dd, 1H), 5.06 (dd, 1H), 4.78-4.65 (m, 1H), 4.28 (s, 3H), 4.11-4.02 (m, 2H), 3.88-3.79 (m, 2H), 3.76-3.66 (m, 1H), 3.56-3.40 (m, 2H), 3.18-3.04 (m, 3H), 2.98-2.80 (m, 3H), 2.65-2.46 (m, 2H), 2.35-1.87 (m, 7H).

## Example 17:

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione salicylate (**compound 8**)

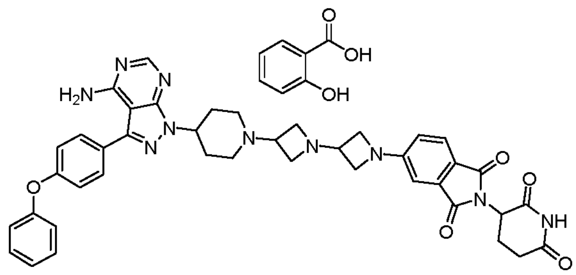

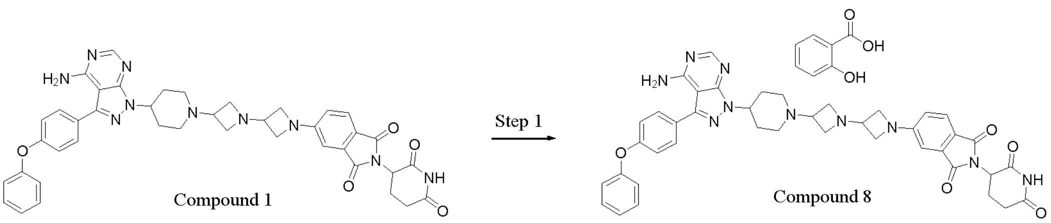


10 mL of an ethanol/water (v/v = 4 : 1) mixed solvent was added to 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (compound 1) (0.400 g, 0.531 mmol). The mixture was heated to 80°C until complete dissolution. Salicylic acid (0.293 g, 2.12 mmol) was added. The resulting mixture was continuously stirred at 80°C until a clear solution was obtained.

The solution was cooled to 60°C, stirred for 1 h, then cooled to room temperature and stirred for 2 h for crystallization. Suction filtration was performed. The filter cake was collected, and 10 mL of an ethanol/water (v/v = 4/1) mixed solvent was added to the filter cake. The resulting product was heated to 80°C, stirred until the solid was dissolved, then cooled to room temperature and stirred for 2 h for crystallization. Filtration was performed. The filter cake was collected and dried under vacuum at 50°C for 16 h to obtain 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione salicylate (compound 8) (yellow solid) (0.140 g, yield: 30%).

$^1$H NMR (400 MHz, DMSO-$d_6$) δ 11.05 (s, 1H), 8.24 (s, 1H), 7.78-7.59 (m, 4H), 7.48-7.40 (m, 2H), 7.39-7.31 (m, 1H), 7.24-7.08 (m, 5H), 6.85-6.74 (m, 3H), 6.65 (dd, 1H), 5.06 (dd, 1H), 4.87-4.73 (m, 1H), 4.15-4.04 (m, 2H), 3.95-3.77 (m, 3H), 3.70-3.58 (m, 2H), 3.41-3.28 (m, 3H), 3.14-3.01 (m, 2H), 2.95-2.81 (m, 1H), 2.65-2.46 (m, 2H), 2.45-2.22 (m, 4H), 2.07-1.94 (m, 3H).

**Example 18:**

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione oxalate (**compound 9**)

5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione (compound 1) (0.500 g, 0.664 mmol) was dissolved in dichloromethane (10 mL). A solution of oxalic acid dihydrate (0.335 g, 2.66 mmol) in methanol (2 mL) was slowly added dropwise. The resulting mixture was stirred at room temperature for 4 h and then filtered. The filter cake was collected, and anhydrous ethanol (12 mL) was added to the filter cake. The resulting product was heated to 80°C, stirred for 2 h, and cooled to room temperature. Filtration was performed. The filter cake was collected and dried under vacuum at 50°C for 16 h to obtain 5-[3-[3-[4-[4-amino-3-(4-phenoxyphenyl)pyrazolo[3,4-d]pyrimidin-1-yl]-1-piperidyl]azetidin-1-yl]azetidin-1-yl]-2-(2,6-dioxo-3-piperidyl)isoindoline-1,3-dione oxalate (compound 9) (yellow solid) (0.480 g).

$^1$H NMR (400 MHz, DMSO-$d_6$) δ 11.06 (s, 1H), 8.26 (s, 1H), 7.71-7.62 (m, 3H), 7.49-7.39 (m, 2H), 7.24-7.09 (m, 5H), 6.84 (d, 1H), 6.70 (dd, 1H), 5.06 (dd, 1H), 4.95-4.83 (m, 1H), 4.21-4.09 (m, 2H), 4.06-3.91 (m, 3H), 3.88-3.74 (m, 2H), 3.70-3.55 (m, 3H), 3.30-3.16 (m, 2H), 2.96-2.81 (m, 1H), 2.74-2.46 (m, 4H), 2.44-2.28 (m, 2H), 2.15-1.94 (m, 3H).

**Test example**

**1. XRD tests of compounds 1, 2 and 3**

The compounds of the present invention were subjected to X-ray powder diffraction tests according to the following methods. The test parameters of the amorphous form and crystal forms I, II and III of compound 1 were as shown in Table 1-1, the test parameters of the crystal forms I, II, III and IV and amorphous form of compound 2 were as shown in Table 1-1, and the test parameters of the crystal forms I and II and amorphous form of compound 3 were as shown in Table 1-2. The test results were as shown in Figures 1, 4, 7, 10, 13-1, 13-2, 16-1, 16-2, 19-1, 19-2, 22-1, 22-2, 25, 28, 31 and 34.

Table 1-1 Test parameters of XRD

| **Device name** | **X-ray powder diffractometer (XRPD) and hot-stage XRPD** | |
|---|---|---|
| Instrument | Model | Bruker D8 Advance Diffractometer |
| | Number | LY-01-034 |
| | Technical indicator | Kα radiation (40 kV, 40 mA) with a copper target wavelength of 1.54 Å, a θ-2θ goniometer, a Mo monochromator and a Lynxeye detector |
| | Acquisition software | Diffrac Plus XRD Commander |
| | Calibration material | Corundum ($Al_2O_3$) |
| | Analysis software | MDI Jade |

(continued)

| Device name | X-ray powder diffractometer (XRPD) and hot-stage XRPD | | |
|---|---|---|---|
| Accessory | Non-reflective sample plate | Specification | 24.6 mm diameter × 1.0 mm thickness |
| | | Manufacturer | MTI corporation |
| | Variable-temperature hot stage | Manufacturer | Shanghai Weitu Instrument Technology Development Co., Ltd. |
| | | Material of sample plate | Copper plate |
| Parameter | Detection angle | 3-40° 2θ/3-30° 20 (hot-stage XRPD) | |
| | Step length | 0.02° 2θ | |
| | Speed | 0.2 $s.step^{-1}$ | |
| | Sample size to be detected | > 2 mg | |
| | Note | Unless otherwise specified, samples are not subjected to grinding before detection | |

Table 1-2 Test parameters of XRD

| Device name | X-ray powder diffractometer (XRPD) and hot-stage XRPD | | |
|---|---|---|---|
| Instrument | Model | Bruker D8 Advance Diffractometer | |
| | Number | LY-01-034 | |
| | Technical indicator | Kα radiation (40 kV, 40 mA) with a copper target wavelength of 1.54 Å, a θ-2θ goniometer, nickel filtration, and a Lynxeye detector | |
| | Acquisition software | Diffrac Plus XRD Commander | |
| | Calibration material | Corundum ($Al_2O_3$) | |
| | Analysis software | MDI Jade | |
| Accessory | Non-reflective sample plate | Specification | 24.6 mm diameter × 1.0 mm thickness |
| | | Manufacturer | MTI corporation |
| | Variable-temperature hot stage | Manufacturer | Shanghai Weitu Instrument Technology Development Co., Ltd. |
| | | Material of sample plate | Copper plate |
| Parameter | Detection angle | 3-40° 2θ/3-30° 20 (hot-stage XRPD) | |
| | Step length | 0.02° 2θ | |
| | Speed | 0.2 $s.step^{-1}$ | |
| | Sample size to be detected | > 2 mg | |
| | Note | Unless otherwise specified, samples are not subjected to grinding before detection | |

**2. DSC tests of compounds 1, 2 and 3**

DSC patterns were collected on TA Instruments Q200 DSC and DSC 3 differential scanning calorimeters. The test parameters of compound 1 and compound 2 were as shown in Table 2-1, and the test parameters of compound 3 were as shown in Table 2-2. The test results were as shown in Figures 2, 5, 8, 11, 14, 17, 20, 23, 26, 29, 32 and 35.

Table 2-1 Test parameters of DSC

| | | |
|---|---|---|
| Instrument | Model | TA Instruments Q200 DSC |
| | Number | LY-01-002 |
| | Control software | Thermal Advantage |
| | Analysis software | Universal Analysis |
| | Sample tray | Aluminium crucible (with a cover, but without perforation) |
| Parameter | Sample size to be detected | 0.5 mg to 5 mg |
| | Protective gas | Nitrogen gas |
| | Gas flow rate | 50 mL/min |
| | Commonly used detection method | Equilibrate at 0°C Ramp 10°C/min to 250°C/290°C |

Table 2-2 Test parameters of DSC

| | | |
|---|---|---|
| Instrument | Model | DSC 3 |
| | Number | LY-01-166 |
| | Control software | STARe |
| | Analysis software | STARe |
| | Sample tray | Aluminium crucible (with a cover and with perforation) |
| Parameter | Sample size to be detected | 1 mg to 10 mg |
| | Protective gas | Nitrogen gas |
| | Gas flow rate | 50 mL/min |
| | Commonly used detection method | Equilibrate at 0°C<br>Ramp 10°C/min to 250°C |

**3. TGA tests of compounds 1, 2 and 3**

TGA patterns were collected on TA Instruments Q500 TGA and TGA/DSC $3^+$ thermogravimetric analysers. The test parameters of compound 1 and compound 2 were as shown in Table 3-1, and the test parameters of compound 3 were as shown in Table 3-2. The test results were as shown in Figures 3, 6, 9, 12, 15, 18, 21, 24, 27, 30, 33 and 36.

Table 3-1 Test parameters of TGA

| | | |
|---|---|---|
| Instrument | Model | TA Instruments Q500 TGA |
| | Number | LY-01-003 |
| | Control software | Thermal Advantage |
| | Analysis software | Universal Analysis |
| | Sample tray | Platinum crucible |

(continued)

| | | |
|---|---|---|
| Parameter | Sample size to be detected | 1 mg to 10 mg |
| | Protective gas | Nitrogen gas |
| | Gas flow rate | 40 mL/min |
| | Commonly used detection method | Hi-Res sensitivity 3.0; Ramp 10.00°C/min, res 5.0 to 150.00°C; Ramp 10.00°C/min to 350°C |

Table 3-2 Test parameters of TGA

| | | |
|---|---|---|
| Instrument | Model | TGA/DSC 3+ |
| | Number | LY-01-167 |
| | Control software | STARe |
| | Analysis software | STARe |
| | Sample tray | Ceramic crucible |
| Parameter | Sample size to be detected | 1 mg to 10 mg |
| | Protective gas | Nitrogen gas |
| | Gas flow rate | 50 mL/min |
| | Commonly used detection method | Hi-Res sensitivity 3.0; Ramp 10.00°C/min, res 5.0 to 150.00°C; Ramp 10.00°C/min to 350°C |

**4. Specific peak value characterization results of XRD tests of compounds 1, 2 and 3**

The X-ray powder diffraction (XRD) pattern of the crystal form I of compound 1 was as shown in Figure 4. Specific peak values were as shown in Table 4.

Table 4

| 2-Theta | d | BG | Height | I% | Area | I% | FWHM |
|---|---|---|---|---|---|---|---|
| 8.323 | 10.6149 | 669 | 2324 | 57.2 | 23926 | 50.1 | 0.175 |
| 10.245 | 8.6272 | 577 | 140 | 3.4 | 647 | 1.4 | 0.079 |
| 10.942 | 8.0791 | 577 | 1554 | 38.3 | 23713 | 49.6 | 0.26 |
| 11.903 | 7.4289 | 553 | 673 | 16.6 | 6101 | 12.8 | 0.154 |
| 13.304 | 6.6497 | 524 | 891 | 21.9 | 9265 | 19.4 | 0.177 |
| 14.386 | 6.1517 | 523 | 511 | 12.6 | 7603 | 15.9 | 0.253 |
| 15.686 | 5.6449 | 732 | 1715 | 42.2 | 18578 | 38.9 | 0.184 |
| 16.407 | 5.3984 | 702 | 1404 | 34.6 | 25605 | 53.6 | 0.31 |
| 16.667 | 5.3147 | 716 | 715 | 17.6 | 15844 | 33.1 | 0.377 |
| 17.243 | 5.1383 | 672 | 224 | 5.5 | 3107 | 6.5 | 0.236 |
| 17.568 | 5.0441 | 675 | 1408 | 34.7 | 17676 | 37 | 0.214 |
| 18.003 | 4.9232 | 679 | 363 | 8.9 | 3830 | 8 | 0.179 |
| 18.888 | 4.6944 | 881 | 4062 | 100 | 42818 | 89.6 | 0.179 |
| 19.747 | 4.492 | 828 | 2841 | 69.9 | 47795 | 100 | 0.286 |

(continued)

| 2-Theta | d | BG | Height | I% | Area | I% | FWHM |
|---|---|---|---|---|---|---|---|
| 20.187 | 4.3951 | 707 | 438 | 10.8 | 11036 | 23.1 | 0.429 |
| 21.249 | 4.1779 | 577 | 650 | 16 | 9218 | 19.3 | 0.241 |
| 21.62 | 4.107 | 586 | 80 | 2 | 671 | 1.4 | 0.143 |
| 22.271 | 3.9884 | 581 | 2283 | 56.2 | 26904 | 56.3 | 0.2 |
| 23.849 | 3.7279 | 538 | 1167 | 28.7 | 25991 | 54.4 | 0.379 |
| 24.83 | 3.5829 | 488 | 190 | 4.7 | 3040 | 6.4 | 0.272 |
| 25.269 | 3.5216 | 470 | 514 | 12.7 | 8436 | 17.7 | 0.279 |
| 26.45 | 3.367 | 435 | 1518 | 37.4 | 26092 | 54.6 | 0.292 |
| 27.123 | 3.2849 | 435 | 83 | 2 | 3142 | 6.6 | 0.644 |
| 27.589 | 3.2305 | 415 | 68 | 1.7 | 856 | 1.8 | 0.214 |
| 28.285 | 3.1525 | 372 | 148 | 3.6 | 2321 | 4.9 | 0.267 |
| 29.45 | 3.0304 | 327 | 71 | 1.7 | 586 | 1.2 | 0.14 |
| 30.111 | 2.9654 | 318 | 323 | 8 | 5231 | 10.9 | 0.275 |
| 31.07 | 2.8761 | 314 | 141 | 3.5 | 2229 | 4.7 | 0.269 |
| 31.649 | 2.8247 | 292 | 74 | 1.8 | 1424 | 3 | 0.327 |
| 32.01 | 2.7937 | 262 | 76 | 1.9 | 1421 | 3 | 0.318 |
| 33.694 | 2.6578 | 254 | 369 | 9.1 | 7891 | 16.5 | 0.364 |
| 35.169 | 2.5496 | 227 | 121 | 3 | 1866 | 3.9 | 0.262 |
| 36.154 | 2.4824 | 214 | 76 | 1.9 | 953 | 2 | 0.213 |
| 36.997 | 2.4277 | 210 | 59 | 1.5 | 1205 | 2.5 | 0.347 |
| 37.625 | 2.3887 | 208 | 61 | 1.5 | 499 | 1 | 0.139 |

The X-ray powder diffraction (XRD) pattern of the crystal form II of compound 1 was as shown in Figure 7. Specific peak values were as shown in Table 5.

Table 5

| 2-Theta | d | BG | Height | I% | Area | I% | FWHM |
|---|---|---|---|---|---|---|---|
| 4.979 | 17.7336 | 1214 | 8900 | 100 | 87065 | 100 | 0.166 |
| 5.479 | 16.117 | 1135 | 3492 | 39.2 | 36750 | 42.2 | 0.179 |
| 7.859 | 11.2407 | 735 | 3518 | 39.5 | 33929 | 39 | 0.164 |
| 10.944 | 8.078 | 583 | 613 | 6.9 | 10168 | 11.7 | 0.282 |
| 11.407 | 7.7507 | 583 | 586 | 6.6 | 6779 | 7.8 | 0.197 |
| 11.945 | 7.4028 | 581 | 109 | 1.2 | 584 | 0.7 | 0.091 |
| 12.685 | 6.9724 | 605 | 145 | 1.6 | 1192 | 1.4 | 0.14 |
| 13.425 | 6.5899 | 630 | 2024 | 22.7 | 44754 | 51.4 | 0.376 |
| 13.724 | 6.4472 | 644 | 3852 | 43.3 | 56786 | 65.2 | 0.251 |
| 14.247 | 6.2115 | 730 | 180 | 2 | 645 | 0.7 | 0.061 |
| 14.927 | 5.9302 | 663 | 2359 | 26.5 | 29085 | 33.4 | 0.21 |
| 15.204 | 5.8227 | 656 | 751 | 8.4 | 11837 | 13.6 | 0.268 |

(continued)

| 2-Theta | d | BG | Height | I% | Area | I% | FWHM |
|---|---|---|---|---|---|---|---|
| 15.902 | 5.5686 | 768 | 850 | 9.6 | 8216 | 9.4 | 0.164 |
| 16.565 | 5.347 | 823 | 2445 | 27.5 | 34850 | 40 | 0.242 |
| 16.946 | 5.2279 | 812 | 1197 | 13.4 | 12636 | 14.5 | 0.18 |
| 17.646 | 5.0221 | 776 | 4534 | 50.9 | 46966 | 53.9 | 0.176 |
| 18.751 | 4.7283 | 577 | 152 | 1.7 | 2342 | 2.7 | 0.262 |
| 19.589 | 4.528 | 634 | 325 | 3.7 | 8890 | 10.2 | 0.465 |
| 20.007 | 4.4344 | 590 | 2610 | 29.3 | 40346 | 46.3 | 0.263 |
| 20.603 | 4.3074 | 680 | 324 | 3.6 | 2205 | 2.5 | 0.116 |
| 21.288 | 4.1704 | 607 | 942 | 10.6 | 33012 | 37.9 | 0.596 |
| 22.047 | 4.0285 | 586 | 1705 | 19.2 | 45071 | 51.8 | 0.45 |
| 23.049 | 3.8555 | 437 | 175 | 2 | 3277 | 3.8 | 0.318 |
| 23.988 | 3.7067 | 400 | 68 | 0.8 | 1079 | 1.2 | 0.27 |
| 24.969 | 3.5633 | 475 | 789 | 8.9 | 16036 | 18.4 | 0.346 |
| 25.768 | 3.4545 | 593 | 981 | 11 | 12591 | 14.5 | 0.218 |
| 27.332 | 3.2603 | 385 | 224 | 2.5 | 8099 | 9.3 | 0.615 |
| 27.651 | 3.2234 | 383 | 406 | 4.6 | 13554 | 15.6 | 0.568 |
| 27.989 | 3.1852 | 395 | 294 | 3.3 | 7071 | 8.1 | 0.409 |
| 28.693 | 3.1087 | 352 | 229 | 2.6 | 2724 | 3.1 | 0.202 |
| 29.629 | 3.0126 | 331 | 87 | 1 | 796 | 0.9 | 0.156 |
| 30.154 | 2.9613 | 335 | 85 | 1 | 1152 | 1.3 | 0.23 |
| 30.557 | 2.9231 | 323 | 66 | 0.7 | 1460 | 1.7 | 0.376 |
| 32.223 | 2.7757 | 251 | 62 | 0.7 | 764 | 0.9 | 0.21 |
| 32.708 | 2.7356 | 262 | 136 | 1.5 | 1641 | 1.9 | 0.205 |
| 33.458 | 2.676 | 250 | 94 | 1.1 | 2578 | 3 | 0.466 |
| 33.91 | 2.6414 | 235 | 107 | 1.2 | 2828 | 3.2 | 0.449 |
| 37.353 | 2.4054 | 200 | 77 | 0.9 | 1822 | 2.1 | 0.402 |

The X-ray powder diffraction (XRD) pattern of the crystal form III of compound 1 was as shown in Figure 10. Specific peak values were as shown in Table 6.

Table 6

| 2-Theta | d | BG | Height | I% | Area | I% | FWHM |
|---|---|---|---|---|---|---|---|
| 5.017 | 17.5993 | 1189 | 3030 | 47.7 | 49913 | 97.5 | 0.28 |
| 5.181 | 17.0438 | 1156 | 2438 | 38.4 | 23296 | 45.5 | 0.153 |
| 8.042 | 10.9853 | 743 | 6351 | 100 | 51190 | 100 | 0.137 |
| 10.042 | 8.8012 | 578 | 180 | 2.8 | 1655 | 3.2 | 0.156 |
| 10.343 | 8.5457 | 565 | 686 | 10.8 | 6708 | 13.1 | 0.166 |
| 10.993 | 8.0414 | 556 | 114 | 1.8 | 776 | 1.5 | 0.116 |
| 12.362 | 7.1542 | 526 | 1039 | 16.4 | 9628 | 18.8 | 0.158 |

(continued)

| 2-Theta | d | BG | Height | I% | Area | I% | FWHM |
|---|---|---|---|---|---|---|---|
| 13.352 | 6.6256 | 500 | 90 | 1.4 | 757 | 1.5 | 0.143 |
| 14.602 | 6.0613 | 575 | 1704 | 26.8 | 10243 | 20 | 0.102 |
| 15.026 | 5.8911 | 605 | 1725 | 27.2 | 17629 | 34.4 | 0.174 |
| 15.563 | 5.6891 | 687 | 1078 | 17 | 8808 | 17.2 | 0.139 |
| 15.725 | 5.631 | 723 | 1908 | 30 | 12761 | 24.9 | 0.114 |
| 16.322 | 5.4262 | 873 | 676 | 10.6 | 2780 | 5.4 | 0.07 |
| 16.588 | 5.3398 | 817 | 512 | 8.1 | 3731 | 7.3 | 0.124 |
| 16.905 | 5.2404 | 692 | 3370 | 53.1 | 29024 | 56.7 | 0.146 |
| 17.228 | 5.1427 | 714 | 5157 | 81.2 | 50079 | 97.8 | 0.165 |
| 17.446 | 5.0791 | 740 | 866 | 13.6 | 19679 | 38.4 | 0.386 |
| 18.189 | 4.8733 | 593 | 3405 | 53.6 | 23288 | 45.5 | 0.116 |
| 18.746 | 4.7296 | 592 | 526 | 8.3 | 5266 | 10.3 | 0.17 |
| 19.405 | 4.5704 | 854 | 4526 | 71.3 | 29764 | 58.1 | 0.112 |
| 19.728 | 4.4964 | 901 | 976 | 15.4 | 7699 | 15 | 0.134 |
| 20.029 | 4.4295 | 603 | 3291 | 51.8 | 32507 | 63.5 | 0.168 |
| 20.345 | 4.3614 | 1045 | 645 | 10.2 | 2976 | 5.8 | 0.074 |
| 20.568 | 4.3146 | 901 | 2278 | 35.9 | 30207 | 59 | 0.226 |
| 21.311 | 4.1659 | 559 | 2693 | 42.4 | 19069 | 37.3 | 0.12 |
| 21.505 | 4.1287 | 538 | 980 | 15.4 | 11540 | 22.5 | 0.188 |
| 21.907 | 4.0538 | 507 | 467 | 7.4 | 4704 | 9.2 | 0.171 |
| 22.41 | 3.9639 | 452 | 435 | 6.8 | 3508 | 6.9 | 0.137 |
| 22.846 | 3.8893 | 432 | 148 | 2.3 | 931 | 1.8 | 0.107 |
| 23.026 | 3.8593 | 428 | 252 | 4 | 2573 | 5 | 0.174 |
| 23.484 | 3.7851 | 416 | 508 | 8 | 5576 | 10.9 | 0.187 |
| 23.953 | 3.7121 | 402 | 659 | 10.4 | 4741 | 9.3 | 0.122 |
| 24.195 | 3.6754 | 392 | 228 | 3.6 | 3468 | 6.8 | 0.259 |
| 24.412 | 3.6432 | 383 | 292 | 4.6 | 2984 | 5.8 | 0.174 |
| 24.847 | 3.5805 | 366 | 364 | 5.7 | 3496 | 6.8 | 0.163 |
| 25.249 | 3.5243 | 352 | 514 | 8.1 | 9114 | 17.8 | 0.302 |
| 25.449 | 3.497 | 345 | 857 | 13.5 | 10768 | 21 | 0.214 |
| 26.03 | 3.4203 | 336 | 84 | 1.3 | 491 | 1 | 0.099 |
| 26.328 | 3.3823 | 327 | 637 | 10 | 6787 | 13.3 | 0.181 |
| 26.934 | 3.3076 | 331 | 361 | 5.7 | 3068 | 6 | 0.145 |
| 27.568 | 3.2329 | 296 | 443 | 7 | 3894 | 7.6 | 0.149 |
| 28.409 | 3.1391 | 264 | 326 | 5.1 | 4278 | 8.4 | 0.223 |
| 29.176 | 3.0583 | 257 | 165 | 2.6 | 1458 | 2.8 | 0.15 |
| 29.587 | 3.0167 | 258 | 242 | 3.8 | 4832 | 9.4 | 0.34 |
| 29.79 | 2.9967 | 242 | 180 | 2.8 | 2773 | 5.4 | 0.262 |

(continued)

| 2-Theta | d | BG | Height | I% | Area | I% | FWHM |
|---|---|---|---|---|---|---|---|
| 30.191 | 2.9577 | 250 | 250 | 3.9 | 2737 | 5.3 | 0.186 |
| 31.171 | 2.8669 | 233 | 162 | 2.6 | 1331 | 2.6 | 0.14 |
| 31.772 | 2.814 | 239 | 249 | 3.9 | 3365 | 6.6 | 0.23 |
| 32.049 | 2.7904 | 238 | 255 | 4 | 3324 | 6.5 | 0.222 |
| 32.472 | 2.755 | 226 | 102 | 1.6 | 1597 | 3.1 | 0.266 |
| 33.135 | 2.7014 | 220 | 210 | 3.3 | 2073 | 4 | 0.168 |
| 34.068 | 2.6295 | 215 | 100 | 1.6 | 1059 | 2.1 | 0.18 |
| 34.416 | 2.6037 | 207 | 161 | 2.5 | 2267 | 4.4 | 0.239 |
| 35.754 | 2.5092 | 219 | 196 | 3.1 | 3196 | 6.2 | 0.277 |
| 36.747 | 2.4437 | 225 | 87 | 1.4 | 868 | 1.7 | 0.17 |
| 37.101 | 2.4212 | 195 | 57 | 0.9 | 511 | 1 | 0.152 |
| 37.938 | 2.3697 | 178 | 113 | 1.8 | 1350 | 2.6 | 0.203 |

The X-ray powder diffraction (XRD) patterns of the crystal form I of compound 2 were as shown in Figures 13-1 and 13-2. Specific peak values were as shown in Table 7.

Table 7

| **2-Theta** | **d** | **Height** | **I%** | **Area** | **I%** |
|---|---|---|---|---|---|
| 4.377 | 20.1693 | 1741 | 100 | 17639 | 100 |
| 8.023 | 11.0103 | 66 | 3.8 | 737 | 4.2 |
| 8.663 | 10.1993 | 1030 | 59.2 | 14242 | 80.7 |
| 9.762 | 9.0531 | 151 | 8.7 | 1098 | 6.2 |
| 10.845 | 8.1509 | 100 | 5.7 | 593 | 3.4 |
| 11.262 | 7.8502 | 133 | 7.6 | 1213 | 6.9 |
| 11.922 | 7.4173 | 252 | 14.5 | 2371 | 13.4 |
| 12.742 | 6.9415 | 577 | 33.1 | 8200 | 46.5 |
| 13.061 | 6.7727 | 843 | 48.4 | 11391 | 64.6 |
| 14.144 | 6.2565 | 188 | 10.8 | 6934 | 39.3 |
| 14.343 | 6.1704 | 453 | 26 | 8216 | 46.6 |
| 14.897 | 5.942 | 74 | 4.3 | 207 | 1.2 |
| 15.31 | 5.7827 | 64 | 3.7 | 1110 | 6.3 |
| 15.605 | 5.6738 | 85 | 4.9 | 1941 | 11 |
| 16.319 | 5.4272 | 75 | 4.3 | 623 | 3.5 |
| 17.041 | 5.1988 | 141 | 8.1 | 1246 | 7.1 |
| 17.442 | 5.0802 | 219 | 12.6 | 3443 | 19.5 |
| 18.18 | 4.8756 | 347 | 19.9 | 6360 | 36.1 |
| 19.524 | 4.543 | 72 | 4.1 | 599 | 3.4 |
| 20.282 | 4.3748 | 490 | 28.1 | 10772 | 61.1 |
| 21.823 | 4.0692 | 559 | 32.1 | 14868 | 84.3 |

(continued)

| 2-Theta | d | Height | I% | Area | I% |
|---|---|---|---|---|---|
| 23.23 | 3.8258 | 87 | 5 | 814 | 4.6 |
| 24.061 | 3.6955 | 106 | 6.1 | 1320 | 7.5 |
| 25.262 | 3.5226 | 136 | 7.8 | 2239 | 12.7 |
| 26.423 | 3.3703 | 86 | 4.9 | 2148 | 12.2 |
| 26.799 | 3.324 | 54 | 3.1 | 1126 | 6.4 |
| 29.503 | 3.0251 | 55 | 3.2 | 813 | 4.6 |
| 39.228 | 2.2947 | 35 | 2 | 337 | 1.9 |

The X-ray powder diffraction (XRD) patterns of the crystal form II of compound 2 were as shown in Figures 16-1 and 16-2. Specific peak values were as shown in Table 8.

Table 8

| 2-Theta | d | Height | I% | Area | I% |
|---|---|---|---|---|---|
| 5.116 | 17.2585 | 514 | 41.8 | 6102 | 41.8 |
| 6.682 | 13.218 | 1230 | 100 | 14607 | 100 |
| 9.986 | 8.8505 | 169 | 13.7 | 2115 | 14.5 |
| 11.036 | 8.0108 | 45 | 3.7 | 636 | 4.4 |
| 13.441 | 6.5823 | 232 | 18.9 | 3725 | 25.5 |
| 13.863 | 6.3829 | 206 | 16.7 | 2553 | 17.5 |
| 15.341 | 5.7711 | 201 | 16.3 | 3304 | 22.6 |
| 15.762 | 5.6176 | 148 | 12 | 2952 | 20.2 |
| 16.503 | 5.3673 | 424 | 34.5 | 5827 | 39.9 |
| 18.976 | 4.6729 | 63 | 5.1 | 650 | 4.4 |
| 20.182 | 4.3963 | 575 | 46.7 | 10939 | 74.9 |
| 20.988 | 4.2293 | 97 | 7.9 | 980 | 6.7 |
| 21.255 | 4.1766 | 62 | 5 | 612 | 4.2 |
| 22.399 | 3.966 | 204 | 16.6 | 2155 | 14.8 |
| 23.121 | 3.8436 | 242 | 19.7 | 5358 | 36.7 |
| 24.14 | 3.6837 | 82 | 6.7 | 1954 | 13.4 |
| 24.54 | 3.6245 | 41 | 3.3 | 538 | 3.7 |
| 26.281 | 3.3883 | 110 | 8.9 | 3092 | 21.2 |
| 26.595 | 3.3489 | 56 | 4.6 | 2211 | 15.1 |
| 27.321 | 3.2616 | 47 | 3.8 | 814 | 5.6 |
| 27.574 | 3.2322 | 40 | 3.3 | 815 | 5.6 |
| 28.283 | 3.1528 | 56 | 4.6 | 864 | 5.9 |
| 28.538 | 3.1252 | 42 | 3.4 | 879 | 6 |
| 30.409 | 2.937 | 35 | 2.8 | 381 | 2.6 |
| 31.342 | 2.8517 | 40 | 3.3 | 512 | 3.5 |

The X-ray powder diffraction (XRD) patterns of the crystal form III of compound 2 were as shown in Figures 19-1 and 19-2. Specific peak values were as shown in Table 9.

Table 9

| 2-Theta | d | Height | I% | Area | I% |
|---|---|---|---|---|---|
| 3.996 | 22.0947 | 148 | 5.8 | 847 | 4.8 |
| 7.475 | 11.816 | 2550 | 100 | 17651 | 100 |
| 9.313 | 9.4881 | 75 | 2.9 | 564 | 3.2 |
| 11.431 | 7.7343 | 54 | 2.1 | 362 | 2.1 |
| 11.776 | 7.5089 | 191 | 7.5 | 1545 | 8.8 |
| 12.242 | 7.2237 | 770 | 30.2 | 5762 | 32.6 |
| 13.322 | 6.6406 | 156 | 6.1 | 1162 | 6.6 |
| 14.511 | 6.099 | 49 | 1.9 | 348 | 2 |
| 14.948 | 5.9219 | 213 | 8.4 | 1091 | 6.2 |
| 15.585 | 5.6813 | 298 | 11.7 | 3871 | 21.9 |
| 16.176 | 5.4749 | 272 | 10.7 | 3225 | 18.3 |
| 16.437 | 5.3885 | 159 | 6.2 | 738 | 4.2 |
| 16.701 | 5.3038 | 157 | 6.2 | 736 | 4.2 |
| 17.687 | 5.0105 | 106 | 4.2 | 1521 | 8.6 |
| 18.742 | 4.7307 | 373 | 14.6 | 5292 | 30 |
| 19.001 | 4.6668 | 264 | 10.4 | 2993 | 17 |
| 20.246 | 4.3825 | 107 | 4.2 | 919 | 5.2 |
| 21.385 | 4.1517 | 151 | 5.9 | 3600 | 20.4 |
| 21.624 | 4.1062 | 104 | 4.1 | 3292 | 18.7 |
| 21.845 | 4.0652 | 122 | 4.8 | 2466 | 14 |
| 22.503 | 3.9478 | 997 | 39.1 | 9765 | 55.3 |
| 22.842 | 3.89 | 123 | 4.8 | 2515 | 14.2 |
| 23.102 | 3.8468 | 91 | 3.6 | 855 | 4.8 |
| 23.845 | 3.7286 | 343 | 13.5 | 3284 | 18.6 |
| 24.331 | 3.6552 | 93 | 3.6 | 2279 | 12.9 |
| 25.038 | 3.5536 | 47 | 1.8 | 201 | 1.1 |
| 25.765 | 3.4549 | 452 | 17.7 | 4944 | 28 |
| 27.825 | 3.2036 | 128 | 5 | 2406 | 13.6 |
| 29.364 | 3.0391 | 87 | 3.4 | 1335 | 7.6 |

The X-ray powder diffraction (XRD) patterns of the crystal form IV of compound 2 were as shown in Figures 22-1 and 22-2. Specific peak values were as shown in Table 10.

Table 10

| 2-Theta | d | Height | I% | Area | I% |
|---|---|---|---|---|---|
| 3.918 | 22.5314 | 331 | 60.2 | 3600 | 38.7 |
| 7.762 | 11.3801 | 146 | 26.5 | 1419 | 15.3 |

(continued)

| 2-Theta | d | Height | I% | Area | I% |
|---|---|---|---|---|---|
| 8.7 | 10.1553 | 550 | 100 | 9302 | 100 |
| 10.136 | 8.7195 | 66 | 12 | 1716 | 18.4 |
| 10.481 | 8.4337 | 145 | 26.4 | 3370 | 36.2 |
| 12.048 | 7.3398 | 54 | 9.8 | 1454 | 15.6 |
| 12.46 | 7.0979 | 190 | 34.5 | 2852 | 30.7 |
| 13.914 | 6.3592 | 56 | 10.2 | 1100 | 11.8 |
| 15.542 | 5.6967 | 224 | 40.7 | 3615 | 38.9 |
| 16.785 | 5.2777 | 139 | 25.3 | 1469 | 15.8 |
| 17.508 | 5.0613 | 87 | 15.8 | 1830 | 19.7 |
| 18.221 | 4.8649 | 205 | 37.3 | 4139 | 44.5 |
| 18.943 | 4.681 | 143 | 26 | 1862 | 20 |
| 19.665 | 4.5107 | 183 | 33.3 | 3792 | 40.8 |
| 23.639 | 3.7605 | 72 | 13.1 | 1363 | 14.7 |

The X-ray powder diffraction (XRD) pattern of the crystal form I of compound 3 was as shown in Figure 28. Specific peak values were as shown in Table 11.

Table 11

| 2-Theta | d | Height | I% | Area | I% |
|---|---|---|---|---|---|
| 3.982 | 22.171 | 1341 | 24.9 | 12956 | 26.1 |
| 4.717 | 18.7191 | 212 | 3.9 | 2332 | 4.7 |
| 5.961 | 14.8149 | 4110 | 76.2 | 48450 | 97.5 |
| 6.183 | 14.2819 | 1080 | 20 | 12242 | 24.6 |
| 7.645 | 11.5541 | 633 | 11.7 | 5532 | 11.1 |
| 9.302 | 9.4998 | 1257 | 23.3 | 16176 | 32.5 |
| 9.578 | 9.2265 | 467 | 8.7 | 5600 | 11.3 |
| 9.921 | 8.908 | 451 | 8.4 | 4411 | 8.9 |
| 10.866 | 8.1354 | 693 | 12.8 | 7712 | 15.5 |
| 11.161 | 7.9208 | 192 | 3.6 | 3691 | 7.4 |
| 11.861 | 7.4553 | 5395 | 100 | 49258 | 99.1 |
| 12.605 | 7.0167 | 432 | 8 | 3167 | 6.4 |
| 12.845 | 6.8859 | 442 | 8.2 | 3326 | 6.7 |
| 13.366 | 6.6189 | 322 | 6 | 2465 | 5 |
| 13.745 | 6.4371 | 508 | 9.4 | 5537 | 11.1 |
| 14.447 | 6.1259 | 368 | 6.8 | 3491 | 7 |
| 15.004 | 5.8997 | 179 | 3.3 | 1285 | 2.6 |
| 15.287 | 5.7912 | 467 | 8.7 | 5870 | 11.8 |
| 15.804 | 5.603 | 1491 | 27.6 | 16385 | 33 |
| 16.202 | 5.466 | 113 | 2.1 | 714 | 1.4 |

(continued)

| 2-Theta | d | Height | I% | Area | I% |
|---|---|---|---|---|---|
| 16.604 | 5.3347 | 641 | 11.9 | 5691 | 11.4 |
| 16.884 | 5.2468 | 1193 | 22.1 | 11488 | 23.1 |
| 17.325 | 5.1144 | 1072 | 19.9 | 8684 | 17.5 |
| 17.885 | 4.9554 | 855 | 15.8 | 8847 | 17.8 |
| 18.246 | 4.8581 | 841 | 15.6 | 12818 | 25.8 |
| 18.546 | 4.7802 | 958 | 17.8 | 13140 | 26.4 |
| 19.207 | 4.6171 | 402 | 7.5 | 5519 | 11.1 |
| 19.91 | 4.4558 | 862 | 16 | 30956 | 62.3 |
| 20.167 | 4.3995 | 899 | 16.7 | 12343 | 24.8 |
| 20.444 | 4.3406 | 1060 | 19.6 | 13328 | 26.8 |
| 21.326 | 4.163 | 238 | 4.4 | 1531 | 3.1 |
| 21.749 | 4.0829 | 2558 | 47.4 | 27358 | 55 |
| 22.166 | 4.0071 | 978 | 18.1 | 24665 | 49.6 |
| 22.409 | 3.9642 | 1003 | 18.6 | 17207 | 34.6 |
| 22.749 | 3.9056 | 204 | 3.8 | 1848 | 3.7 |
| 23.927 | 3.716 | 3166 | 58.7 | 49712 | 100 |
| 24.616 | 3.6136 | 188 | 3.5 | 1593 | 3.2 |
| 25.306 | 3.5165 | 248 | 4.6 | 2013 | 4 |
| 25.77 | 3.4542 | 542 | 10 | 3586 | 7.2 |
| 26.21 | 3.3973 | 1085 | 20.1 | 18055 | 36.3 |
| 26.707 | 3.3351 | 244 | 4.5 | 1638 | 3.3 |
| 27.369 | 3.2559 | 361 | 6.7 | 5116 | 10.3 |
| 27.689 | 3.2191 | 723 | 13.4 | 12127 | 24.4 |
| 27.952 | 3.1894 | 477 | 8.8 | 12761 | 25.7 |
| 28.431 | 3.1367 | 353 | 6.5 | 4167 | 8.4 |
| 28.988 | 3.0777 | 175 | 3.2 | 3531 | 7.1 |
| 29.195 | 3.0563 | 219 | 4.1 | 3535 | 7.1 |
| 29.77 | 2.9986 | 428 | 7.9 | 10655 | 21.4 |
| 30.269 | 2.9503 | 531 | 9.8 | 5579 | 11.2 |
| 30.826 | 2.8983 | 75 | 1.4 | 212 | 0.4 |
| 31.13 | 2.8706 | 104 | 1.9 | 2031 | 4.1 |
| 31.512 | 2.8367 | 581 | 10.8 | 12473 | 25.1 |
| 32.586 | 2.7456 | 91 | 1.7 | 800 | 1.6 |
| 34.209 | 2.619 | 218 | 4 | 2492 | 5 |
| 34.912 | 2.5678 | 86 | 1.6 | 1384 | 2.8 |

The X-ray powder diffraction (XRD) pattern of the crystal form II of compound 3 was as shown in Figure 34. Specific peak values were as shown in Table 12.

Table 12

| 2-Theta | d | Height | I% | Area | I% |
|---|---|---|---|---|---|
| 3.982 | 22.1703 | 4212 | 100 | 73527 | 100 |
| 6.345 | 13.9191 | 1726 | 41 | 19664 | 26.7 |
| 8.103 | 10.9026 | 1208 | 28.7 | 12546 | 17.1 |
| 9.661 | 9.1473 | 1086 | 25.8 | 13129 | 17.9 |
| 10.473 | 8.4399 | 95 | 2.3 | 436 | 0.6 |
| 12.205 | 7.246 | 4062 | 96.4 | 48626 | 66.1 |
| 12.784 | 6.9191 | 901 | 21.4 | 12703 | 17.3 |
| 14.882 | 5.9481 | 245 | 5.8 | 1714 | 2.3 |
| 15.346 | 5.7689 | 208 | 4.9 | 964 | 1.3 |
| 15.785 | 5.6096 | 1143 | 27.1 | 11201 | 15.2 |
| 16.325 | 5.4252 | 959 | 22.8 | 5761 | 7.8 |
| 16.747 | 5.2895 | 1406 | 33.4 | 23883 | 32.5 |
| 17.127 | 5.1728 | 1068 | 25.4 | 30246 | 41.1 |
| 17.407 | 5.0904 | 704 | 16.7 | 22768 | 31 |
| 18.446 | 4.806 | 226 | 5.4 | 1473 | 2 |
| 18.948 | 4.6797 | 183 | 4.3 | 3450 | 4.7 |
| 19.388 | 4.5746 | 1225 | 29.1 | 22320 | 30.4 |
| 20.449 | 4.3394 | 665 | 15.8 | 8048 | 10.9 |
| 21.427 | 4.1435 | 383 | 9.1 | 5700 | 7.8 |
| 22.308 | 3.9819 | 91 | 2.2 | 1544 | 2.1 |
| 23.227 | 3.8264 | 1225 | 29.1 | 26339 | 35.8 |
| 23.547 | 3.7751 | 532 | 12.6 | 17218 | 23.4 |
| 24.649 | 3.6088 | 768 | 18.2 | 10917 | 14.8 |
| 25.749 | 3.457 | 698 | 16.6 | 17885 | 24.3 |
| 26.77 | 3.3274 | 160 | 3.8 | 2917 | 4 |
| 27.091 | 3.2887 | 322 | 7.6 | 7139 | 9.7 |
| 27.608 | 3.2283 | 194 | 4.6 | 1590 | 2.2 |
| 28.31 | 3.1498 | 582 | 13.8 | 12038 | 16.4 |
| 28.871 | 3.0899 | 210 | 5 | 6754 | 9.2 |
| 29.433 | 3.0322 | 143 | 3.4 | 2317 | 3.2 |
| 31.015 | 2.881 | 139 | 3.3 | 1867 | 2.5 |
| 31.69 | 2.8211 | 95 | 2.3 | 1678 | 2.3 |
| 33.072 | 2.7064 | 145 | 3.4 | 3709 | 5 |
| 33.45 | 2.6767 | 90 | 2.1 | 1869 | 2.5 |

**5. Chemical stability data of compound 1 and pharmaceutical salt thereof**

Samples were taken and tested at high temperature (40°C) and under high humidity (RH 92.5%) respectively. The purity (represented by a percentage) was detected by HPLC. The experimental results were as shown in Table 16.

With regard to methods for preparing test solutions and conditions for detecting purity by HPLC, reference was made to Tables 13, 14 and 15.

Table 13 Preparation method 1 for test solution

| | |
|---|---|
| Diluent | Acetonitrile : methanol = 1 : 1 |
| Blank solution | Acetonitrile : methanol = 1 : 1 |
| Test solution | An appropriate amount of test samples was weighed precisely and dissolved and diluted with a diluent to prepare a test solution, which contains about 0.5 mg of the test samples per 1 mL. |

Table 14 Preparation method 2 for test solution

| | |
|---|---|
| Diluent | Aqueous solution containing 0.05% TFA: MeOH = 8 : 2 |
| Blank solution | Aqueous solution containing 0.05% TFA: MeOH = 8 : 2 |
| Test solution | An appropriate amount of test samples was weighed precisely and dissolved and diluted with a diluent to prepare a test solution, which contains about 0.5 mg of the test samples per 1 mL. |

Table 15 Conditions for detecting purity by HPLC

| | |
|---|---|
| Instrument | LC-20AT (Shimadzu) |
| Chromatographic column | Agilent Eclipse plus C18, 4.6 mm × 150 mm, 3.5 μm |
| Mobile phase | A: 0.02 mol/L sodium dihydrogen phosphate solution (the pH was adjusted to 2.5 with phosphoric acid)<br>B: Acetonitrile |
| Detection wavelength | 220 nm |
| Flow rate | 1.0 mL/min |
| Column temperature | 30°C |
| Injection volume | 10 μL |
| Operation time | 45 min |
| Elution procedure | see below |

Elution procedure:

| Time (min) | Mobile phase A (%) | Mobile phase B (%) |
|---|---|---|
| 0 | 80 | 20 |
| 15 | 70 | 30 |
| 35 | 65 | 35 |
| 40 | 10 | 90 |
| 40.10 | 80 | 20 |
| 45 | 80 | 20 |

Table 16 Chemical stability of various salts and crystal forms of compound 1 under different conditions (the content was determined by HPLC)

| Name \ Condition | Preparation method for test solution | 0 day | 40°C 30 days | 92.5% RH 30 days |
|---|---|---|---|---|
| Amorphous form of compound 1 | Preparation method 1 | 98.88% | 98.85% | 98.82% |
| Crystal form III of compound 1 | Preparation method 1 | 99.41% | 99.28% | 99.30% |
| Crystal form I of compound 3 | Preparation method 1 | 99.85% | 99.74% | 99.76% |
| Compound 5 | Preparation method 1 | 98.44% | N/A | 98.05% |
| Compound 9 | Preparation method 2 | 98.67% | N/A | 98.28% |

Conclusion: the amorphous form and crystal form III of compound 1 and the pharmaceutical salts (e.g., crystal form I of compound 3, compound 5 and compound 9) of compound 1 had relatively good chemical stability.

**6. Stability data of crystal form of compounds 1 and 3**

**6.1.** Stability of crystal form of compound 1 and compound 3. See table 17.

Table 17 Stability of crystal form of compound 1 and compound 3

| Crystal form before transformation | Transformation condition | Crystal form after transformation |
|---|---|---|
| Amorphous form of compound 1 | High humidity (97% RH), room temperature, for 30 days | Amorphous form of compound 1 |
| Crystal form I of compound 1 | Hermetically sealed, room temperature, for 30 days | Crystal form I of compound 1 |
| Crystal form II of compound 1 | Hermetically sealed, room temperature, for 30 days | Crystal form II of compound 1 |
| Crystal form III of compound 1 | Hermetically sealed, room temperature, for 30 days | Crystal form III of compound 1 |
| Crystal form I of compound 3 | Hermetically sealed, room temperature, for 30 days | Crystal form I of compound 3 |
| Amorphous form of compound 3 | Exposed to an atmosphere, high humidity (25°C ± 2°C, 85% RH ± 10% RH), for 10 days | Amorphous form of compound 3 |
| Crystal form II of compound 3 | Hermetically sealed, for 20 days | Crystal form II of compound 3 |

Conclusion: the amorphous form and crystal forms I, II and III of compound 1 and the amorphous form and crystal forms

I and II of compound 3 had relatively good stability.

**6.2.** Competitive experiments of crystal forms I, II and III of compound 1 were performed at room temperature to investigate the stability of the crystal forms in isopropyl acetate and a water/acetonitrile (v/v = 1 : 1) solvent, and Competitive experiments of the crystal forms I and II of compound 3 were performed at room temperature to investigate the stability of the crystal forms in an ethanol/water (v/v = 1 : 1) solvent and an acetone/water (v/v = 1 : 1) solvent. See Table 18 for details.

Table 18 Crystal slurrying competition experiment of crystal forms of compound 1 and compound 3

| Crystal form | Experimental condition | Experimental XRD result |
|---|---|---|
| Mixed sample of crystal forms I, II and III of | Competing for crystal slurrying in isopropyl acetate for 2 days | Crystal form III of compound 1 |
| compound 1 in equal weights | Competing for crystal slurrying in water/ acetonitrile (v/v = 1 : 1) for 2 days | Crystal form III of compound 1 |
| Mixed sample of crystal forms I and II of compound 3 in equal weights | Competing for crystal slurrying in ethanol/ water (v/v = 1 : 1) for 3 days | Crystal form I of compound 3 |
| | Competing for crystal slurrying in acetone/ water (v/v = 1 : 1) for 3 days | Crystal form I of compound 3 |

It can be seen from the above crystal slurrying competition experiments of the crystal forms of compound 1 that the crystal form III was the most stable crystal form of compound 1 at room temperature. It can be seen from the above crystal slurrying competition experiments of the crystal forms of compound 3 that the crystal form I was the most stable crystal form of compound 3 at room temperature.

**7. Solubility data in water at 25°C**

Table 19 Solubility of various pharmaceutical salts of compound 1 in water at 25°C

| Name | Amorphous form of compound 1 | Crystal form I of compound 3 | Compound 4 | Compound 5 | Compound 7 | Compound 9 |
|---|---|---|---|---|---|---|
| Solubility in water at 25°C | 0.009 mg/mL | 0.172 mg/mL | 0.158 mg/mL | 0.166 mg/mL | 0.143 mg/mL | 0.346 mg/mL |

Conclusion: compound 1 and the pharmaceutical salts thereof had a certain level of solubility in water at 25°C. The solubility of the pharmaceutical salts of compound 1 (such as the crystal form I of compound 3, compound 4, compound 5, compound 7 and compound 9) was significantly improved as compared with the solubility of compound 1, by more than about 15 times.

**8. Detection of BTK degradation in Mino cells**

The Mino human mantle cell lymphoma cell line was purchased from ATCC and cultured under conditions of RPMI-1640 + 15% FBS + 1% double antibody in a 37°C, 5% $CO_2$ incubator. Cells were plated in a 6-well plate, with $5 \times 10^5$ cells/well. After plating, compounds at different concentrations were added and cultured in a 37°C, 5% $CO_2$ incubator for 48 h. After culturing, the cells were collected. The cells were lysed on ice for 15 minutes by adding RIPA lysis buffer (Beyotime, Cat. P0013B) and centrifuged at 12000 rpm at 4°C for 10 minutes. The protein sample of the supernatant was collected, subjected to protein quantification by using a BCA kit (Beyotime, Cat. P0009) and then diluted to 0.25 mg/mL. The expressions of BTK (CST, Cat. 8547S) and the internal reference β-actin (CST, Cat. 3700S) were detected using a fully automated western blot quantitative analyser (Proteinsimple) with a kit (Protein simple, Cat. SM-W004). The expression level of BTK relative to the internal reference was calculated by using Compass software, and the $DC_{50}$ value was calculated by using Origen9.2 software according to formula (1). Specifically, the $BTK_{administration}$ denoted the expression level of BTK in administration groups at different doses, and the $BTK_{vehicle}$ denoted the expression level of BTK in the vehicle control group.

$$BTK\% = BTK_{administration}/BTK_{vehicle} \times 100 \qquad \text{formula (1)}$$

Table 20 $DC_{50}$ values for BTK degradation in Mino cells

| Serial No. | Compound No. | $DC_{50}$ (nM) |
|---|---|---|
| 1 | Compound 2 | 22.9 |
| 2 | Compound 1 | 10.9 |

Conclusion: compound 1 and compound 2 had a significant degradation effect on BTK in Mino cells.

**9. Detection of BTK protein degradation in spleen of mice**

Female ICR mice, 6-8 weeks old, were purchased from Beijing Vital River Laboratory Animal Technology Co., Ltd., and the experiment was started after 3 days of adaptation. After 3 consecutive days of intragastric administration of compounds at different doses, the spleens of mice were taken. The spleen cells were collected, lysed on ice for 15 min by adding RIPA lysis buffer (Beyotime, Cat. P0013B), and then centrifuged at 12000 rpm at 4°C for 10 min. The protein sample of the supernatant was collected, subjected to protein quantification by using a BCA kit (Beyotime, Cat. P0009) and then diluted to 0.25 mg/mL. The expressions of BTK (CST, Cat. 8547S) and the internal reference β-actin (CST, Cat. 3700S) were detected by using a fully automated western blot quantitative analyser (Proteinsimple). The expression level of BTK relative to the internal reference was calculated by using Compass software, and the $DD_{50}$ value was calculated by using Origen9.2 software according to formula (2). Specifically, the $BTK_{administration}$ denoted the expression level of BTK in administration groups at different doses, and the $BTK_{vehicle}$ denoted the expression level of BTK in the vehicle control group.

$$BTK\% = BTK_{administration}/BTK_{vehicle} \times 100\% \qquad \text{formula (2)}$$

Table 21 $DD_{50}$ values of compounds for BTK protein degradation in spleens of mice

| Serial No. | Compound No. | $DD_{50}$ (mg/kg) |
|---|---|---|
| 1 | Compound 2 | 3.8 |
| 2 | Compound 1 | 3.8 |

Conclusion: compound 1 and compound 2 had a significant degradation effect on BTK proteins in spleens of mice.

**10. *In vitro* kinase detection**

Kinases BTK wt (Carna, Cat. No 08-180) and BTK C481S (Carna, Cat. No 08-547) were prepared into a 2.5× kinase solution, and substrates FAM-P2 (GL Biochem, Cat. No. 112394) and ATP ((Sigma, Cat. No. A7699-1G) were prepared into a 2.5× substrate solution, respectively. 5 μL of compounds at different concentrations were added to a 384-well plate.10 μL of 2.5× kinase solution was added, and the resulting mixture was incubated at room temperature for 10 min. 10 μL of 2.5× substrate solution was added, and the mixture was incubated at 28°C for an appropriate period of time. The reaction was stopped by adding 30 μL of stop buffer, and the detection was carried out by using Caliper EZ reader2. The $IC_{50}$ value was calculated by using XLFit excel add-in version 5.4.0.8 software. The calculation formula of the inhibition rate was shown in formula (3), wherein max denoted the readout of the DMSO control, min denoted the readout of the negative control, and conversion denoted the readout of the compound

$$\text{Inhibition rate \%} = (\text{max-conversion})/(\text{max-min}) * 100\%. \qquad \text{formula (3)}$$

The results were as shown in Table 22:

Table 22 $IC_{50}$ value on BTK wt/C481S kinase inhibition

| Serial No. | Compound No. | BTK C481S $IC_{50}$ (nM) | BTK wt $IC_{50}$ (nM) |
|---|---|---|---|
| 1 | Compound 1 | 8 | 6.3 |

Conclusion: compound 1 had a significant inhibitory effect on BTK wt/C481S kinase.

**11. Pharmacokinetic test of dogs**

**Experimental objective:** in this experiment, a single dose of each test compound was administered to Beagle dogs intravenously and intragastrically, the concentrations of the test compounds in plasma of dogs were measured, and the pharmacokinetic characteristics and bioavailability of the test compounds in dogs were evaluated.

**Experimental animal:** male Beagle dogs (about 8-11 kg, 0.5-1 weeks old, 6 dogs/compound), purchased from Beijing Marshall Biotechnology Co. Ltd.

**Experimental method:** as shown in Table 23, on the day of the experiment, 6 Beagle dogs were randomly according to their body weight. The animals were fasted but with water available for 14 to 18 hours one day before administration, and were fed 4 hours after administration.

Table 23

| Group | Quantity | Administration information | | | | | | |
|---|---|---|---|---|---|---|---|---|
| | Male | Test compound | Administrat ion dosage* (mg/kg) | Administra tion concentrati on (mg/mL) | Administr ation volume (mL/kg) | Collected samples | Mode of administr ation | Vehicle |
| G1 | 3 | Compound of the present invention | 1 | 1 | 1 | Plasma | Intraveno usly | 5% DMSO + 5% Solutol + 90% Saline |
| G2 | 3 | Compound of the present invention | 10 | 2 | 5 | Plasma | Intragastri cally | 0.5% MC |
| *Dosage is calculated based on free base. | | | | | | | | |

**Sampling:** before and after administration, 1.0 ml of blood was taken from jugular veins, and placed in an EDTAK2 centrifuge tube. Centrifugation was carried out at 5000 rpm at 4°C for 10 min, and the plasma was collected.
Time points for plasma collection in G1&G2 groups: 0, 5 min, 15 min, 30 min, 1 h, 2 h, 4 h, 6 h, 8 h, 10 h, 12 h and 24 h.
Before analysis and detection, all samples were stored at -80°C. The samples were detected by using HPLC-MS/MS.

Table 24 Pharmacokinetic parameters of compounds in plasma of dogs

| Test compounds | Mode of administration* | $AUC_{0-t}$ (pg/ml·h) | Having bioavailability or not |
|---|---|---|---|
| Amorphous form of compound 1 | i.g. (10 mg/kg) | 26900 ± 3300 | Yes |
| Crystal form I of compound 3 | i.g. (10 mg/kg) | 75100 ± 50000 | Yes |
| Compound 5 | i.g. (10 mg/kg) | 59500 ± 21000 | Yes |
| Compound 7 | i.g. (10 mg/kg) | 95500 ± 65000 | Yes |
| Compound 9 | i.g. (10 mg/kg) | 79400 ± 20000 | Yes |
| *Note: i.g. (intragastrical) administration, | | | |

Conclusion: compound 1 and pharmaceutical salts thereof had a certain level of oral bioavailability in dogs. Oral exposure amounts of the crystal form I of compound 3, compound 5, compound 7 and compound 9 were significantly increased as compared with the oral exposure amount of compound 1, by more than 2 times.

## Claims

**1.** A pharmaceutical salt of a compound as shown in formula (I),

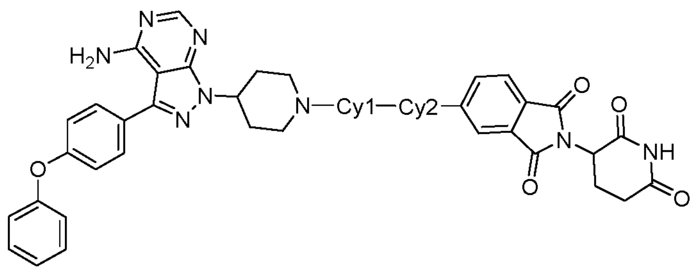

(I)

wherein

Cy1 or Cy2 is each independently selected from piperidyl or azacyclobutyl; and
the pharmaceutical salt is selected from maleate, fumarate, halogen acid salt (preferably hydrobromide and hydrochloride), sulfate, phosphate, L-tartrate, citrate, L-malate, hippurate, D-glucuronate, glycollate, mucate, succinate, lactate, orotate, pamoate, glycinate, alanine salt, arginine salt, cinnamate, benzoate, benzenesulfonate, p-toluenesulfonate, acetate, propionate, valerianate, triphenyl acetate, L-proline salt, ferulate, 2-hydroxyethanesulfonate, mandelate, nitrate, mesylate, malonate, gentisate, salicylate, oxalate or glutarate.

**2.** The pharmaceutical salt according to claim 1, wherein the compound as shown in formula (I) is selected from a compound as shown in formula (Ia) or (Ib),

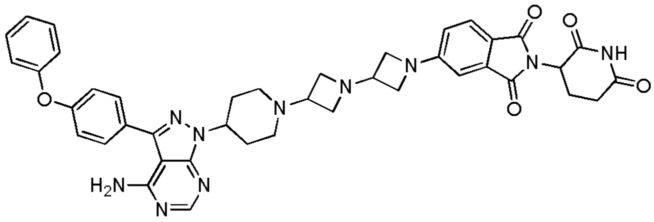

(Ia)

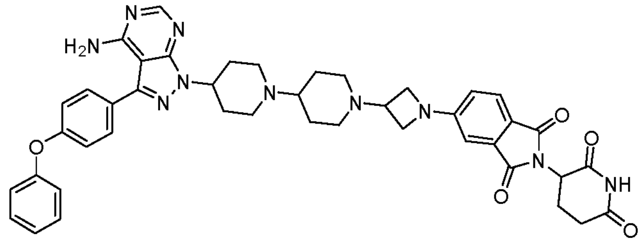

(Ib)

and the pharmaceutical salt is selected from maleate, fumarate, halogen acid salt (preferably hydrobromide and hydrochloride), sulfate, phosphate, L-tartrate, citrate, L-malate, hippurate, D-glucuronate, glycollate, mucate, succinate, lactate, orotate, pamoate, glycinate, alanine salt, arginine salt, cinnamate, benzoate, benzenesulfonate, p-toluenesulfonate, acetate, propionate, valerianate, triphenyl acetate, L-proline salt, ferulate, 2-hydroxyethanesulfonate, mandelate, nitrate, mesylate, malonate, gentisate, salicylate, oxalate or glutarate.

3. The pharmaceutical salt according to claim 2, wherein the pharmaceutical salt is selected from maleate, fumarate, L-tartrate, citrate, L-malate, salicylate or oxalate.

4. The pharmaceutical salt according to claim 1, wherein the pharmaceutical salt of the compound as shown in formula (I) is selected from a compound as shown in formula (II),

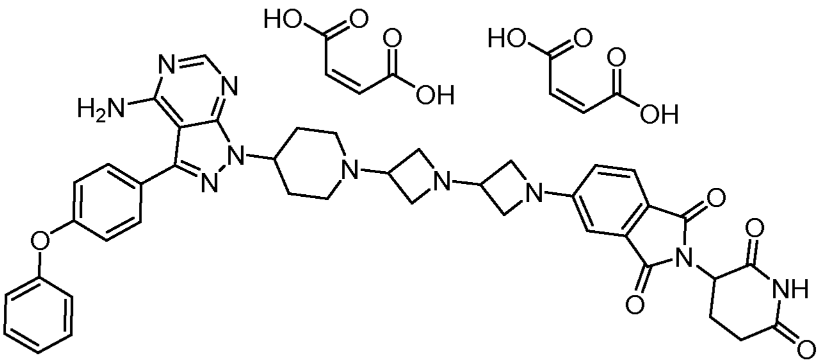

(II).

5. A crystal form I of the compound as shown in formula (II), wherein the crystal form I has an X-ray powder diffraction pattern comprising characteristic diffraction peaks at 5.96° ± 0.2°, 9.30° ± 0.2°, 11.86° ± 0.2°, 15.80° ± 0.2°, 21.75° ± 0.2° and 23.93° ± 0.2° 2θ, as determined by using Cu-Kα radiation.

6. The crystal form I of the compound as shown in formula (II) according to claim 5, wherein the crystal form I has an X-ray powder diffraction pattern further comprising characteristic diffraction peaks at 3.98° ± 0.2°, 7.65° ± 0.2°, 10.87° ± 0.2°, 16.88° ± 0.2°, 17.89° ± 0.2° and 26.21° ± 0.2° 2θ, as determined by using Cu-Kα radiation.

7. The crystal form I of the compound as shown in formula (II) according to claim 6, wherein the crystal form I has an X-ray powder diffraction pattern further comprising characteristic diffraction peaks at 15.29° ± 0.2°, 17.33° ± 0.2°, 18.55° ± 0.2°, 19.21° ± 0.2°, 19.91° ± 0.2° and 22.41° ± 0.2° 2θ, as determined by using Cu-Kα radiation.

8. The crystal form I of the compound as shown in formula (II) according to claim 7, wherein the crystal form I has an X-ray powder diffraction pattern further comprising characteristic diffraction peaks at 4.72° ± 0.2°, 9.58° ± 0.2°,

9.92° ± 0.2°, 12.85° ± 0.2°, 13.37° ± 0.2°, 13.75° ± 0.2°, 14.45° ± 0.2°, 27.37° ± 0.2°, 28.43° ± 0.2°, 30.27° ± 0.2°, 31.51° ± 0.2° and 34.21° ± 0.2° 2θ, as determined by using Cu-Kα radiation.

**9.** The crystal form I of the compound as shown in formula (II) according to claim 8, wherein the crystal form I, as determined by using Cu-Kα radiation, has an X-ray powder diffraction pattern as shown in Figure 28.

**10.** The crystal form I of the compound as shown in formula (II) according to claim 8, **characterized in that** the crystal form I has a differential scanning calorimetry curve as shown in Figure 29 or a thermogravimetric analysis curve as shown in Figure 30.

**11.** A crystal form III of the compound as shown in formula (Ia), wherein the crystal form III has an X-ray powder diffraction pattern comprising characteristic diffraction peaks at 5.02° ± 0.2°, 8.04° ± 0.2°, 16.91° ± 0.2°, 17.23° ± 0.2°, 18.19° ± 0.2°, 19.41° ± 0.2° and 20.03° ± 0.2° 2θ, as determined by using Cu-Kα radiation,

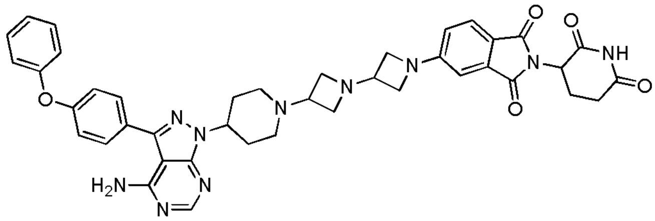

(Ia).

**12.** The crystal form III of the compound as shown in formula (Ia) according to claim 11, wherein the crystal form III has an X-ray powder diffraction pattern further comprising characteristic diffraction peaks at 12.36° ± 0.2°, 14.60° ± 0.2°, 15.03° ± 0.2°, 15.73° ± 0.2°, 20.57° ± 0.2°, 21.31° ± 0.2° and 25.45° ± 0.2° 2θ, as determined by using Cu-Kα radiation.

**13.** The crystal form III of the compound as shown in formula (Ia) according to claim 12, wherein the crystal form III has an X-ray powder diffraction pattern further comprising characteristic diffraction peaks at 5.19° ± 0.2°, 16.32° ± 0.2°, 18.75° ± 0.2°, 19.73° ± 0.2°, 21.91° ± 0.2°, 22.41° ± 0.2°, 23.48° ± 0.2°, 23.95° ± 0.2° and 26.33° ± 0.2° 2θ, as determined by using Cu-Kα radiation.

**14.** The crystal form III of the compound as shown in formula (Ia) according to claim 13, wherein the crystal form III has an X-ray powder diffraction pattern further comprising characteristic diffraction peaks at 10.34° ± 0.2°, 24.85° ± 0.2°, 26.93° ± 0.2°, 27.57° ± 0.2°, 28.41° ± 0.2°, 29.59° ± 0.2°, 30.19° ± 0.2°, 31.77° ± 0.2°, 33.13° ± 0.2° and 35.75° ± 0.2° 2θ, as determined by using Cu-Kα radiation.

**15.** The crystal form III of the compound as shown in formula (Ia) according to claim 14, wherein the crystal form III, as determined by using Cu-Kα radiation, has an X-ray powder diffraction pattern as shown in Figure 10.

**16.** The crystal form III of the compound as shown in formula (Ia) according to claim 14, **characterized in that** the crystal form III has a differential scanning calorimetry curve as shown in Figure 11 or a thermogravimetric analysis curve as shown in Figure 12.

**17.** A crystal form I of the compound as shown in formula (Ib), wherein the crystal form I has an X-ray powder diffraction pattern comprising characteristic diffraction peaks at 4.38° ± 0.2°, 8.66° ± 0.2°, 13.06° ± 0.2°, 14.34° ± 0.2°, 18.18° ± 0.2°, 20.28° ± 0.2° and 21.82° ± 0.2° 2θ, as determined by using Cu-Kα radiation,

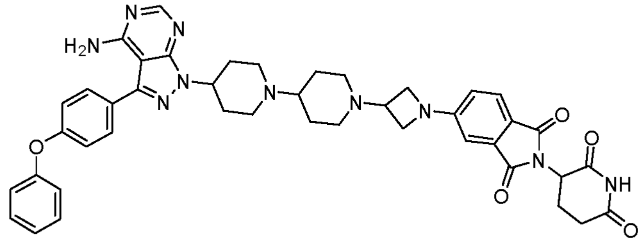

(Ib).

**18.** The crystal form I of the compound as shown in formula (Ib) according to claim 17, wherein the crystal form I has an X-ray powder diffraction pattern further comprising characteristic diffraction peaks at 11.92° ± 0.2°, 12.74° ± 0.2° and 17.44° ± 0.2° 2θ, as determined by using Cu-Kα radiation.

**19.** The crystal form I of the compound as shown in formula (Ib) according to claim 18, wherein the crystal form I has an X-ray powder diffraction pattern further comprising characteristic diffraction peaks at 9.76° ± 0.2°, 11.26° ± 0.2°, 14.14° ± 0.2°, 17.04° ± 0.2°, 23.23° ± 0.2°, 24.06 ± 0.2°, 25.26° ± 0.2° and 26.42 ± 0.2° 2θ, as determined by using Cu-Kα radiation.

**20.** The crystal form I of the compound as shown in formula (Ib) according to claim 19, wherein the crystal form I, as determined by using Cu-Kα radiation, has an X-ray powder diffraction pattern substantially as shown in Figure 13-1 and/or Figure 13-2.

**21.** The crystal form I of the compound as shown in formula (Ib) according to claim 19, **characterized in that** the crystal form I has a differential scanning calorimetry (DSC) curve as shown in Figure 14 or a thermogravimetric analysis curve as shown in Figure 15.

**22.** A method for preparing the pharmaceutical salt according to claim 1, wherein the method comprises: a step of allowing the compound as shown in formula (I) and an acid to form a salt.

**23.** The method according to claim 22, wherein a solvent used therein is selected from one or more of a $C_{1-6}$ halogenated alkane solvent, a $C_{2-6}$ ester solvent, a $C_{2-6}$ ether solvent, a $C_{1-6}$ alcohol solvent or water.

**24.** The method according to claim 23, wherein the solvent used therein is selected from one or more of dichloromethane, 1,2-dichloroethane, ethyl acetate, methanol, ethanol, isopropanol, diethyl ether, tetrahydrofuran and water.

**25.** The method according to claim 24, wherein the solvent used therein is selected from one or more of dichloromethane, methanol, ethanol and water.

**26.** The method according to claim 23, wherein the method comprises: preparing the compound as shown in formula (II) by using the compound as shown in formula (Ia) and maleic acid as raw materials.

**27.** A method for preparing the crystal form of the compound as shown in formula (Ia), (Ib) or (II), wherein the method comprises a step of preparing the compound as shown in formula (II), (Ia) or (Ib) in any crystal form or the compound as shown in formula (II), (Ia) or (Ib) in an amorphous form by means of recrystallization or slurrying, wherein a solvent for the recrystallization or slurrying is selected from one of or a mixed solvent of two or more of a $C_{2-6}$ ester solvent, a $C_{2-6}$ ether solvent, a $C_{1-6}$ alcohol solvent, a $C_{1-6}$ nitrile solvent, an alkane solvent and water.

**28.** The method according to claim 27, wherein the solvent for the recrystallization or slurrying is selected from one of or a mixed solvent of two or more of ethyl acetate, isopropyl acetate, n-heptane, acetonitrile, tetrahydrofuran, trifluoroethanol, methanol, ethanol and water.

**29.** The method according to claim 27, wherein the recrystallization or slurrying is performed at a temperature of 4°C to 100°C, preferably room temperature to 90°C, more preferably 40°C to 90°C.

**30.** The method according to claim 29, wherein the crystal form is the crystal form I of the compound as shown in formula (II), and the method comprises steps of mixing the compound as shown in formula (II) with a suitable solvent to form a suspension, heating, stirring and slurrying the mixture, leaving the resulting product to stand for crystallization, and performing filtering and separation, wherein the solvent is selected from ethanol.

**31.** The method according to claim 29, wherein the crystal form is the crystal form III of the compound as shown in formula (Ia), and the method comprises a step of mixing the compound as shown in formula (Ia) in an amorphous form with a suitable solvent, heating, stirring and slurrying the mixture, and performing filtering and separation, wherein the solvent is selected from an acetonitrile/water mixed solvent.

**32.** A pharmaceutical composition, wherein the pharmaceutical composition contains a therapeutically effective amount of the pharmaceutical salt of the compound according to any one of claims 1 to 4 or the crystal form according to any one of claims 5 to 21, and a pharmaceutically acceptable excipient.

**33.** Use of the pharmaceutical salt of the compound according to any one of claims 1 to 4, the crystal form according to claim 5, 11 or 17 or the pharmaceutical composition according to claim 32 in the preparation of a drug for treating and/or preventing tumour or cancer.

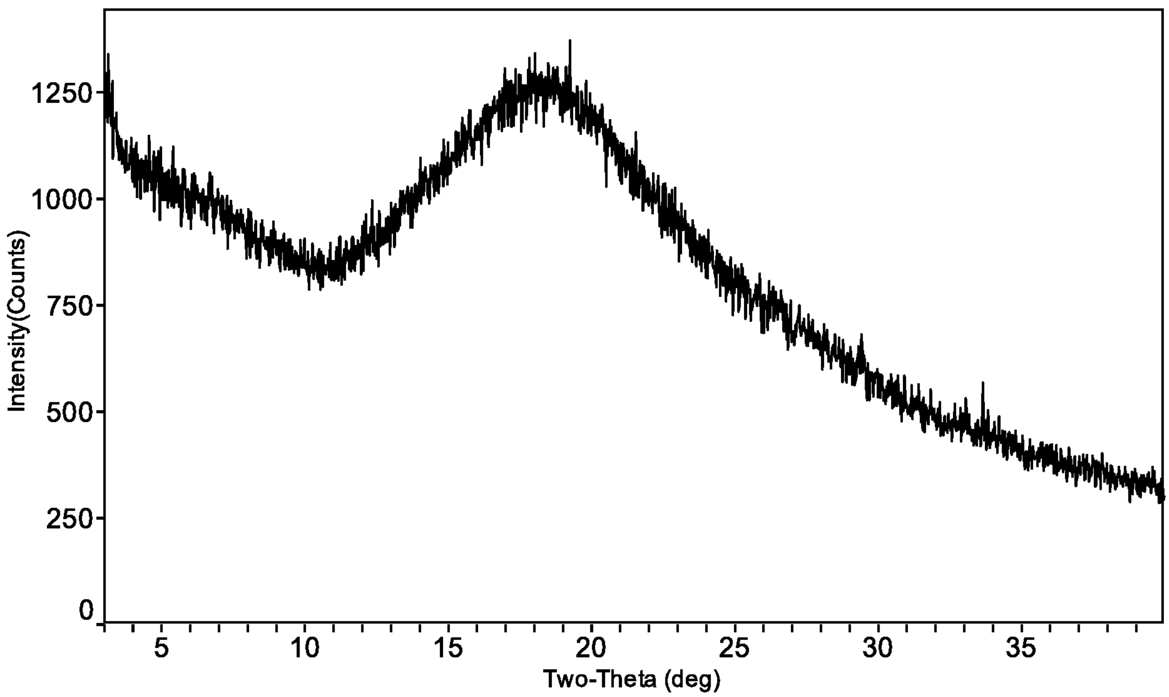


XRD pattern of an amorphous form of compound 1

*FIG. 1*

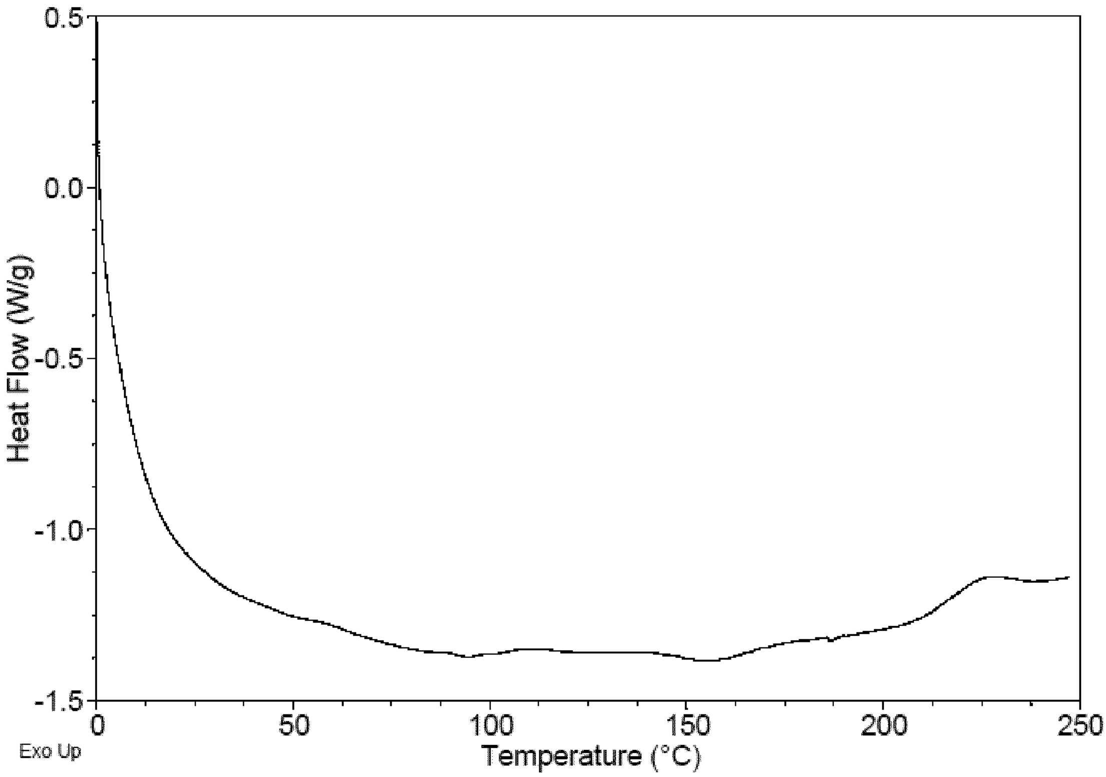


DSC pattern of an amorphous form of compound 1

*FIG. 2*

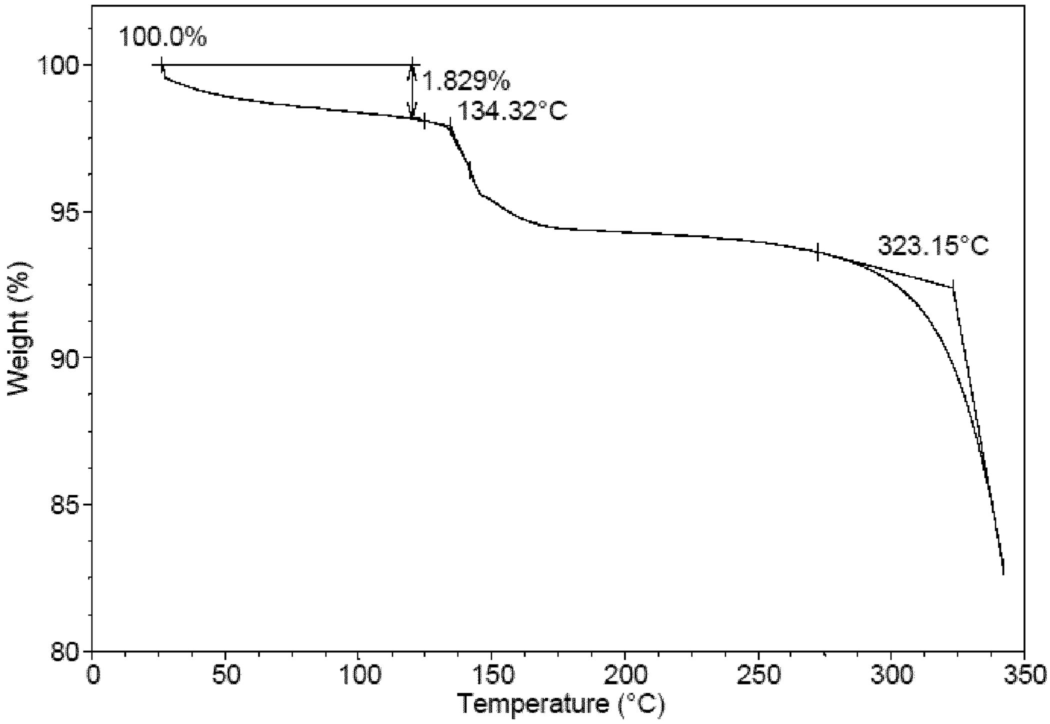


TGA pattern of an amorphous form of compound 1

*FIG. 3*

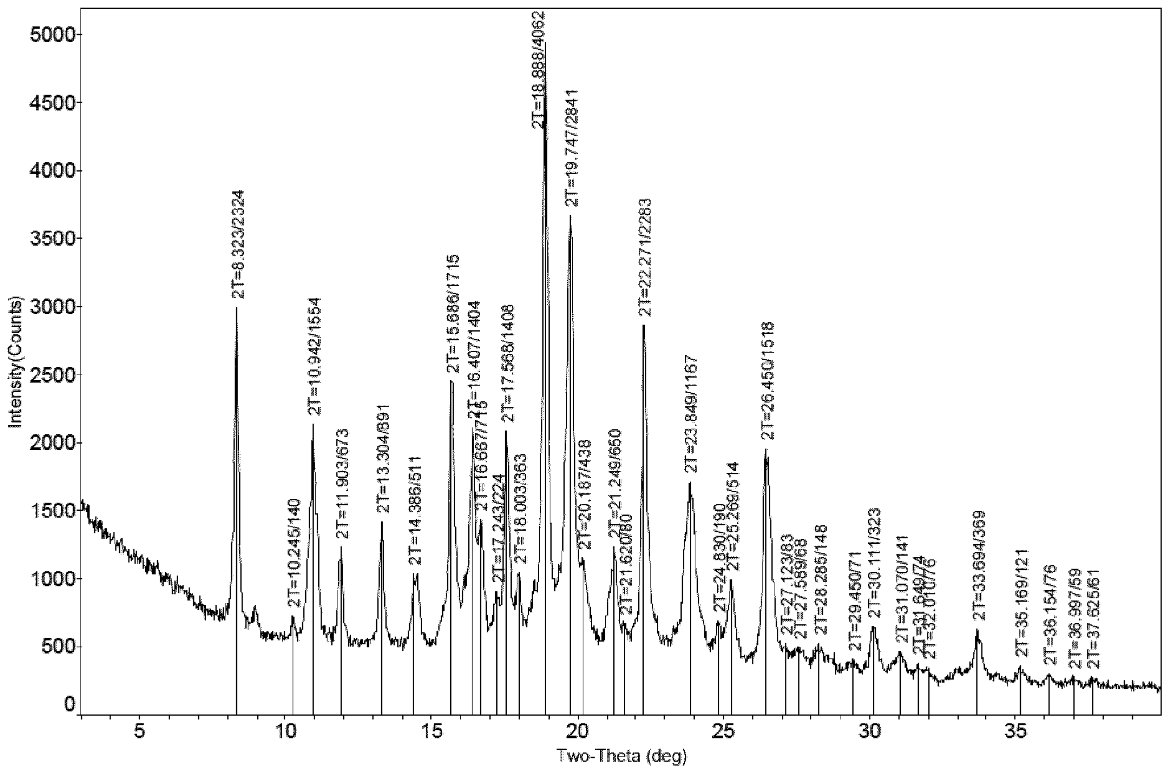


XRD pattern of a crystal form I of compound 1

*FIG. 4*

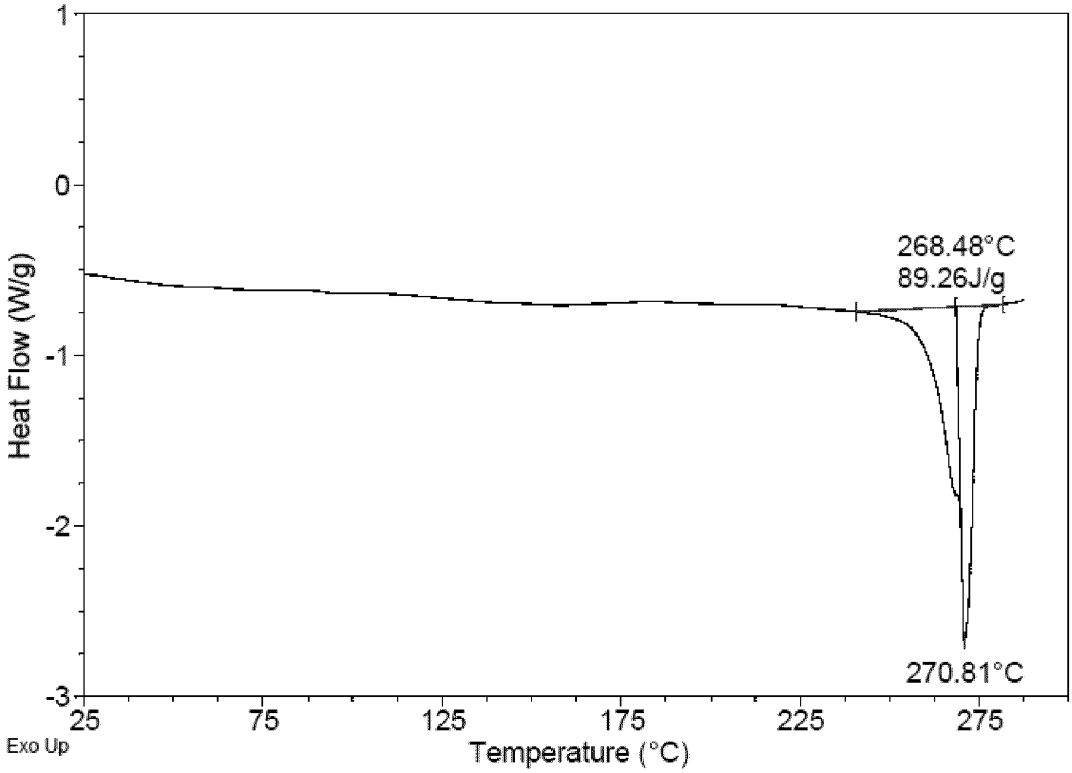

DSC pattern of a crystal form I of compound 1

*FIG. 5*

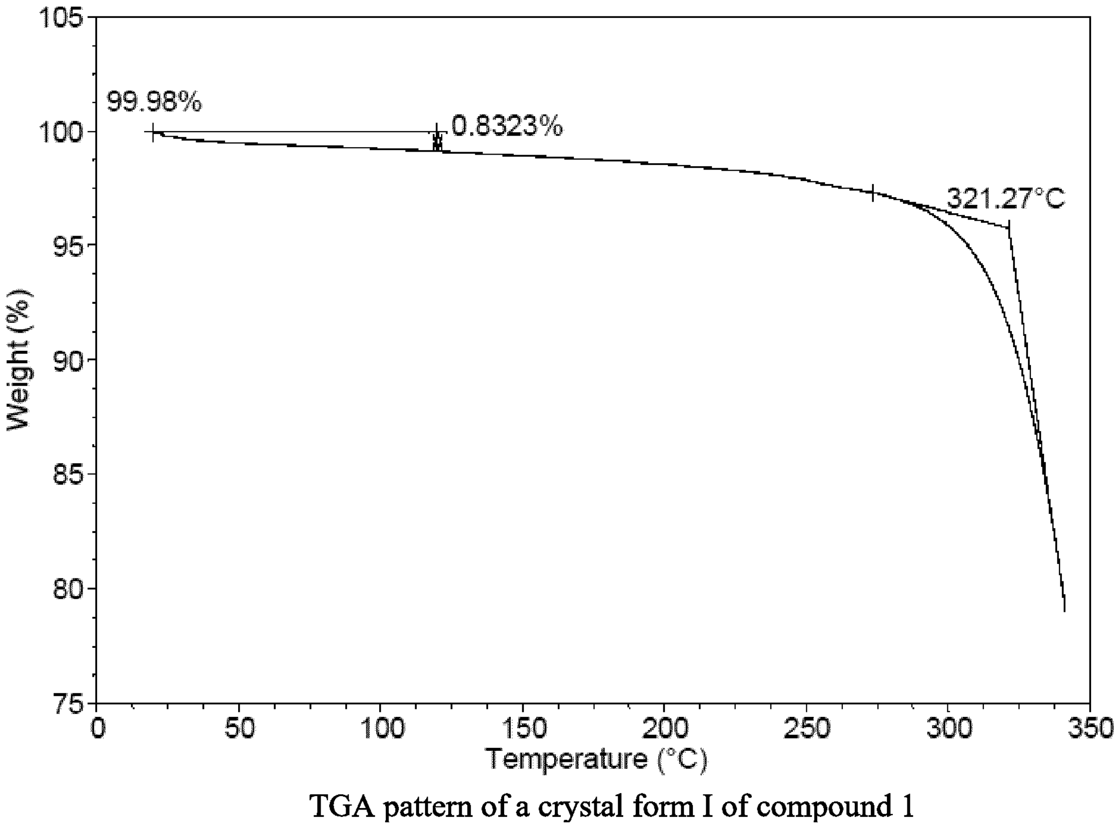

TGA pattern of a crystal form I of compound 1

*FIG. 6*

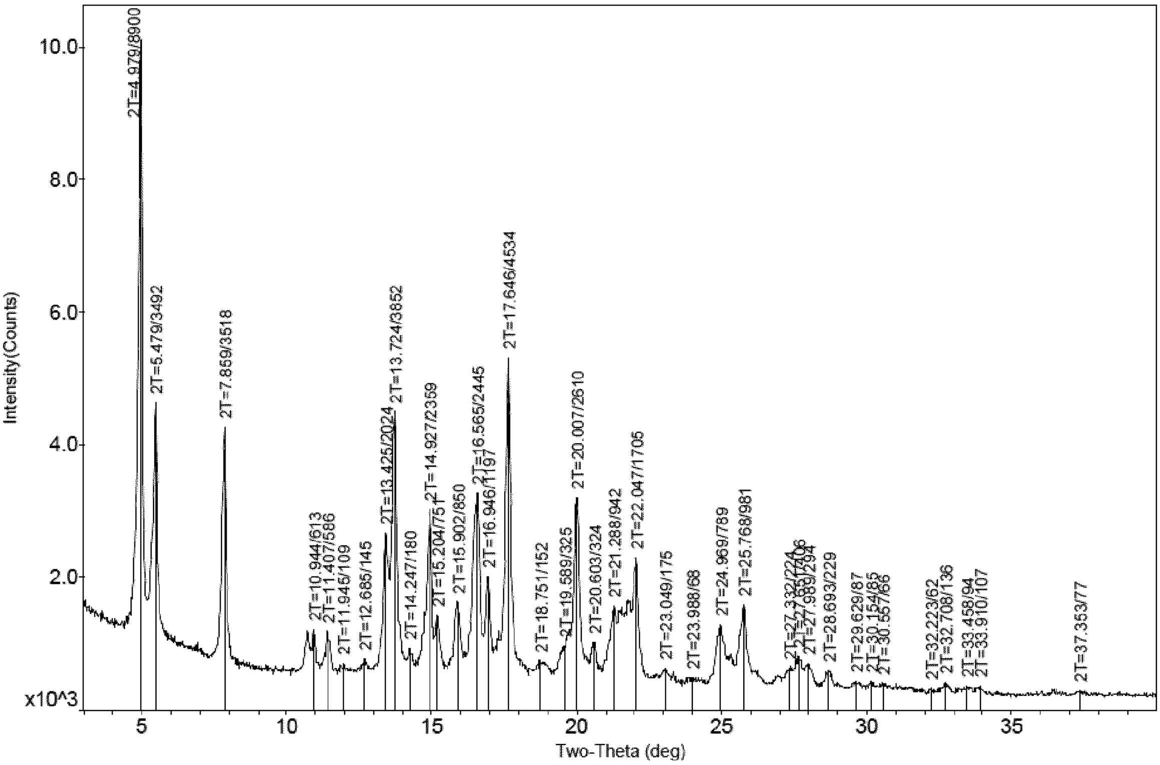


XRD pattern of a crystal form II of compound 1

*FIG. 7*

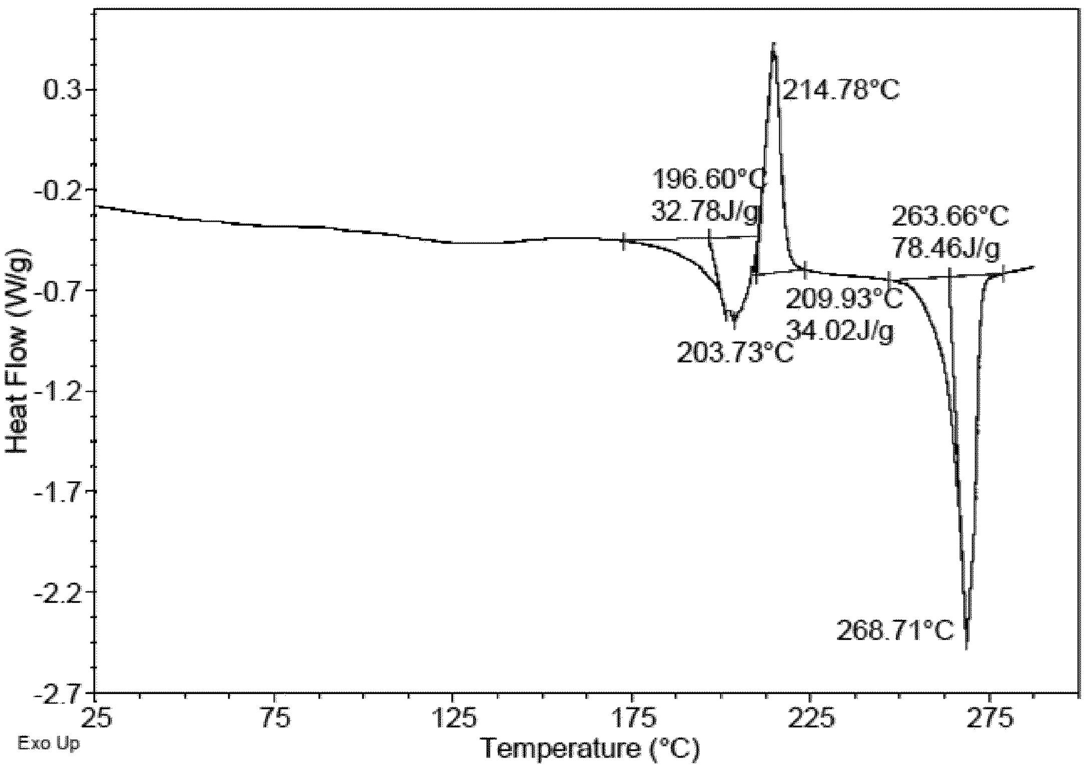


DSC pattern of a crystal form II of compound 1

*FIG. 8*

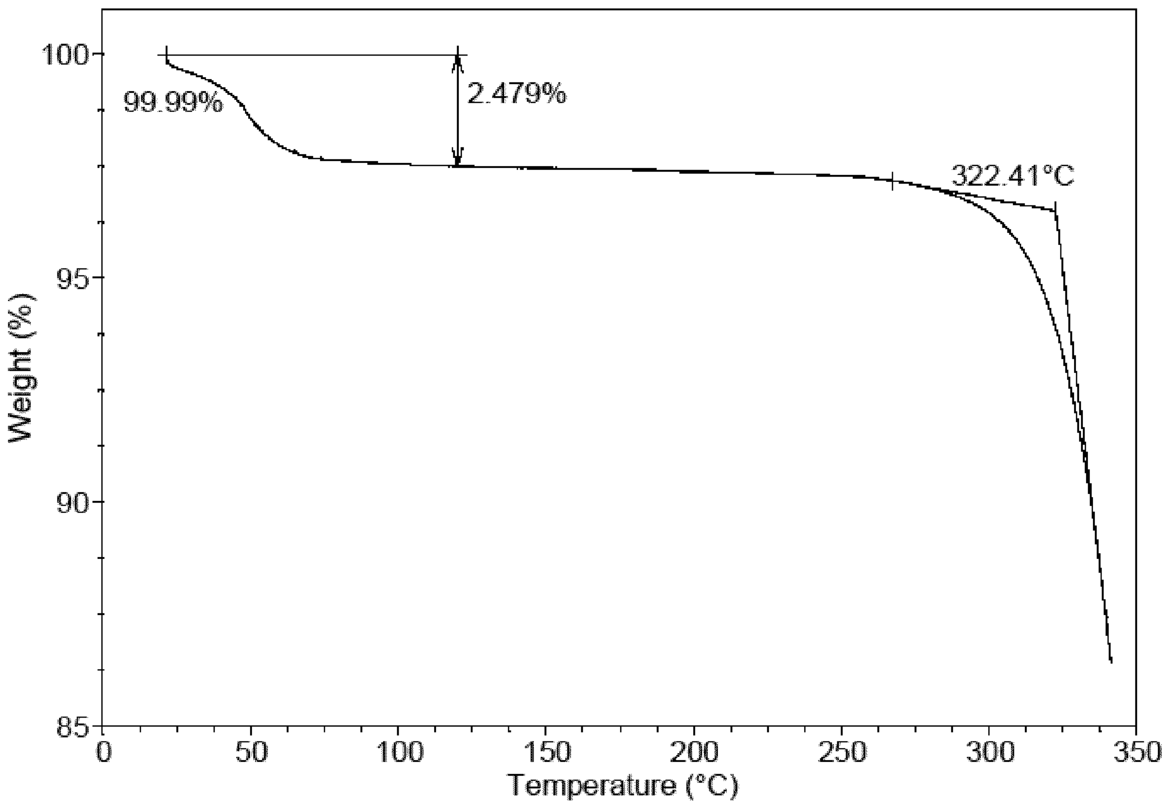


TGA pattern of a crystal form II of compound 1

*FIG. 9*

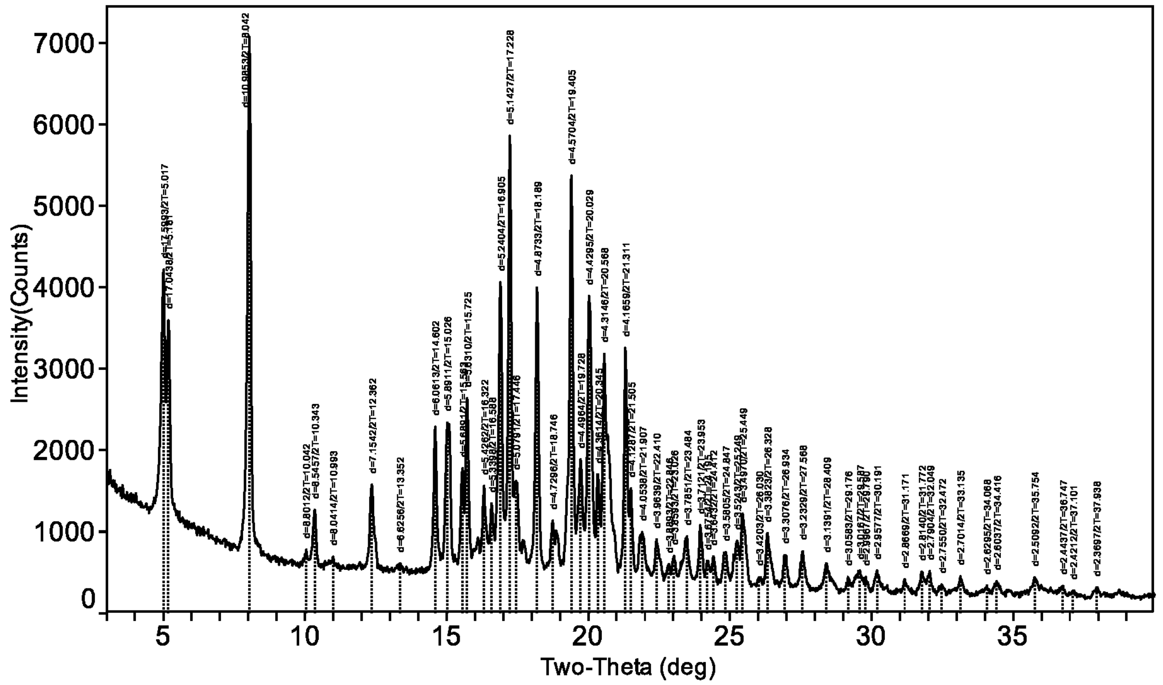


XRD pattern of a crystal form III of compound 1

*FIG. 10*

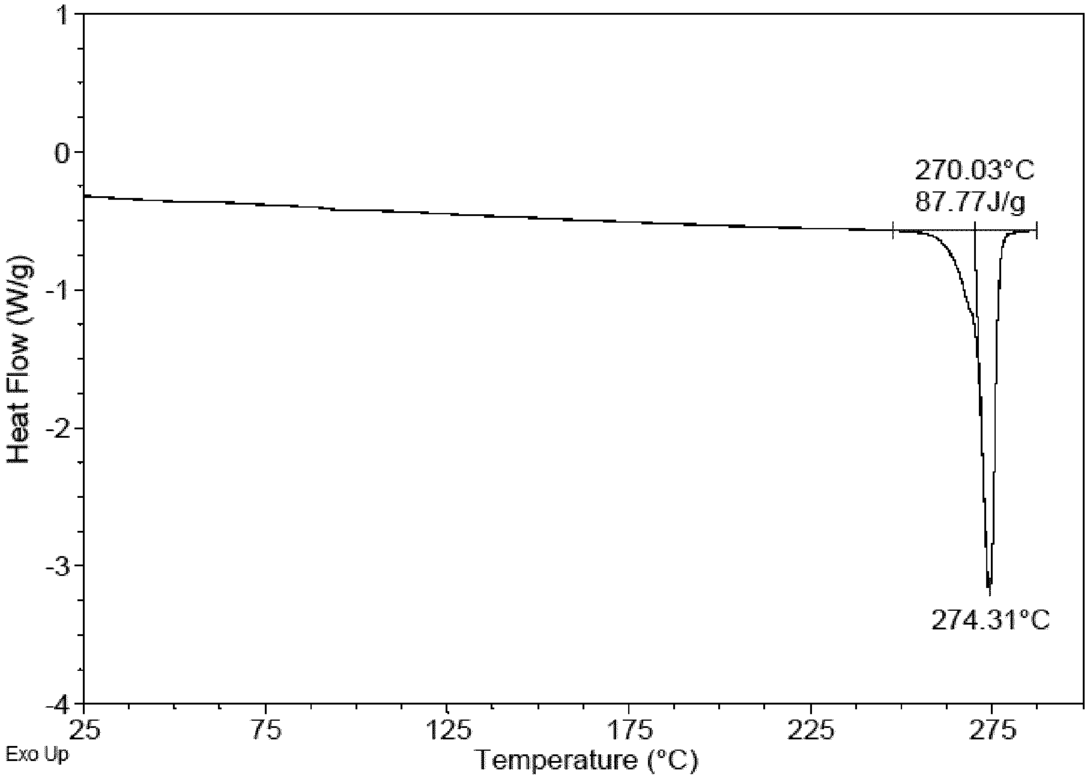


DSC pattern of a crystal form III of compound 1

*FIG. 11*

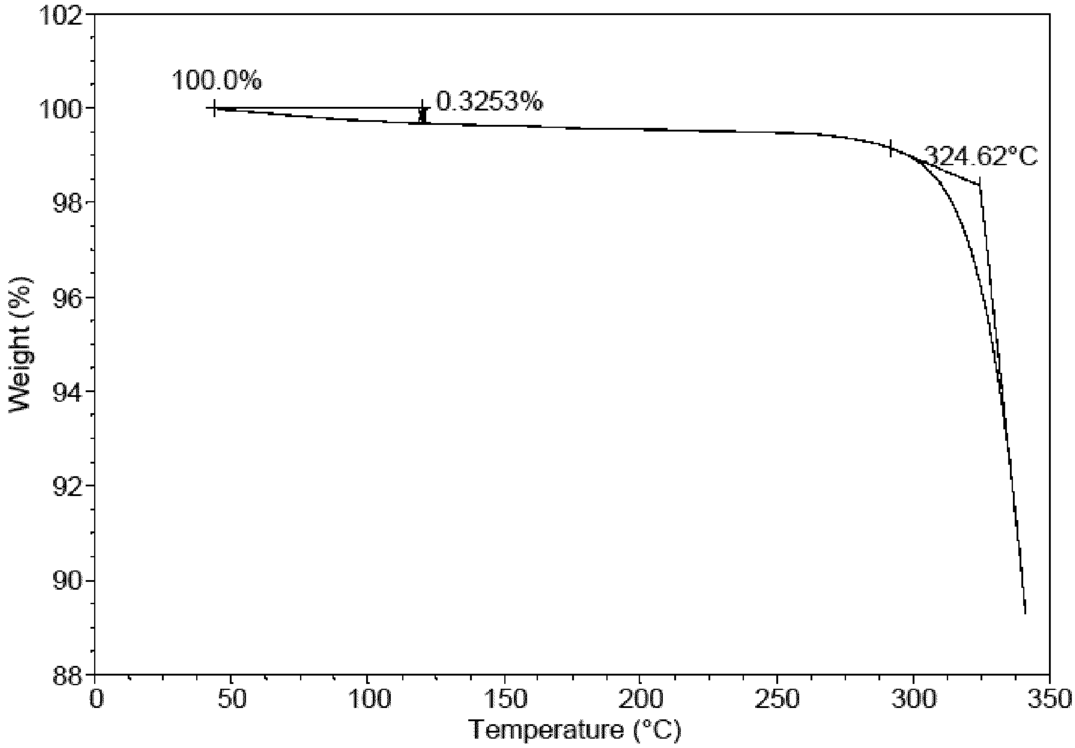


TGA pattern of a crystal form III of compound 1

*FIG. 12*

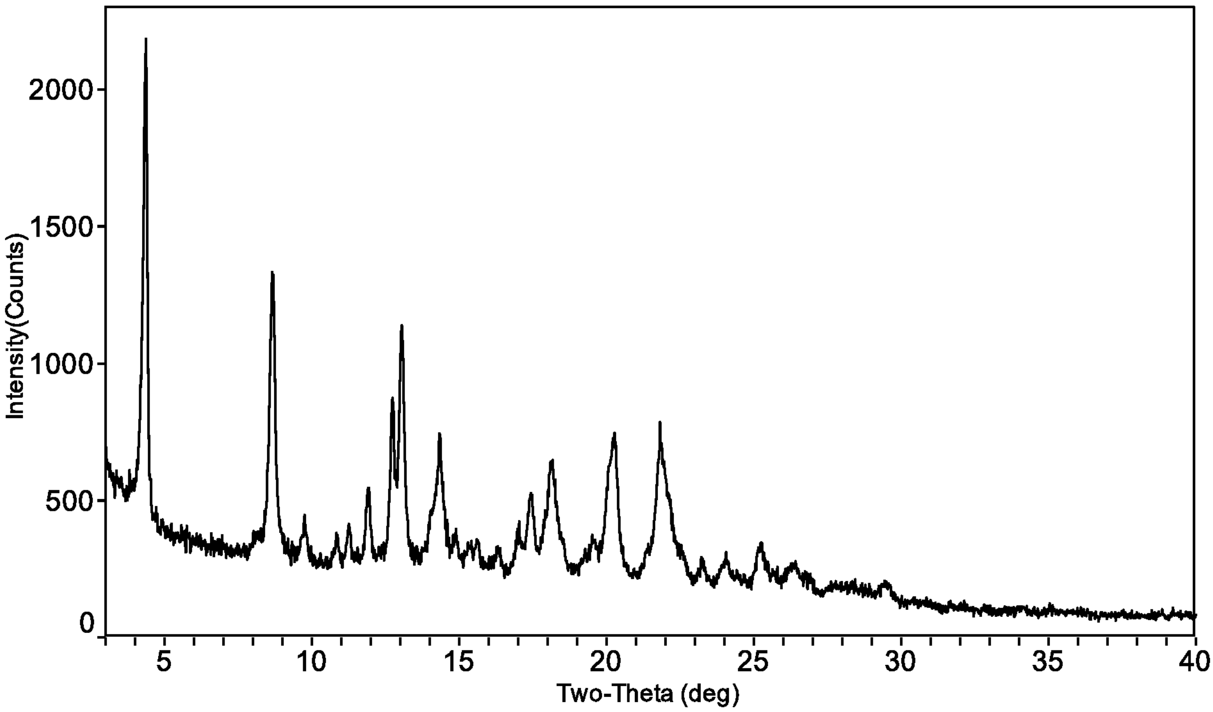


XRD pattern of a crystal form I of compound 2

*FIG. 13-1*

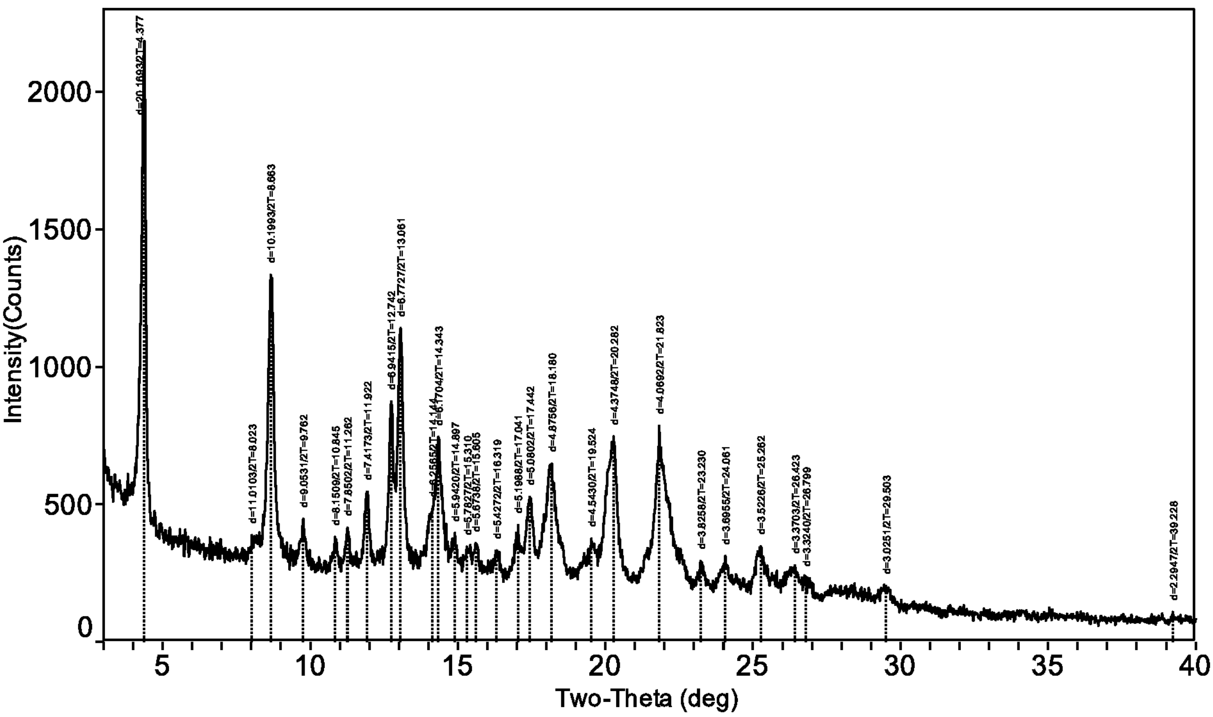


XRD pattern of a crystal form I of compound 2

*FIG. 13-2*

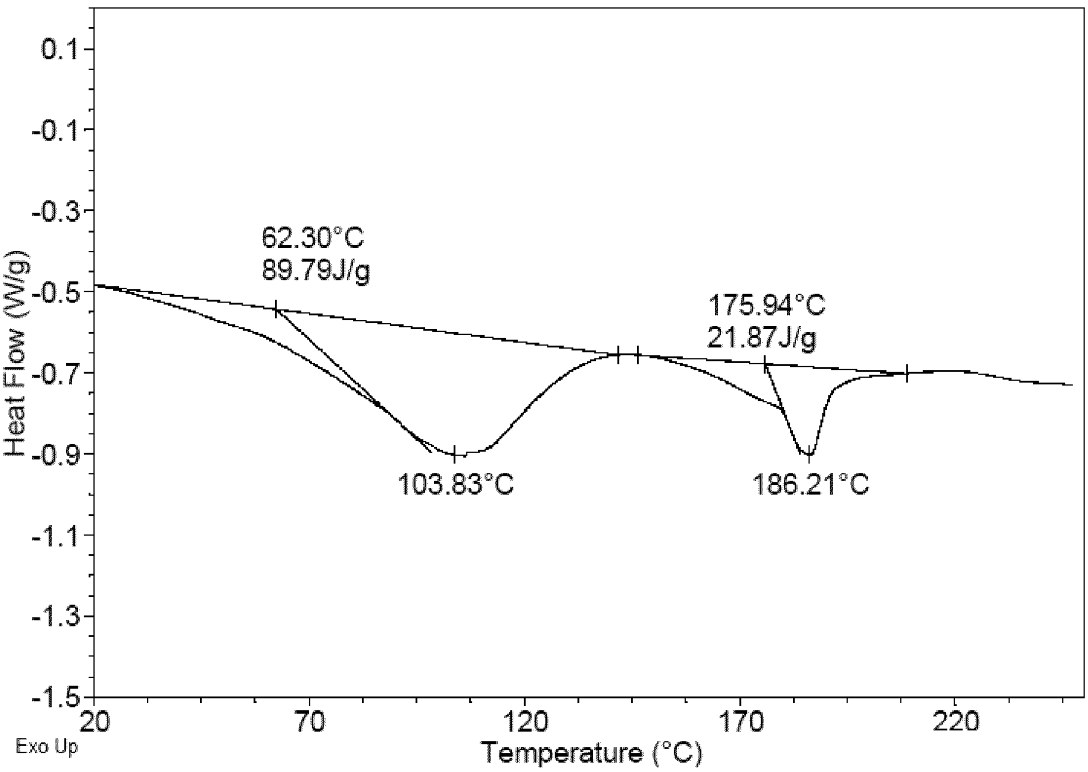


DSC pattern of a crystal form I of compound 2

*FIG. 14*

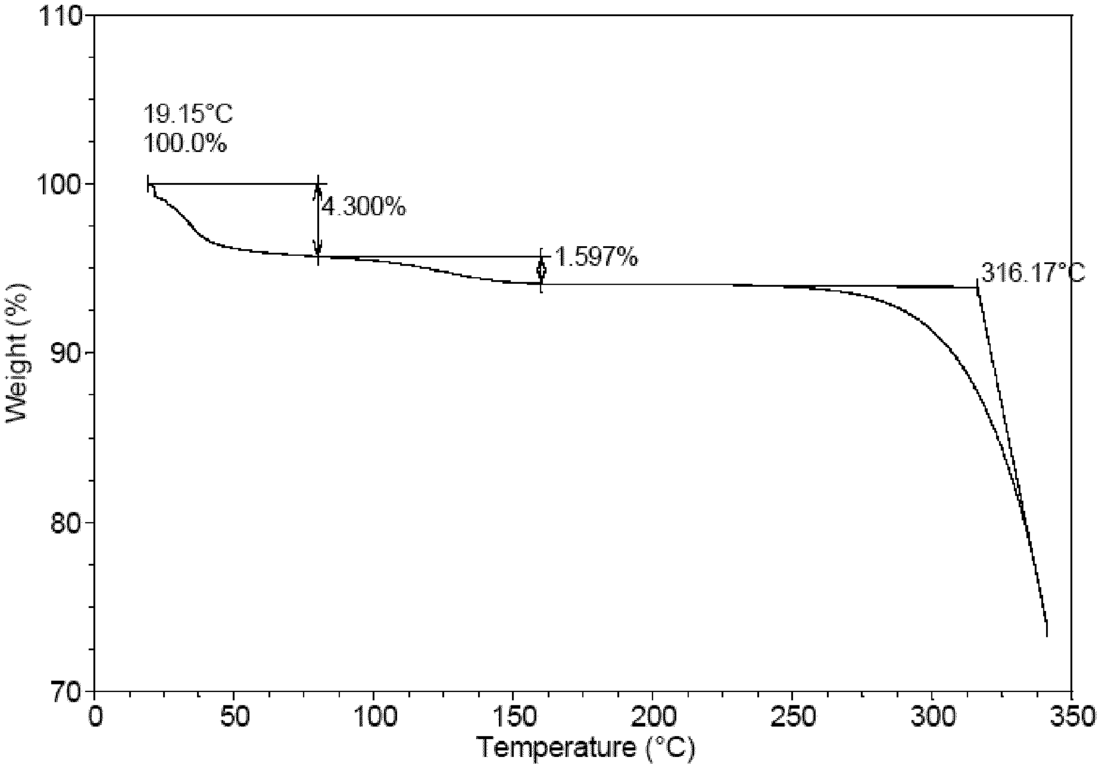


TGA pattern of a crystal form I of compound 2

*FIG. 15*

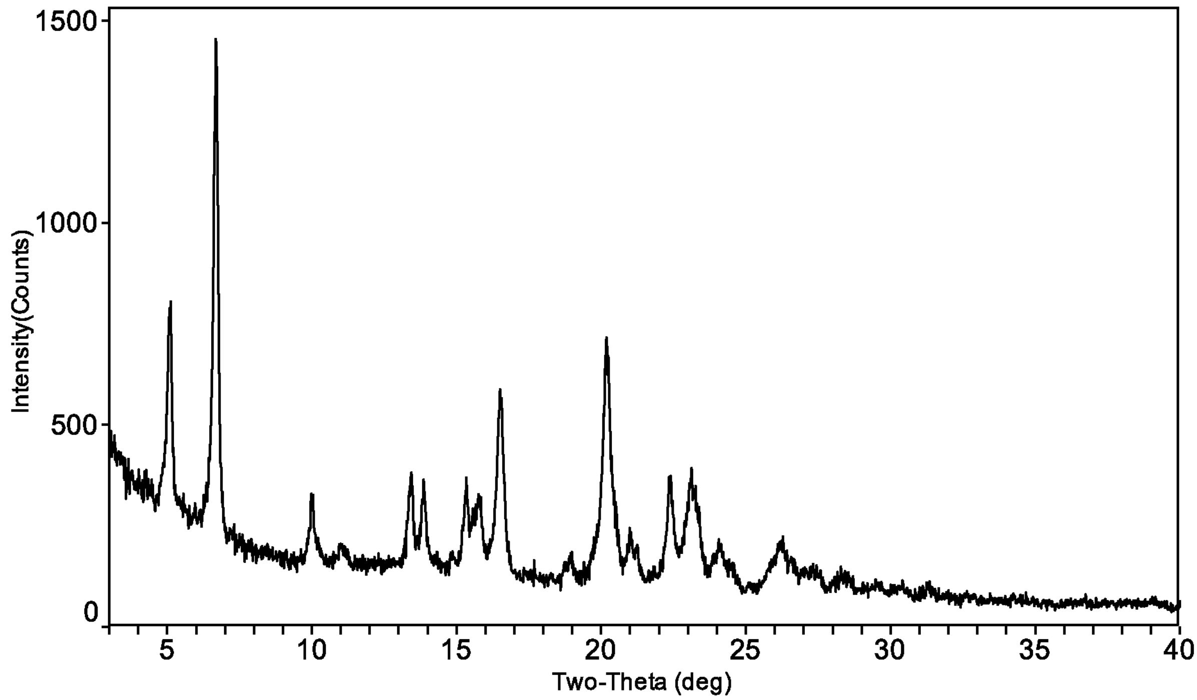


XRD pattern of a crystal form II of compound 2

*FIG. 16-1*

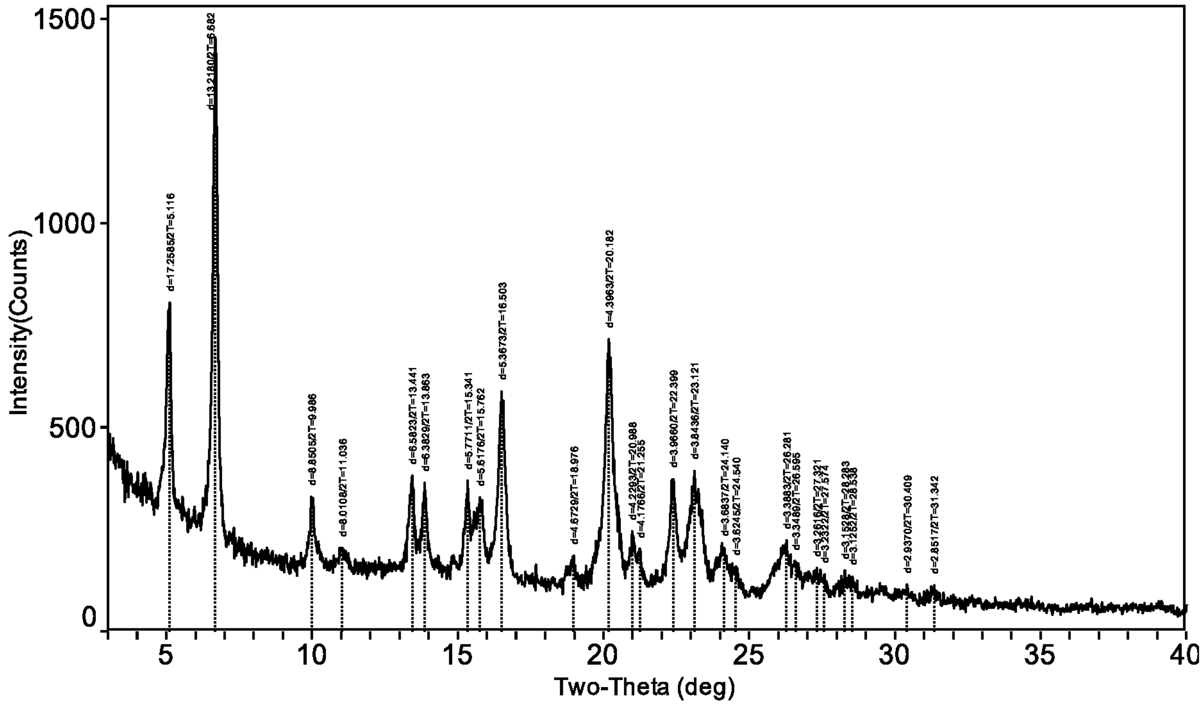


XRD pattern of a crystal form II of compound 2

*FIG. 16-2*

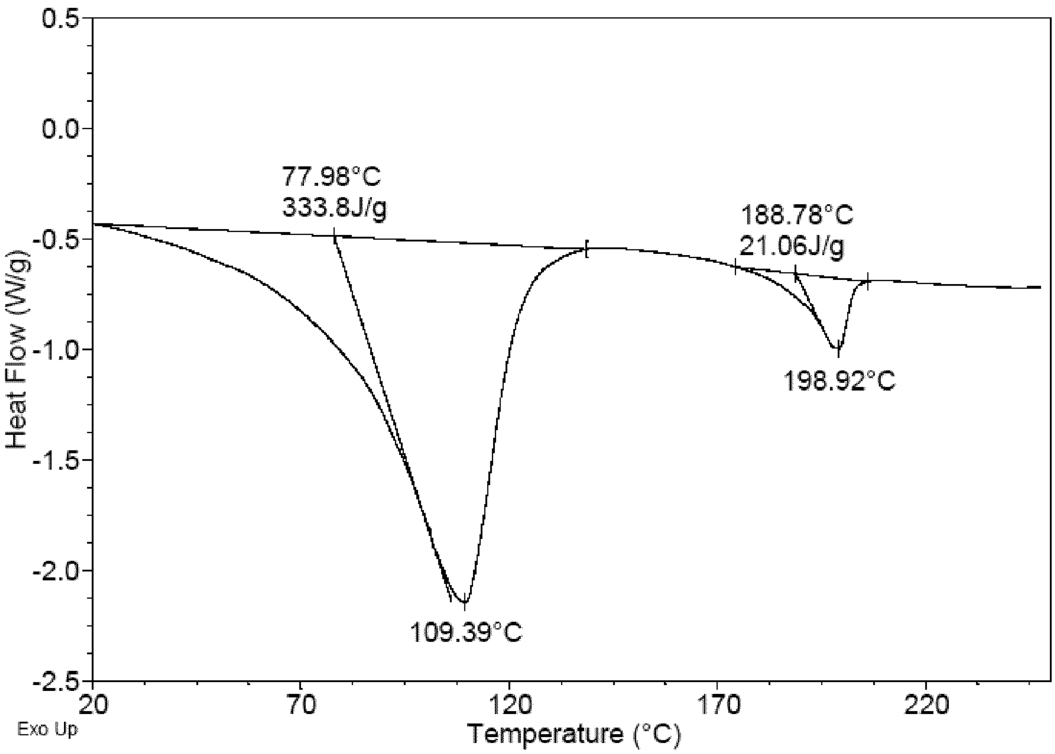


DSC pattern of a crystal form II of compound 2

*FIG. 17*

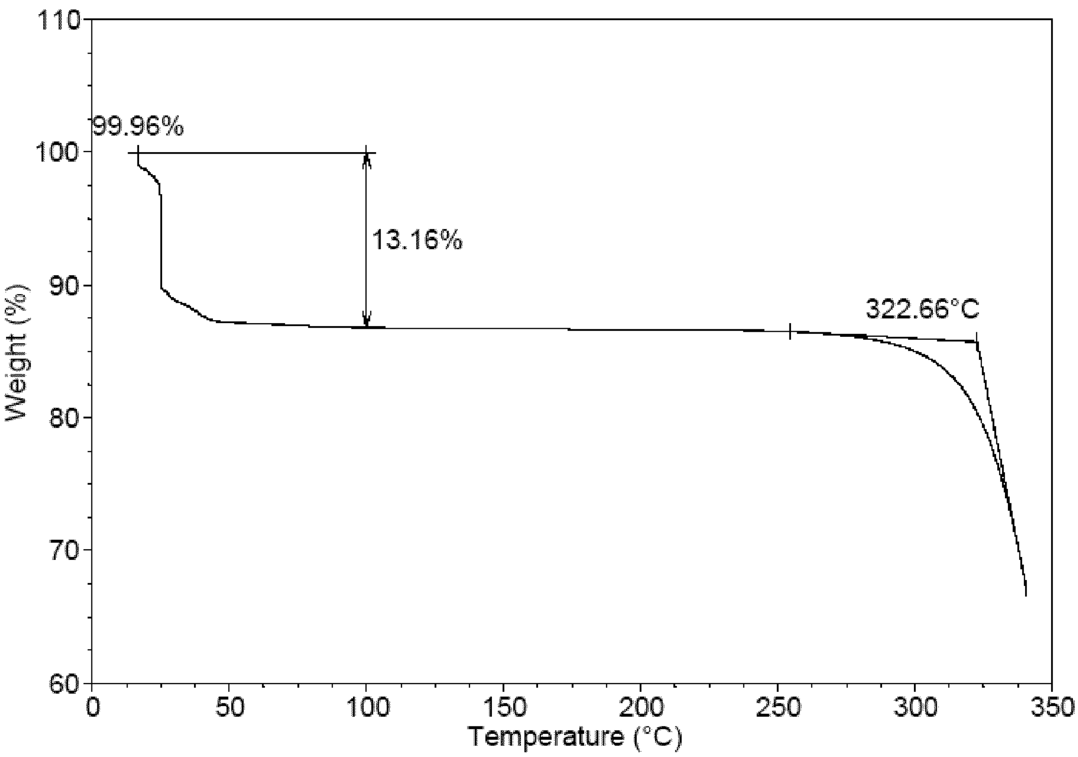


TGA pattern of a crystal form II of compound 2

*FIG. 18*

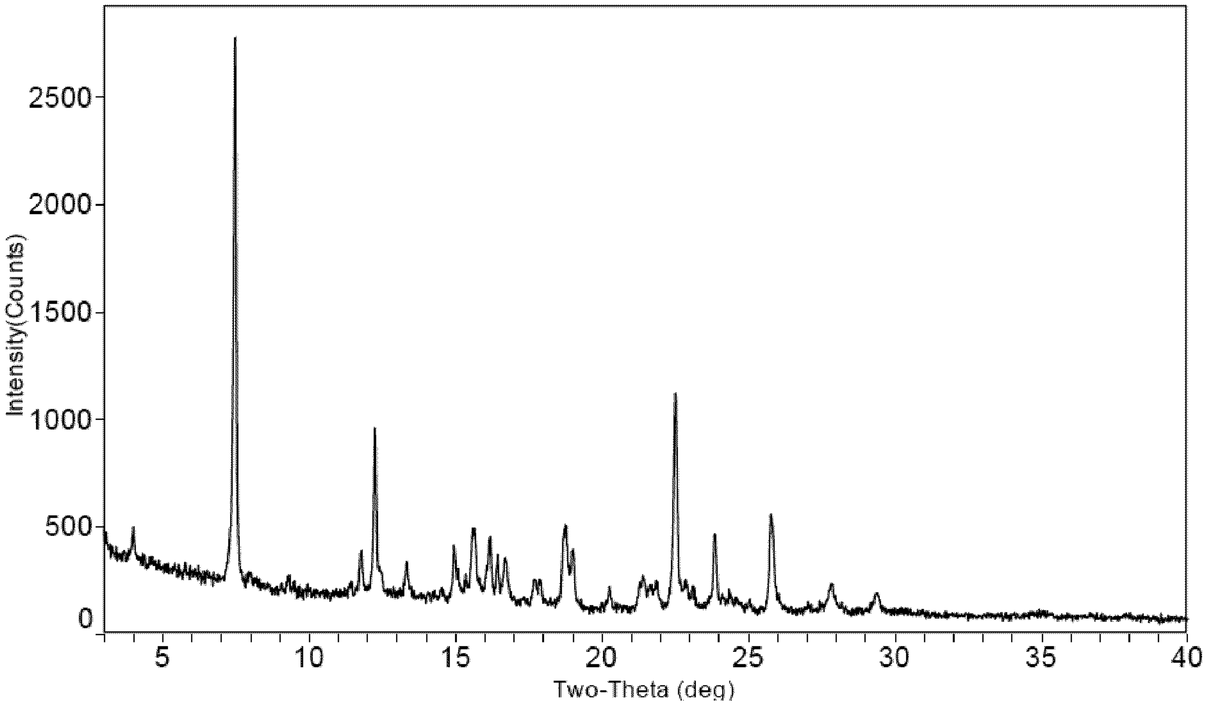


XRD pattern of a crystal form III of compound 2

*FIG. 19-1*

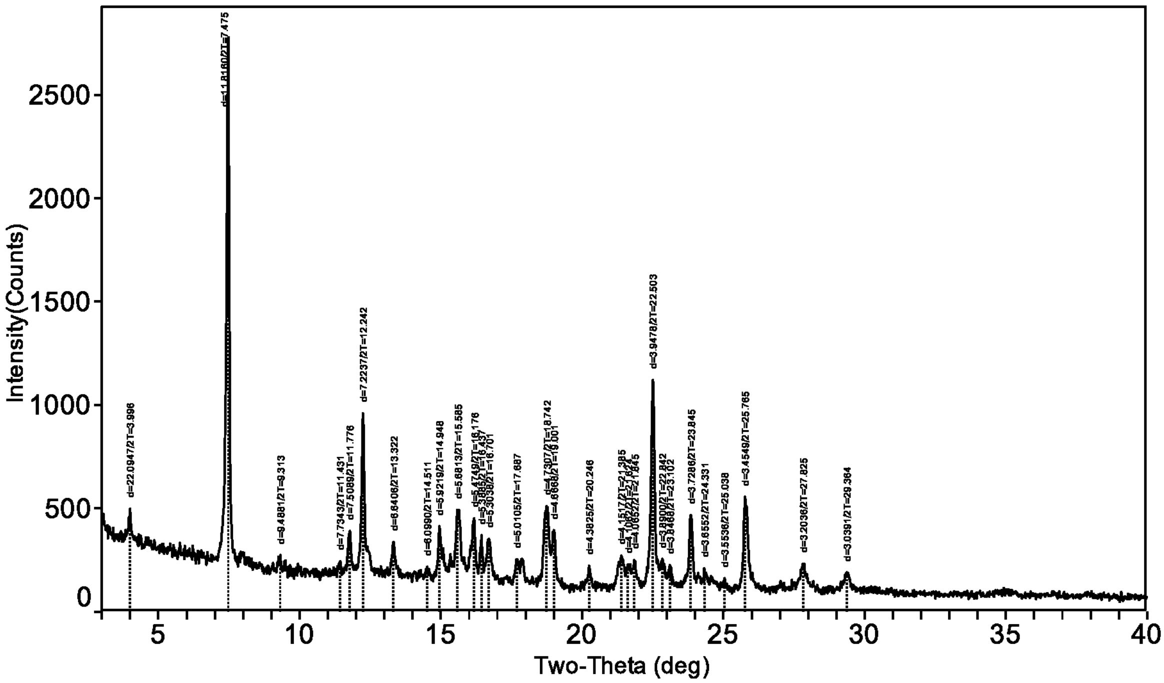


XRD pattern of a crystal form III of compound 2

*FIG. 19-2*

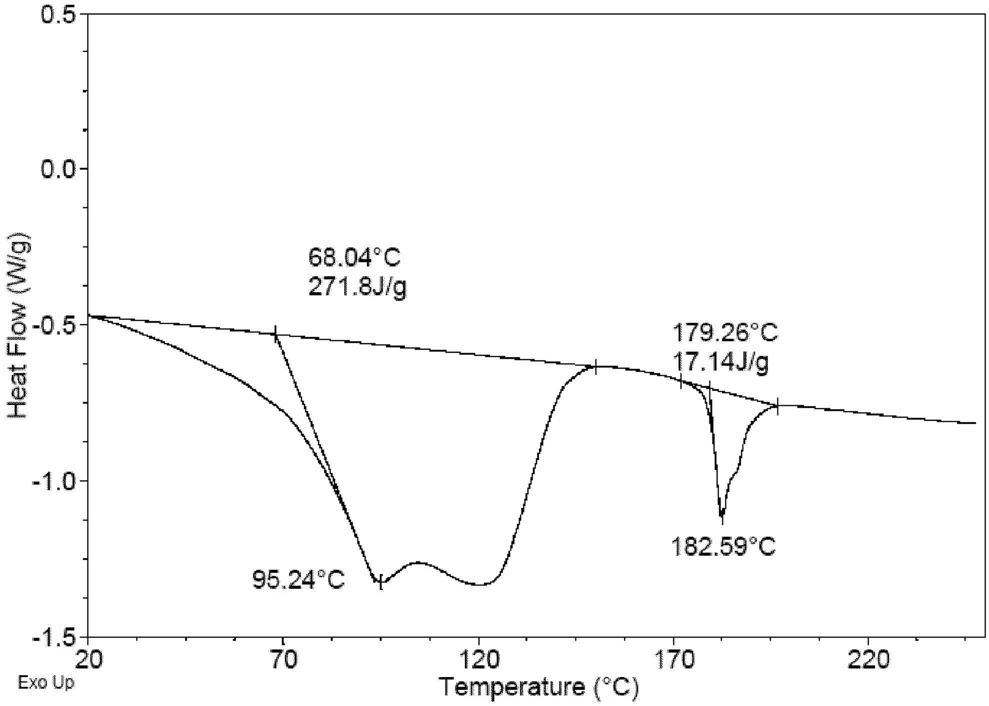


DSC pattern of a crystal form III of compound 2

*FIG. 20*

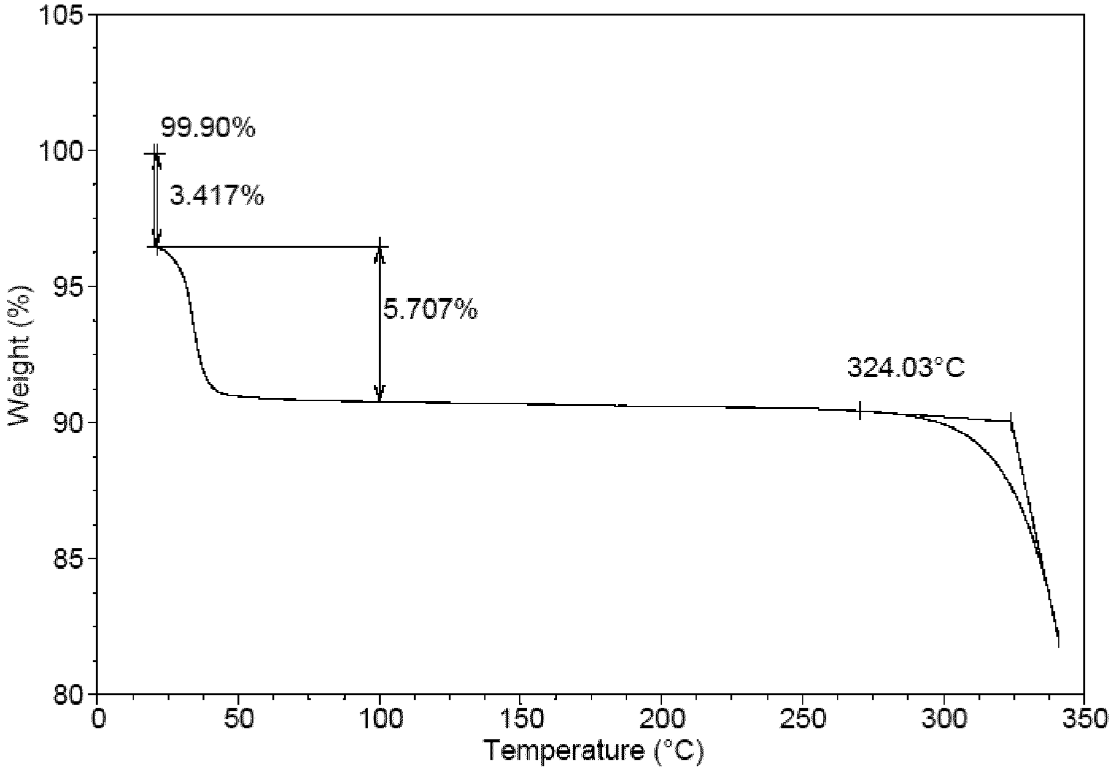


TGA pattern of a crystal form III of compound 2

*FIG. 21*

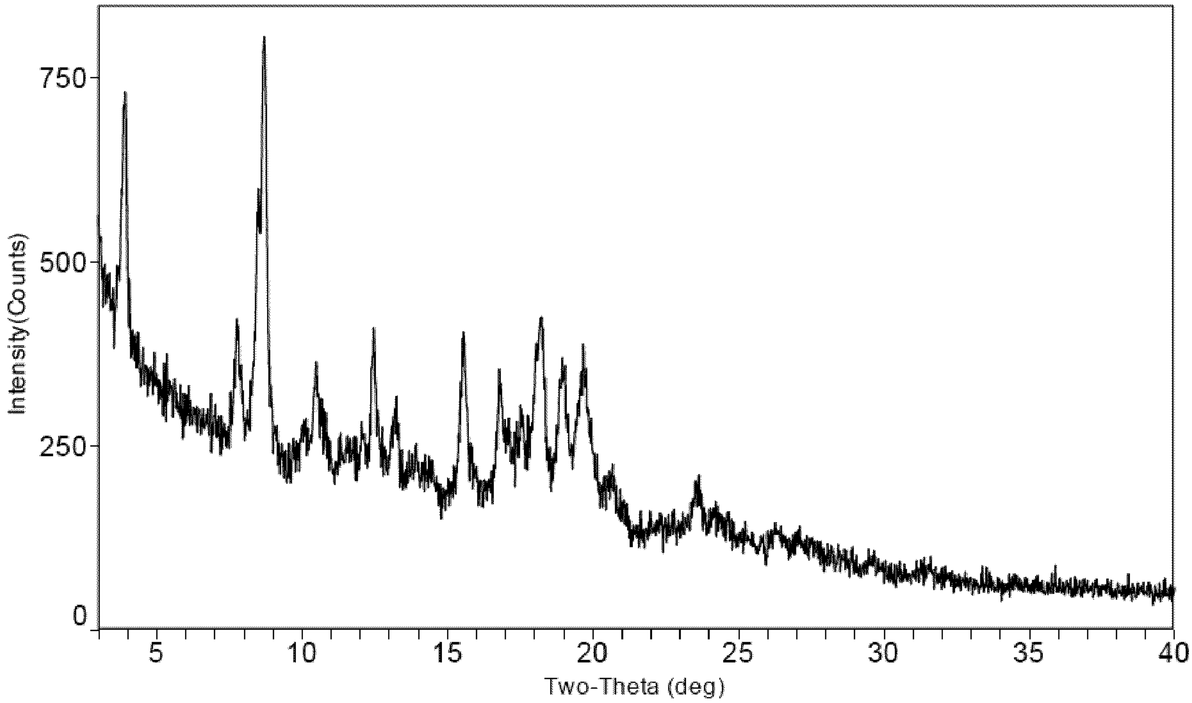


XRD pattern of a crystal form IV of compound 2

*FIG. 22-1*

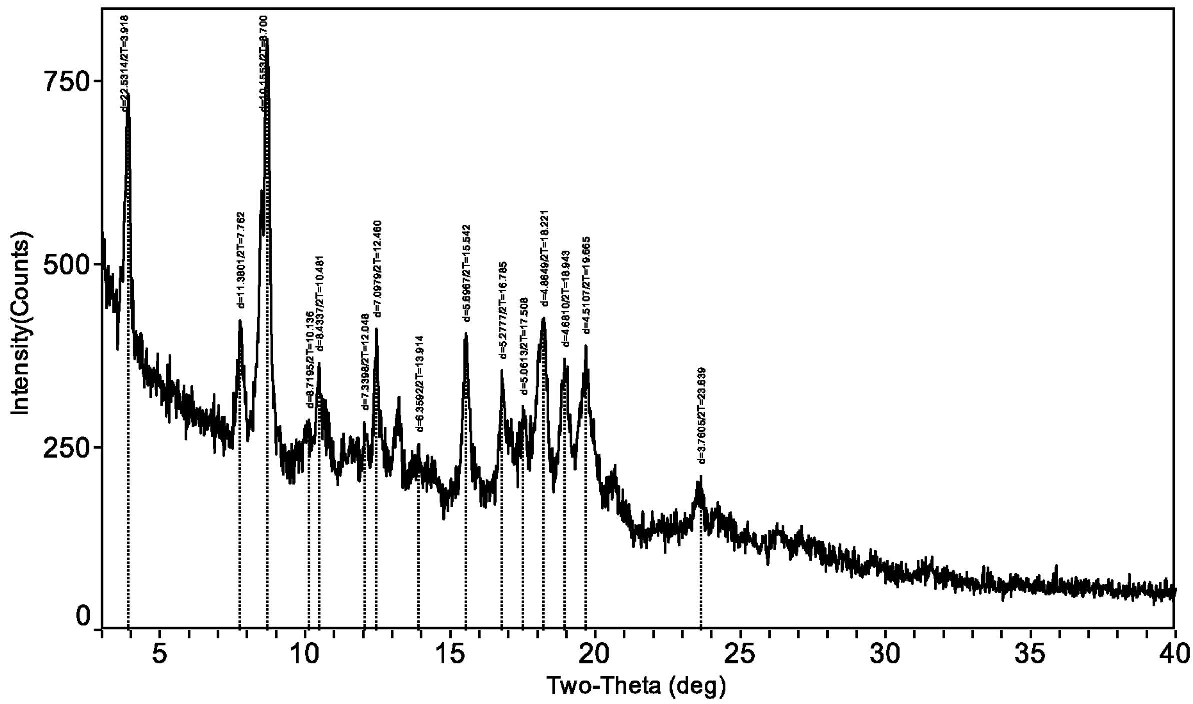


XRD pattern of a crystal form IV of compound 2

*FIG. 22-2*

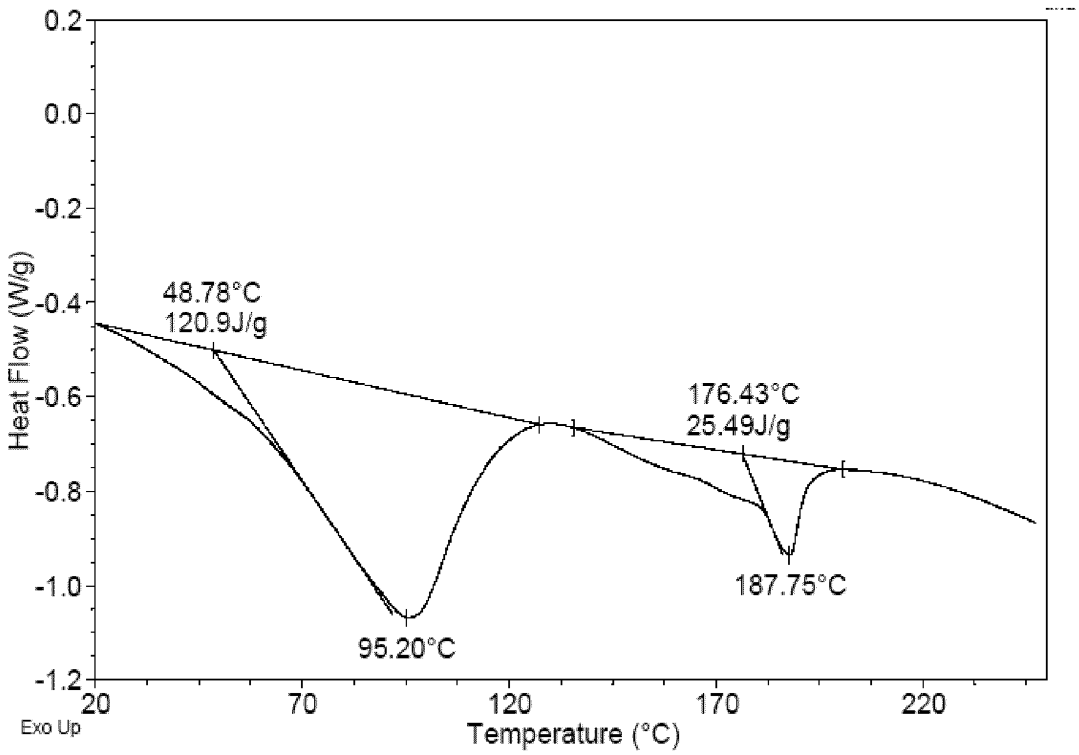


DSC pattern of a crystal form IV of compound 2

*FIG. 23*

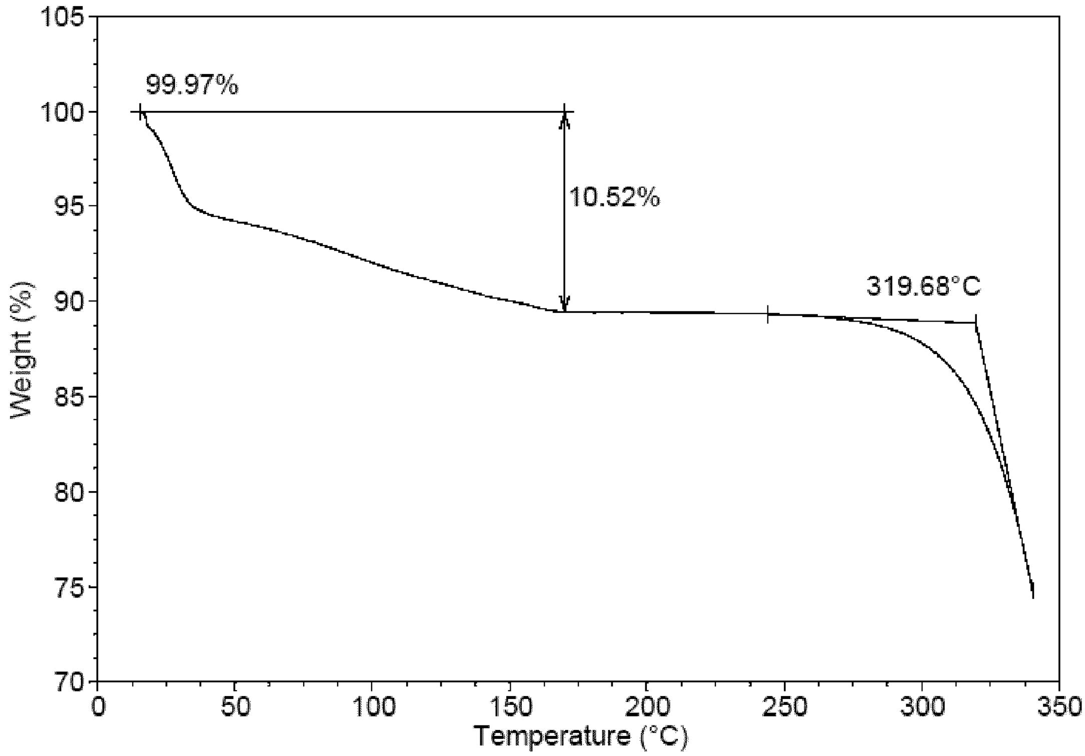


TGA pattern of a crystal form IV of compound 2

*FIG. 24*

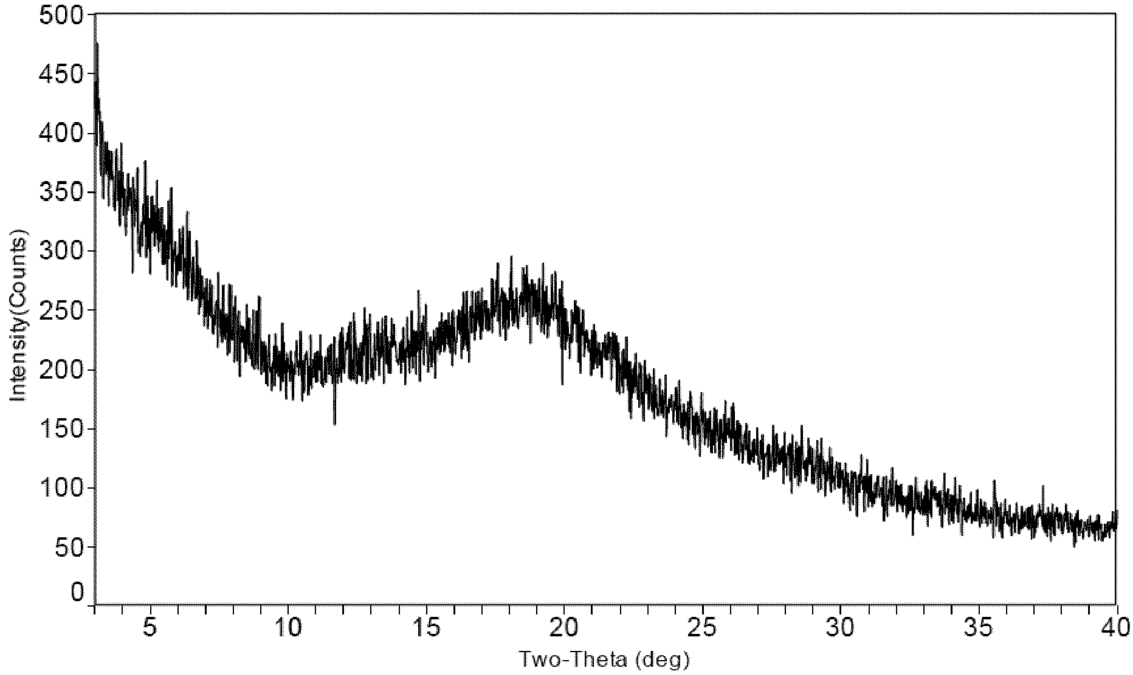


XRD pattern of an amorphous form of compound 2

*FIG. 25*

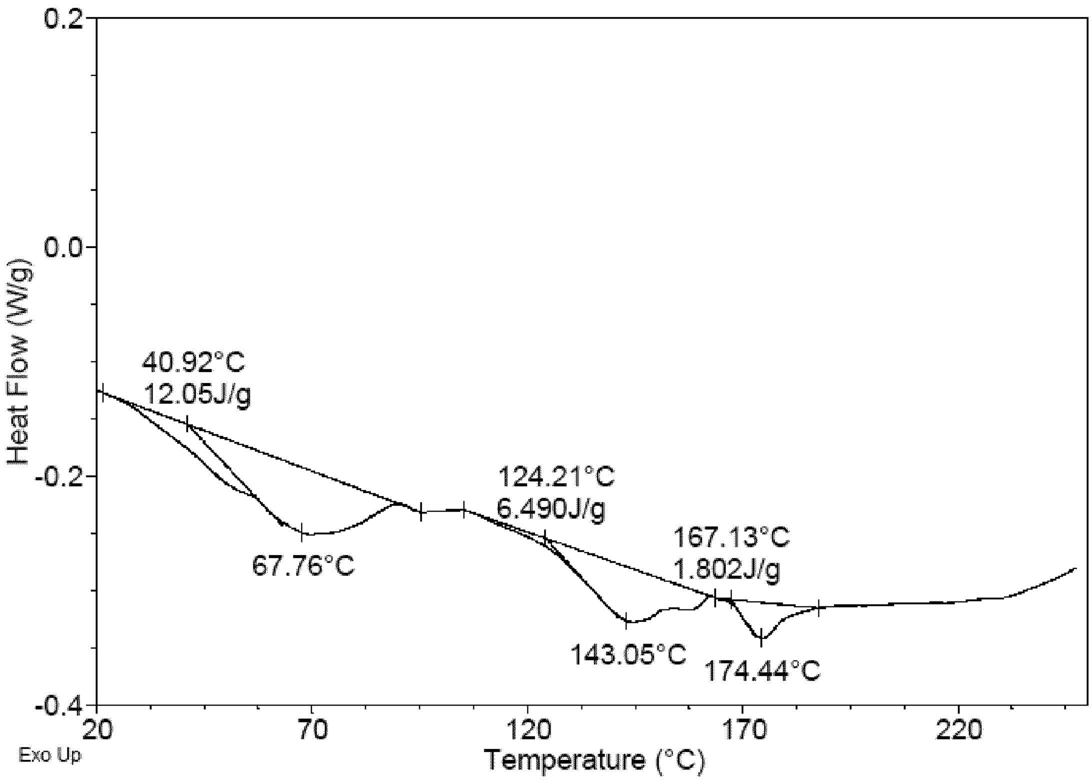


DSC pattern of an amorphous form of compound 2

*FIG. 26*

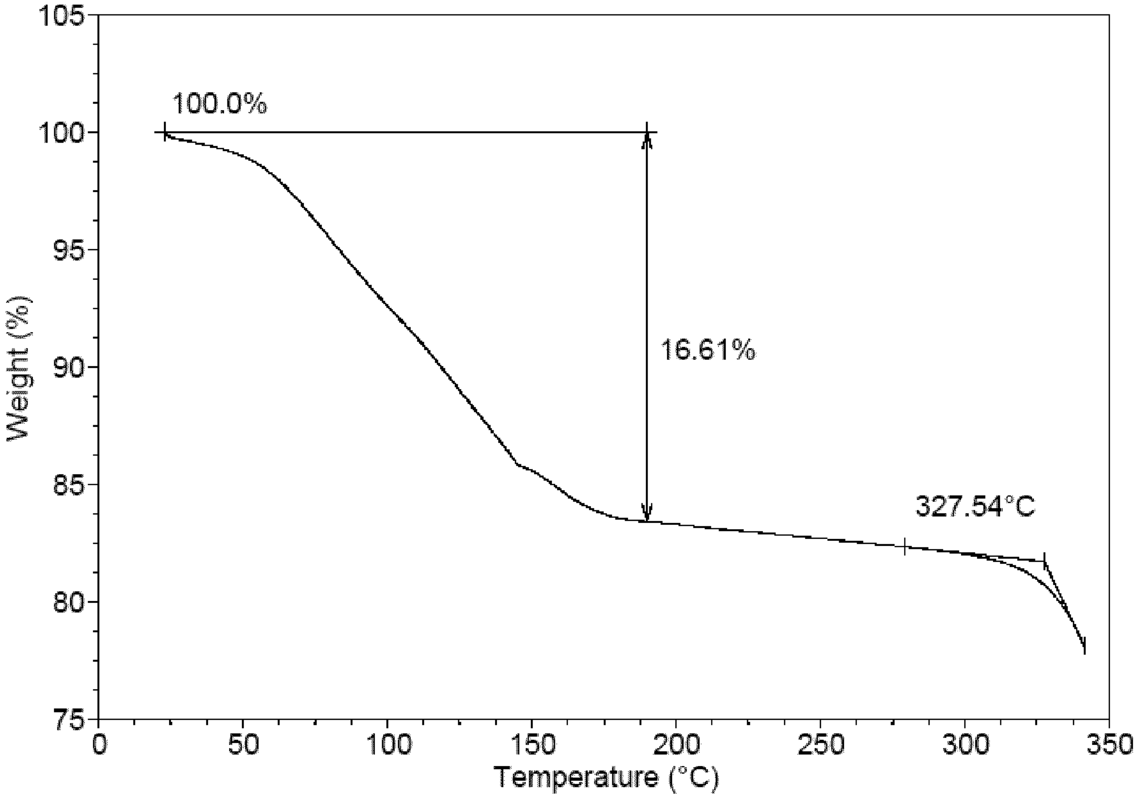


TGA pattern of an amorphous form of compound 2

*FIG. 27*

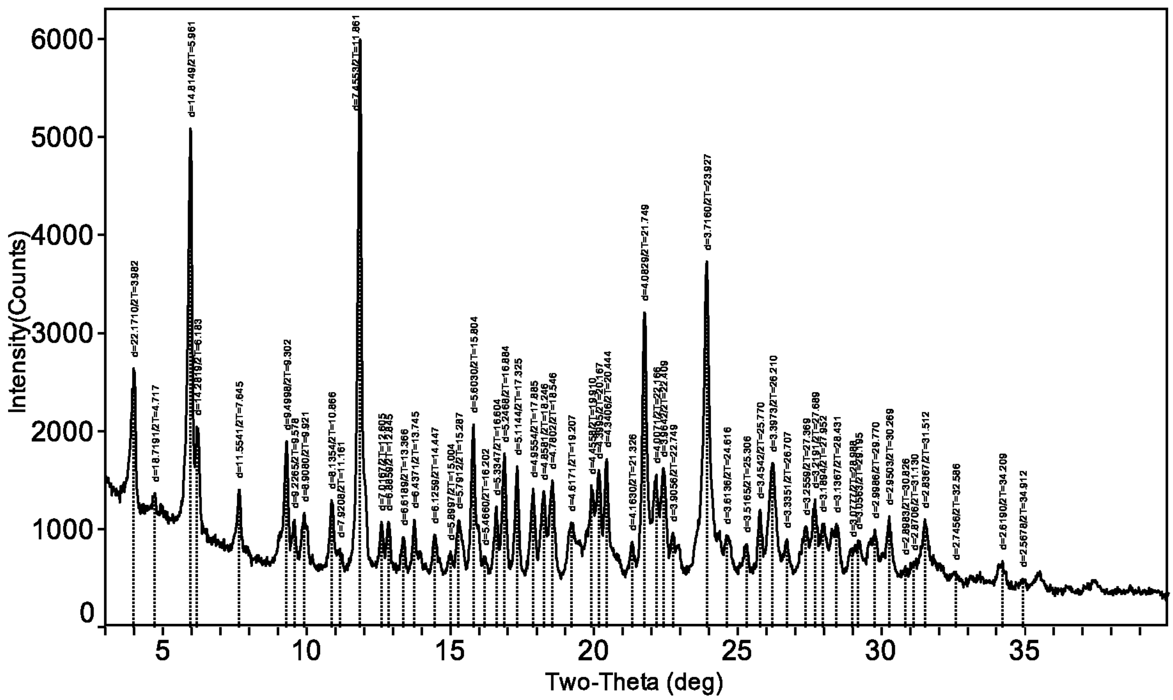


XRD pattern of a crystal form I of compound 3

*FIG. 28*

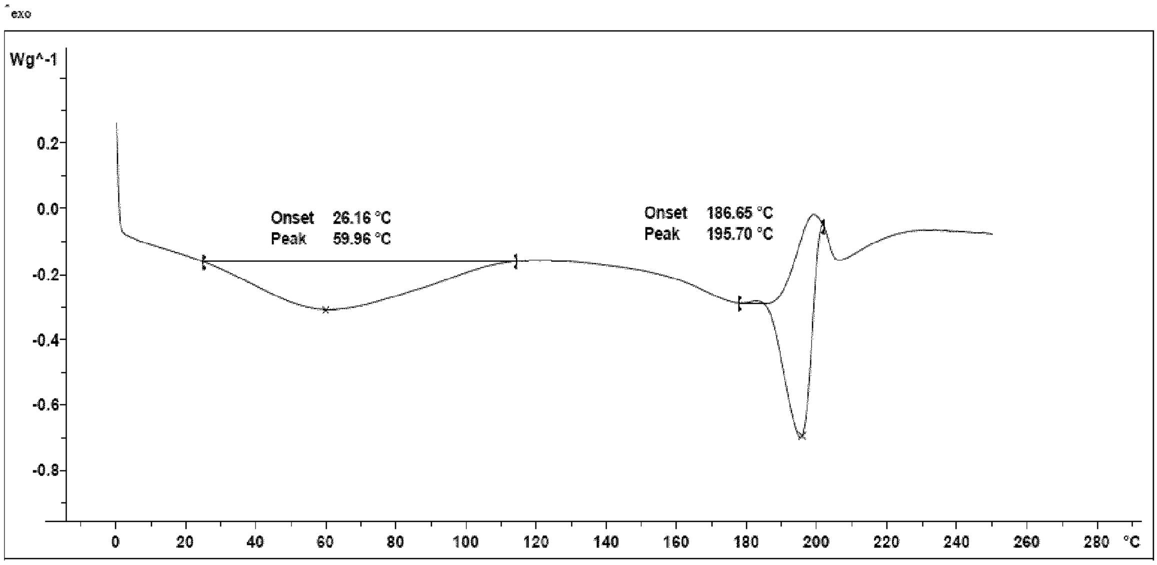


DSC pattern of a crystal form I of compound 3

*FIG. 29*

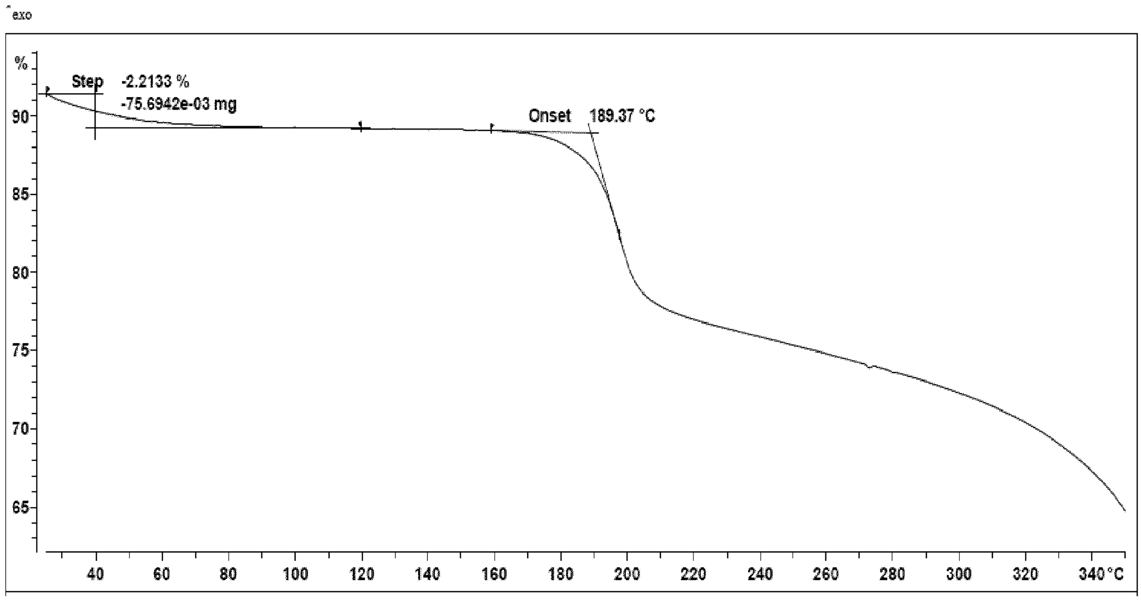


TGA pattern of a crystal form I of compound 3

*FIG. 30*

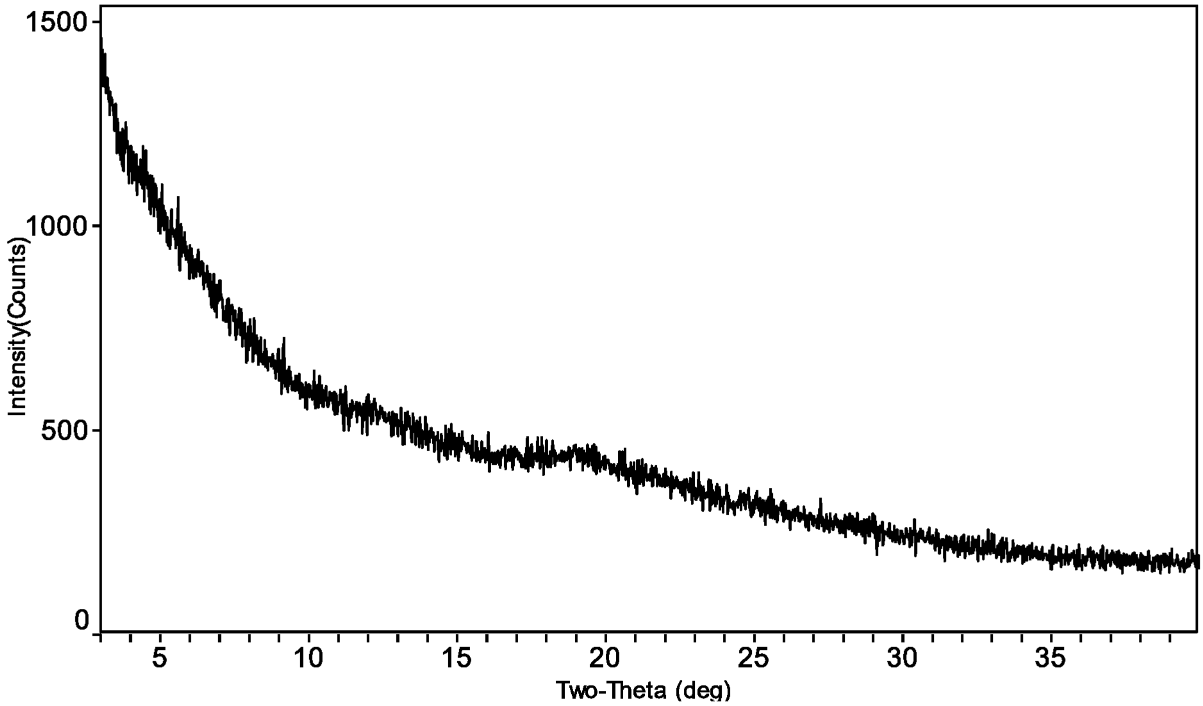


XRD pattern of an amorphous form of compound 3

*FIG. 31*

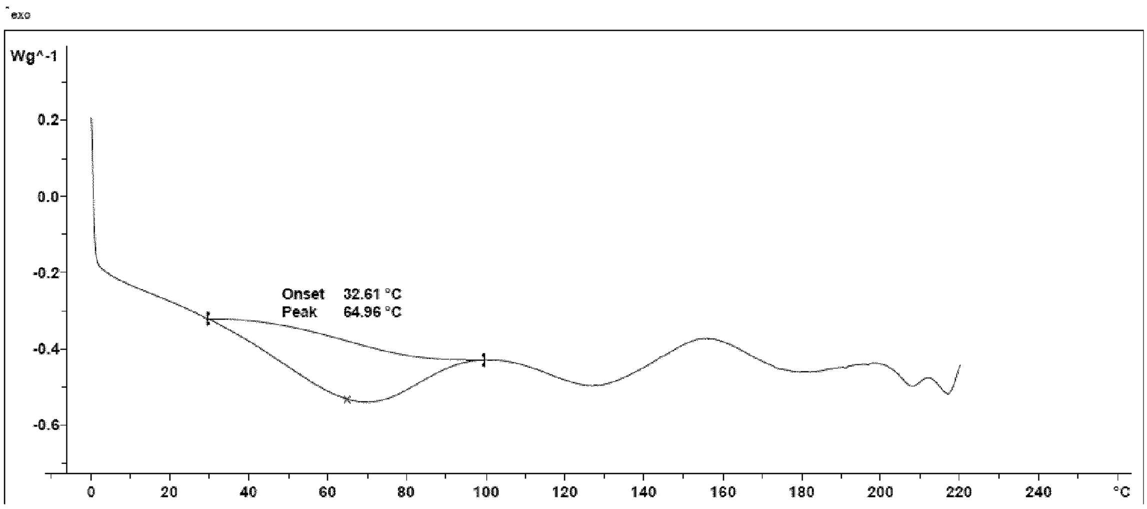


DSC pattern of an amorphous form of compound 3

*FIG. 32*

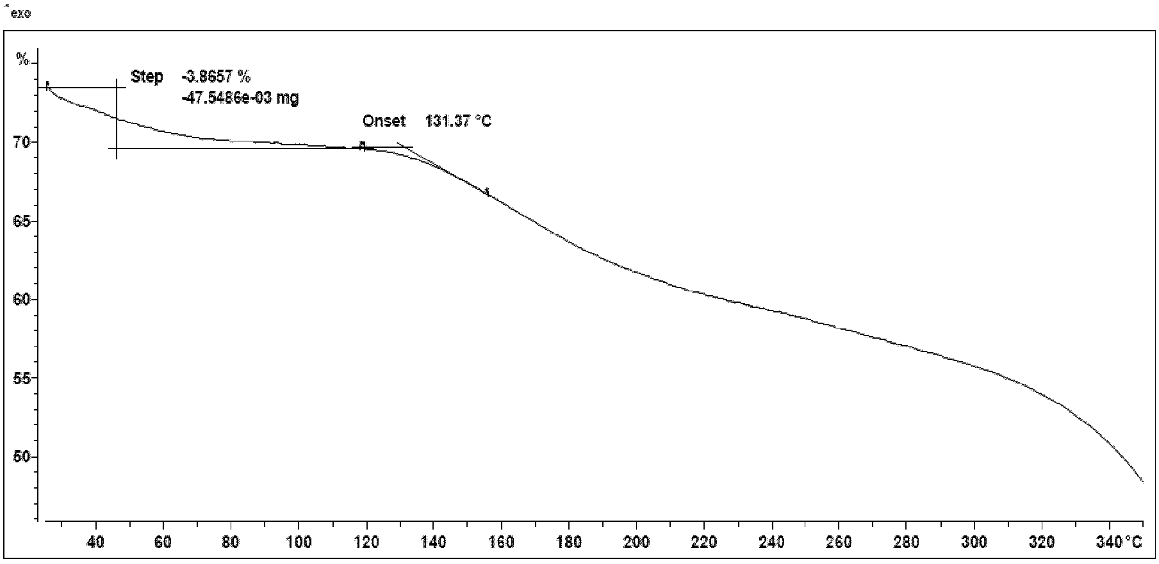


TGA pattern of an amorphous form of compound 3

*FIG. 33*

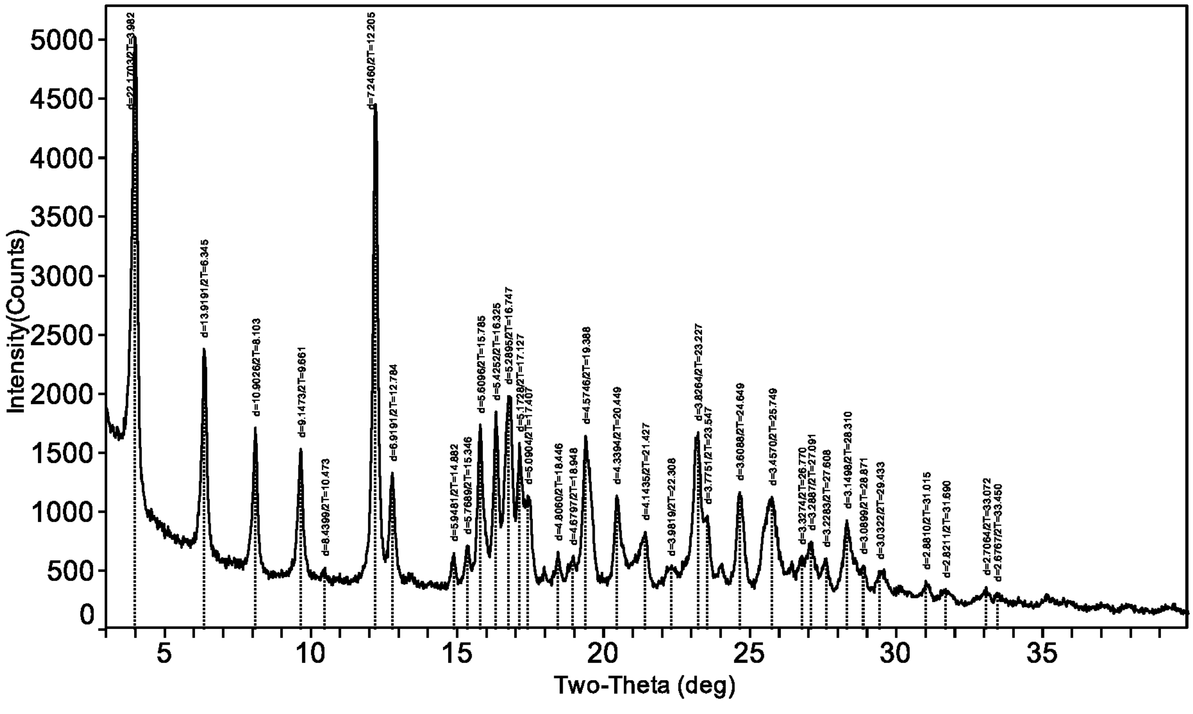


XRD pattern of a crystal form II of compound 3

*FIG. 34*

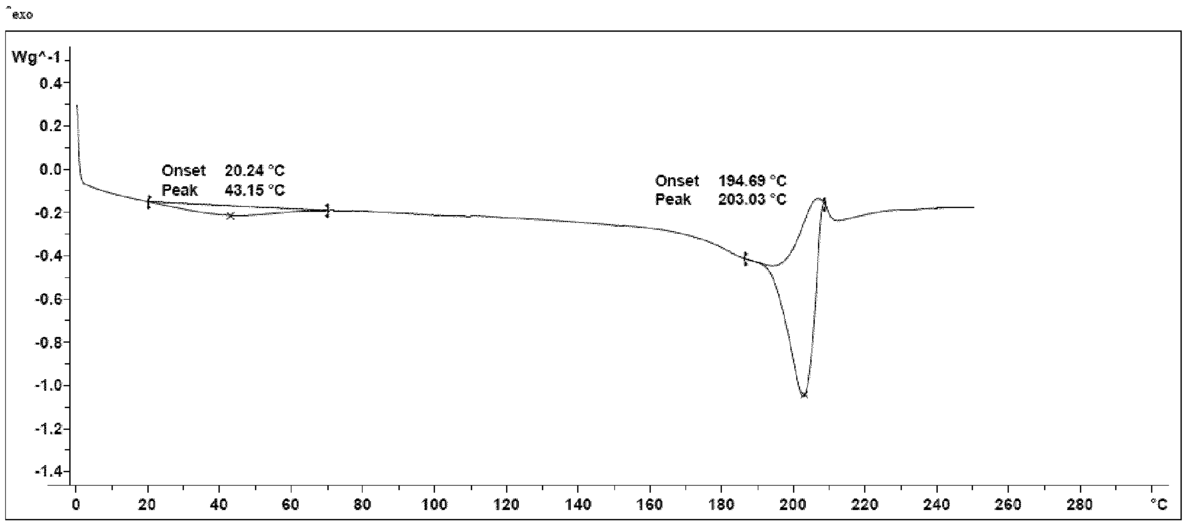


DSC pattern of a crystal form II of compound 3

*FIG. 35*

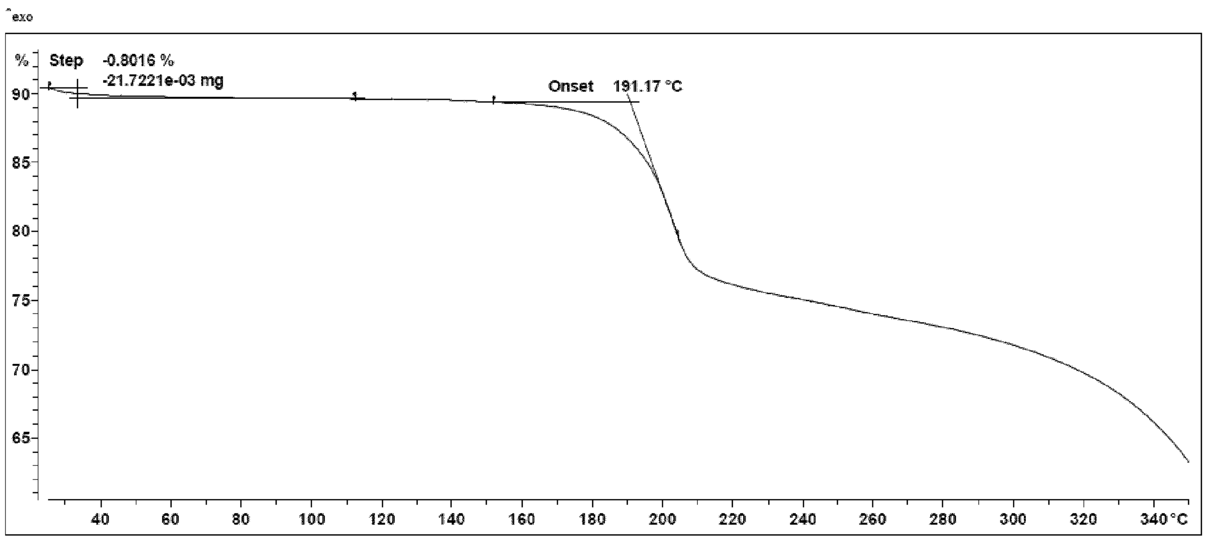


TGA pattern of a crystal form II of compound 3

*FIG. 36*

**INTERNATIONAL SEARCH REPORT**

International application No. **PCT/CN2021/117174**

**A. CLASSIFICATION OF SUBJECT MATTER**

C07D 487/04(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, WPI, EPODOC, STN-REGISTRY, STN-MARPAT, STN-CAPLUS, 海思科, 布鲁顿酪氨酸蛋白激酶, 酪氨酸蛋白激酶, 哌啶, 氮杂环丁基, BTK, tyrosine kinase, PROTAC, piperid+, azetidin+, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2020239103 A1 (SICHUAN HAISCO PHARMACEUTICAL CO., LTD.) 03 December 2020 (2020-12-03)<br>claims 1, 13-19, embodiments 2, 8, 8-1, 17, 17-1, 17-a, 17-b, 23 | 1-33 |
| A | CN 109422752 A (SHANGHAI MEIZHI PHARMACEUTICAL TECHNOLOGY CO., LTD.) 05 March 2019 (2019-03-05)<br>claims 1-10 | 1-33 |
| A | WO 2019127008 A1 (TSINGHUA UNIVERSITY) 04 July 2019 (2019-07-04)<br>claims 1-24 | 1-33 |
| A | CN 110724143 A (TSINGHUA UNIVERSITY) 24 January 2020 (2020-01-24)<br>claims 1-18 | 1-33 |
| A | US 2020121684 A1 (YALE UNIVERSITY) 23 April 2020 (2020-04-23)<br>description paragraphs [0013]-[0034], figure figure 6A, embodiment | 1-33 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 November 2021** | **09 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/117174**

| Patent document cited in search report | | | Publication date (day/month/year) | Patent family member(s) | | | Publication date (day/month/year) |
|---|---|---|---|---|---|---|---|
| WO | 2020239103 | A1 | 03 December 2020 | TW | 202110825 | A | 16 March 2021 |
| CN | 109422752 | A | 05 March 2019 | CN | 110461846 | A | 15 November 2019 |
| | | | | WO | 2019042445 | A1 | 07 March 2019 |
| | | | | EP | 3677584 | A1 | 08 July 2020 |
| | | | | JP | 2020532552 | A | 12 November 2020 |
| | | | | US | 2020199132 | A1 | 25 June 2020 |
| | | | | EP | 3677584 | A4 | 07 April 2021 |
| WO | 2019127008 | A1 | 04 July 2019 | | None | | |
| CN | 110724143 | A | 24 January 2020 | WO | 2021068380 | A1 | 15 April 2021 |
| | | | | CN | 110724143 | B | 23 March 2021 |
| US | 2020121684 | A1 | 23 April 2020 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2017197056 A

### Non-patent literature cited in the description

- *J. Med. Chem.,* 2015, vol. 58, 9625-9638